# EUROPEAN PATENT SPECIFICATION

(11) **EP 3 996 682 B1**
(45) Date of publication and mention of the grant of the patent: **28.02.2024**
(21) Application number: 20735633.8
(22) Date of filing: 08.07.2020
(51) Int. Cl.: A61K 31/519, A61K 9/107, A61K 9/48, A61K 47/10, A61K 47/14, A61K 47/22, A61K 47/44

(54) **PHARMACEUTICAL COMPOSITION COMPRISING A TETRAHYDROPYRAZOLOPYRIMIDINONE COMPOUND**
PHARMAZEUTISCHE ZUSAMMENSETZUNG MIT EINER TETRAHYDROPYRAZOLOPYRIMIDINONVERBINDUNG
COMPOSITION PHARMACEUTIQUE COMPRENANT UN COMPOSÉ DE TÉTRAHYDROPYRAZOLOPYRIMIDINONE

(30) Priority: 09.07.2019 WO PCT/EP2019/068419
(43) Date of publication of application: 18.05.2022
(73) Proprietor: Idorsia Pharmaceuticals Ltd, 4123 Allschwil (CH)
(72) Inventor: AMBUEHL, Michael, 4123 Allschwil (CH); FOURNIER, Elvire, 4123 Allschwil (CH); FRAICHARD, Amandine, 4123 Allschwil (CH); FROIDEVAUX, Sylvie, 68480 Bendorf (FR); GEISELER, Oliver, 4123 Allschwil (CH); HERRMANN, Charlyse, 4123 Allschwil (CH); HUBLER, Francis, 4123 Allschwil (CH); MURPHY, Mark, 4123 Allschwil (CH); RENNEBERG, Dorte, 4123 Allschwil (CH); STAMM, Simon, 4123 Allschwil (CH); VON RAUMER, Markus, 4123 Allschwil (CH)
(74) Representative: Velker, Jörg
(86) International application number: PCT/EP2020/069230
(87) International publication number: WO 2021/005101

(56) References cited:
- WO-A1-2005/063209
- WO-A1-2019/137927

## Description

The present invention relates to pharmaceutical compositions which are self-emulsifying, self-microemulsifying, or self-nanoemulsifying in aqueous medium, comprising as active ingredient the compound 2-(2,2-Difluoropropyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one, said compound being hereinafter also referred to as COMPOUND:

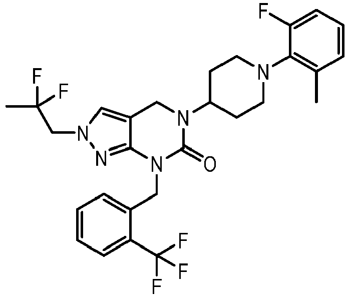

The invention further relates to a crystalline form of COMPOUND in free base form, and its use for the preparation of the present compositions. The invention further relates to pharmaceutical uses of the present compositions for the prevention / prophylaxis or treatment of diseases and disorders related to pathogenic events associated with elevated levels of C5a and/or with C5aR activation.

The COMPOUND is a BCS class II compound having a logP of 6.9 (measured in octanol/phosphate buffer saline - pH 7.4). The preparation of the COMPOUND, which is a C5a receptor modulator, and the medicinal use thereof, especially for the prevention / prophylaxis or treatment of diseases and disorders related to pathogenic events associated with elevated levels of C5a and/or with C5aR activation such as vasculitic diseases or disorders, inflammatory diseases or disorders involving intravascular microvesicle release, immune complex (IC) diseases or disorders, neurodegenerative diseases or disorders, complement related inflammatory diseases or disorders, bullous diseases or disorders, diseases or disorders related to ischemia and/or ischemic reperfusion injury, inflammatory bowel diseases or disorders, and autoimmune diseases or disorders; as well as in contact sensitivity or an inflammation caused by contact with artificial surfaces; increased leukocyte and platelet activation (and infiltration to tissues thereof); pathologic sequelae associated to an intoxication or an injury such as a trauma, an hemorrhage, a shock, or surgery including transplantation, such sequelae including multiple organ failure (MOF), septic shock, shock due to intoxication, or acute lung inflammatory injury; pathologic sequelae associated with insulin-dependent diabetes mellitus; myocardial infarction or thrombosis; edema or an increased capillary permeability; reduction of coronary endothelial dysfunction induced by cardiopulmonary bypass and/or cardioplegia; or cancer; is described in PCT/EP2019/050372.

C5aR1 (CD88) is a seven transmembrane bound G protein coupled receptor (GPCR) belonging to the rhodopsin like family, the gene of which is located on chromosome 19. It couples to pertussis toxin sensitive Gialpha2, Gialpha3 or pertussis toxin insensitive Galpha16 and initiates several downstream signaling pathways. C5aR1 is expressed on a number of immune cell types including monocytes, neutrophils, mast cells, basophils and eosinophils. In addition, it is expressed on many other cell types including hepatocytes, pulmonary and endothelial cells, microglia, neurons and renal glomerular cells. There are a number of ligands described which bind to the C5aR. These include C5a, C5adesArg and C5a +1kDa. C5a is a central effector molecule of the complement system which itself is a complex enzymatic cascade evolved to crucially complement the immune system against invading pathogens, however, a significant body of evidence shows that inadvertent complement activation leads to many acute inflammatory disorders and autoimmune diseases (Ricklin, D., et al. (2010) "Complement: a key system for immune surveillance and homeostasis." Nat Immunol 11(9): 785-797) and specifically C5a has been shown to be elevated in a number of these inflammatory and autoimmune disorders. The complement system is activated through four pathways: The classical pathway, and the mannose binding lectin (MBL) pathway which is similar to the classical pathway except for the initial recognition and activation steps which recognize pathogens or antibody complexes. The alternative pathway is activated by binding of spontaneously activated complement C3 protein (C3b fragment) to pathogen surface. These three pathways all lead to the eventual formation of C3 convertases, which is the point where the 3 pathways converge (Guo, R. F. and P. A. Ward (2005) Annu Rev Immunol 23: 821-852). Subsequently C3 convertases lead to the formation of the anaphalatoxins C3a and C5a, together with other complement proteins required to produce the membrane attack complex. A fourth pathway, the extrinsic pathway involves plasma proteases (eg. elastase, thrombin) which act directly on C3 or C5 leading to the subsequent production of C3a and C5a. The anaphylatoxin C5a leads to the recruitment and activation of inflammatory cells of the innate and adaptive system, partly through the enhancement of cell adhesion molecule expression, the release of granule-based enzymes, delayed or enhanced apoptosis, phagocytosis, oxidative burst, histamine secretion and release and chemotaxis. In addition, it elicits the release of other pro inflammatory mediators, such as TNF-a, IL-1, IL-6, IL-8, prostaglandins, and leukotrienes (N.S. Merle et al. (2015) "Complement System Part II: Role in Immunity." Front Immunol 6: 257), activation of endothelial cells and vascular permeability which may lead to events in which at the end thrombotic microangiopathy can occur. Therefore, C5a represents one of the most potent inflammatory molecules produced during immune responses and because of its fundamental biology it is potentially implicated in a very wide range of pathologies (Janeway's Immunobiology, 8th edition (2012), Kenneth Murphy, Garland Science, p. 48-72).

C5a is central to the immune system and as such is important in key aspects of inflammation and tissue injury. In addition, there is considerable experimental evidence in the literature that implicates increased levels of C5a with a number of diseases and disorders, in particular in autoimmune and inflammatory diseases and disorders (Ricklin, D., et al. (2010) Nat Immunol 11(9): 785-797).

There is a large body of evidence about C5a and its receptor C5aR in contributing to vasculitic diseases, which demonstrate that C5a levels are elevated and give rise to leukocyte migration and subsequent inflammation which then leads to the eventual destruction of vessel walls (Charles J., et al (2013) Semin Nephrol 33(6): 557-564; Vasculitis, 2nd Edition (2008), Edited by Ball and Bridges, Oxford University Press, pp 47-53; Huang, Y. M., et al. (2015) Arthritis Rheumatol 67(10): 2780-2790; Kallenberg, C. G. and P. Heeringa (2015) Mol Immunol 68(1): 53-56). Inhibition of the C5aR with a C5aR antagonist was effective at ameliorated anti-myeloperoxidase (MPO)-induced NCGN in mice expressing the human C5a receptor (Xiao, H. et al (2014) J Am Soc Nephrol 25(2): 225-231) and was confirmed to be effective in a phase II trial of patients with anti-neutrophil cytoplasmic antibody (ANCA) associated vasculitis (ClinicalTrials.gov Identifier NCT02222155). Therefore, a C5a antagonist may be useful to treat vasculitic diseases such as ANCA associated vasculitis, leukoclastic vasculitis, Wegener's granulomatosis, microscopic polyangiitis, Churg-Strauss syndrome, Henoch-Schönlein purpura, polyateritis nodosa, rapidly progressive glomerulonephritis (RPGN), cryoglobulinaemia, giant cell arteritis (GCA), Behcet's disease and Takayasu's arteritis (TAK).

C5a is generated when human blood makes contact with artificial surfaces, such as in cardiopulmonary bypass and hemodialysis procedures for instance on the artificial surface of the heart - lung machine in association with vascular surgery such as coronary artery bypass grafting or heart valve replacement or on surfaces of a kidney dialysis machine (Howard, R. J., etal. (1988) Arch Surg 123(12): 1496-1501; Kirklin, J. K., etal. (1983) J Thorac Cardiovasc Surg 86(6): 845-857; Craddock, P. R., et al. (1977) J Clin Invest 60(1): 260-264; Craddock, P. R., et al. (1977) N Engl J Med 296(14): 769-774) or in association with contact with other artificial vessels or container surfaces (e.g. ventricular assist devices, artificial heart machines, transfusion tubing, blood storage bags, plasmapheresis, plateletpheresis, and the like). As such C5aR antagonists could prove useful in preventing deleterious consequences of contact sensitivity and/or inflammation caused by contact with artificial surfaces. In addition, it may be useful in treating inflammatory disorders involving intravascular microvesicle release such as for example thrombotic microangiopathy and sickle cell disease (Zecher, D., et al. (2014) Arterioscler Thromb Vasc Biol 34(2): 313-320). A C5aR antagonist could also prove useful in certain hemotological diseases which are associated with activation of coagulation and fibrinolytic systems, disseminated intravascular coagulation (DIC), pernicious anemia, warm and cold autoimmune hemolytic anemia (AIHA), anti- phospholipid syndrome and its associated complications, arterial and venous thrombosis, pregnancy complications such as recurrent miscarriage and fetal death, preeclampsia, placental insufficiency, fetal growth restriction, cervical remodeling and preterm birth, idiopathic thrombocytopenic purpura (ITP), atypical hemolytic uremic syndrome (aHUS), paroxysmal nocturnal hemoglobinuria (PNH) and allergic transfusion reactions. The C5-specific humanized antibody, eculizumab is approved for paroxysmal nocturnal hemoglobinuria and atypical haemolytic uraemic syndrome (aHUS) (Wong EK, Kavanagh D, Transl Res. (2015) 165(2):306-20) and has been shown to be efficacious in renal transplant such as acute antibody-mediated kidney allograft rejection and cold agglutinin disease further supporting a potential role for C5aR antagonists in these diseases.

In myocardial ischemia-reperfusion injury C5a has been described to have an important function. Complement depletion reduced myocardial infarct size in mice (Weisman, H. F., T. et al. (1990) Science 249(4965): 146-151; De Hoog, V. C., et al. (2014) Cardiovasc Res 103(4): 521-529) and treatment with anti-C5a antibodies reduced injury in a rat model of hindlimb ischemia-reperfusion (Bless, N. M., et al. (1999) Am J Physiol 276(1 Pt 1): L57-63). Reperfusion injury during myocardial infarction was also markedly reduced in pigs that were re-treated with a monoclonal anti-C5a IgG (Amsterdam, E. A., et al. (1995) Am J Physiol 268(1 Pt 2): H448-457). A recombinant human C5aR antagonist reduces infarct size in a porcine model of surgical revascularization (Riley, R. D., et al. (2000) J Thorac Cardiovasc Surg 120(2): 350-358) providing evidence for the utility of a C5aR antagonist in these diseases. In addition, diseases related to ischemia / reperfusion injury, such as those resulting from transplants, including solid organ transplant, where C5a has been shown to play an important role (Farrar, C. A. and S. H. Sacks (2014) Curr Opin Organ Transplant 19(1): 8-13), could benefit from a C5aR antagonist as could related syndromes such as ischemic reperfusion injury, ischemic colitis and cardiac ischemia (Mueller, M., et al. (2013) Immunobiology 218(9): 1131-1138).

Furthermore, diseases where complement plays a role such as coronary thrombosis (Distelmaier, K., et al. (2009) Thromb Haemost 102(3): 564-572), vascular occlusion, post-surgical vascular reocclusion, atherosclerosis, traumatic central nervous system injury, arrhythmogenic cardiomyopathy (Mavroidis, M., et al. (2015) Basic Res Cardiol 110(3): 27) and Gaucher disease (Pandey et al. (2017) Nature 543: 108-112) could also benefit from a C5aR antagonist. Thus, C5aR modulators may be used preventatively in a patient at risk for myocardial infarction or thrombosis (i.e. a patient who has one or more recognized risk factors for myocardial infarction or thrombosis, such as, but not limited to, obesity, smoking, high blood pressure, hypercholesterolemia, previous or genetic history of myocardial infarction or thrombosis) in order reduce the risk of myocardial infarction or thrombosis.

C5a causes increased capillary permeability and edema, leukocyte and platelet activation and infiltration to tissues, as well as bronchoconstriction (Sarma, J. V. and P. A. Ward (2012) Cell Health Cytoskelet 4: 73-82; Czermak, B. J., et al. (1998) J Leukoc Biol 64(1): 40-48). Administration of an anti-C5a monoclonal antibody was shown to reduce cardiopulmonary bypass and cardioplegia-induced coronary endothelial dysfunction (Tofukuji, M., et al. (1998) J Thorac Cardiovasc Surg 116(6): 1060-1068).

C5a and its receptor are also involved in the pathogenesis of acute respiratory distress syndrome (ARDS) (Hammerschmidt, D. E., et al. (1980) Lancet 1(8175): 947-949), Chronic Obstructive Pulmonary Disorder (COPD) (Marc, M. M., et al. (2004) Am J Respir Cell Mol Biol 31(2): 216-219), and multiple organ failure (MOF) (Huber-Lang, M., et al. (2001) "Role of C5a in multiorgan failure during sepsis." J Immunol 166(2): 1193-1199; Heideman, M. and T. E. Hugli (1984) J Trauma 24(12): 1038-1043;). C5a increases monocyte production of two important proinflammatory cytokines TNF-a and IL-I which contribute to pathology in these diseases. C5a has also been shown to play an important role in the development of tissue injury, and particularly pulmonary injury, in animal models of septic shock (Smedegard, G., et al. (1989) Am J Pathol 135(3): 489-497; Unnewehr, H., et al. (2013) J Immunol 190(8): 4215-4225). In sepsis models using rats, pigs and non-human primates, anti-C5a antibodies administered to the animals before treatment with endotoxin or E. coli resulted in decreased tissue injury, as well as decreased production of IL-6 (Hopken, U., et al. (1996) Eur J Immunol 26(5): 1103-1109; Stevens, J. H., et al. (1986) J Clin Invest 77(6): 1812-1816). Inhibition of C5a with anti-C5a polyclonal antibodies has been shown to significantly improve survival rates in a caecal ligation/puncture model of sepsis in rats (Czermak, B. J., et al. (1999) Nat Med 5(7): 788-792). In the same sepsis model, anti-C5a antibodies were shown to inhibit apoptosis of thymocytes (Guo, R. F., et al. (2000) J Clin Invest 106(10): 1271-1280). Anti-C5a antibodies were also protective in a cobra venom factor model of lung injury in rats, and in immune complex-induced lung injury (Mulligan, M. S., et al. (1996) J Clin Invest 98(2): 503-512). The importance of C5a in immune complex-mediated lung injury was also shown in mouse (Bozic, C. R., et al. (1996) Science 273(5282): 1722-1725). Therefore, a C5aR antagonist could be of benefit in many inflammatory disorders and related conditions including neutropenia, sepsis, septic shock, stroke, inflammation associated with severe burns (Hoesel, L. M., et al. (2007) J Immunol 178(12): 7902-7910), osteoarthritis (Yuan, G., et al. (2003) Chin Med J (Engl) 116(9): 1408-1412), as well as acute (adult) respiratory distress syndrome (ARDS), chronic obstructive pulmonary disorder (COPD), bronchial asthma (Pandey, M. K. (2013) Curr Allergy Asthma Rep 13(6): 596-606), systemic inflammatory response syndrome (SIRS), tissue graft rejection, hyperacute rejection of transplanted organs, and the like, and multiple organ dysfunction syndrome (MODS). In addition, C5aR antagonists may be beneficial in treating pathologic sequelae associated with insulin-dependent diabetes mellitus such as diabetic kidney disease (Li, L., et al. (2015) Metabolism 64(5): 597-610), diabetic retinopathy (Cheng, L., et al. (2013). Invest Ophthalmol Vis Sci 54(13): 8191-8198), lupus nephropathy (Bao, L., et al. (2005) Eur J Immunol 35(8): 2496-2506), Heyman nephritis, membranous nephritis, and other forms of glomerulonephritis such as C3 glomerulopathy including dense deposit disease (DDD) (Zhang et al., Clin J Am Soc Nephrol (2014) 9: 1876-1882). Furthermore, the compound eculizumab has been shown to have potential utility for the treatment of neuromyelitis optica.

C5aR antagonists substantially reduced ovalbumin (OVA)-induced total cell (60%), neutrophil (66%) and eosinophil (65%) influxes in lavage fluid sampling suggesting that C5aR blockage might represent a novel therapeutic agent for reducing asthmatic outcomes (Staab, E. B., et al. (2014) Int Immunopharmacol 21(2): 293-300).

The complement system and in particular C5a contribute to the development of many bullous diseases among other things through activation of innate cells including mast cells and neutrophils (e.g. bullous pemphigoid, bullous acquisita, pemphigus foliaceus and pemphigus vulgaris). The detachment of epidermal basal keratinocytes from the underlying basement membrane is thought to be caused by autoantibodies to keratinocytes at the cutaneous basement membrane leading to blisters and a high influx of neutrophils in both the upper dermal layers and within the blister cavities. In experimental models a reduction of neutrophils or absence of complement (total or C5- selective) can inhibit formation of sub-epidermal blisters (Heimbach, L., et al. (2011) J Biol Chem 286(17): 15003-15009; Gammon, W. R. (1989) Immunol Ser 46: 509-525). Recent evidence has emerged to suggest that inhibition of C5a may prove beneficial in the treatment of the skin disorder hidradenitis suppurativa where an antibody against human C5a was shown to improve patient outcome in an open label phase II clinical trial. A C5a receptor antagonist may therefore be useful in bullous diseases.

Complement is believed to be important in inflammatory bowel disease (IBD) pathology and the C5aR is found to be expressed in the epithelial cells of the colon. (Cao, Q., et al. (2012) Am J Physiol Cell Physiol 302(12): C1731-1740). In addition, pharmacological inhibition of C5a activity by PMX205 a peptidic C5aR antagonist is efficacious in preventing DSS-induced colitis, providing further evidence that targeting CD88 in patients with IBD irritable bowel syndrome, ulcerative colitis, Crohn's disease, inflammatory bowel disease (IBD) (Johswich, K., et al. (2009) Inflamm Bowel Dis 15(12): 1812-1823) could be of therapeutic benefit (Woodruff, T. M., et al. (2003) J Immunol 171(10): 5514-5520; Jain, U., et al. (2013) Br J Pharmacol 168(2): 488-501).

There is a body of evidence suggesting a role for C5a and its receptor in pathologies of the CNS. C5aR expression is upregulated on reactive astrocytes, microglia, and endothelial cells in an inflamed human central nervous system (O'Barr, S. A., et al. (2001) J Immunol 166(6): 4154-4162; Gasque, P., et al. (1997) Am J Pathol 150(1): 31-41) and C5a has been reported to be involved in the pathogenesis of many neurodegenerative diseases, such as amyotrophic lateral sclerosis (ALS) (Mantovani, S., et al. (2014) J Neuroimmunol 276(1-2): 213-218; Humayun, S., et al. (2009) J Neuroimmunol 210(1-2): 52-62; Woodruff, T. M., et al. (2008) J Immunol 181(12): 8727-8734), Alzheimer disease (Fonseca, M. I., et al. (2013) J Neuroinflammation 10: 25; Ager, R. R., et al. (2010) J Neurochem 113(2): 389-401), Parkinson's disease (Wang, X. J., et al. (2007) Neurochem Int 50(1): 39-50) and Huntington's disease (Singhrao et al. (1999) Experimental Neurology 159, 362-376). Furthermore C5a is found to be elevated in the CSF of Guillain-Barre syndrome patients (Hartung, H. P., et al. (1987) Neurology 37(6): 1006-1009; Wakerley, B. R. and N. Yuki (2015) Expert Rev Neurother 15(8): 847-849) and an anti C5 antibody was found to be effective in reducing neuropathy in the mouse (Halstead, S. K., et al. (2008) Brain 131 (Pt 5): 1197-1208; Basta, M. and D. R. Branch (2014) Clin Exp Immunol 178 Suppl 1: 87-88). Also, inhibition of the C5a receptor alleviates experimental CNS lupus (Zwirner, J., et al. (1999) Mol Immunol 36(13-14): 877-884; Jacob, A., B. Hack, et al. (2010) J Neuroimmunol 221(1-2): 46-52). Therefore, C5aR antagonists provided herein may be to treat ALS, Alzheimer's disease, multiple sclerosis, Guillain-Barre syndrome, Parkinson's disease, Huntington's disease and also cognitive function decline associated with cardiopulmonary bypass surgery and related procedures in addition to central nervous system involvement in diseases such as SLE, Sjögren's syndrome and associated immunological profiles.

In many autoimmune diseases Immunoglobulin G-containing immune complex (IC) depositions are found. These contribute to the pathophysiology of the diseases which frequently manifest in different organs of the body including the kidneys, heart, lungs, liver, blood vessels, the nervous system and the skin. There are numerous such IC diseases and examples are systemic lupus erthyematosus (SLE), cryoglobulinemia, rheumatoid arthritis, Sjögren's syndrome (Lawley, T. J., et al. (1979) J Immunol 123(3): 1382-1387), Goodpasture syndrome (antiglomerular basement antibody disease), and hypersensitivity. Immune complexes are known to induce C5 convertases leading to C5a production which subsequently contributes to these diseases (Karsten, C. M. and J. Kohl (2012) Immunobiology 217(11): 1067-1079). In animal models reproducing the mechanisms of IC activation of complement, C5aR has been shown to play an important role. Studies show that C5aR deficient mice and the use of a peptidic C5aR antagonist result in protection from tissue injury induced by ICs. (Strachan, A. J., et al. (2000) J Immunol 164(12): 6560-6565; Kohl, J. and J. E. Gessner (1999) Mol Immunol 36(13-14): 893-903; Baumann, U., et al. (2000) J Immunol 164(2): 1065-1070). Therefore, inhibitors of C5aR could be useful to treat IC diseases including the autoimmune diseases, rheumatoid arthritis (Jose, P. J., et al. (1990) Ann Rheum Dis 49(10): 747-752; Grant, E. P., et al. (2002) J Exp Med 196(11): 1461-1471; Yuan, G., et al. (2003) Chin Med J (Engl) 116(9): 1408-1412), osteoarthritis, systemic lupus erythematosus (Porcel, J. M., et al. (1995) Clin Immunol Immunopathol 74(3): 283-288; Pawaria, S., et al. (2014) J Immunol 193(7): 3288-3295), lupus nephritis (Bao, L., et al. (2005) Eur J Immunol 35(8): 2496-2506), lupus glomerulonephritis and IgA nephropathy (Liu, L., et al. (2014) J Clin Immunol 34(2): 224-232), Heyman nephritis, membranous nephritis and other forms of glomerulonephritis, vasculitis, dermatomyositis (Fiebiger, E., et al. (1998) J Clin Invest 101(1): 243-251), pemphigus, systemic sclerosis (scleroderma) (Sprott, H., et al. (2000) J Rheumatol 27(2): 402-404), bronchial asthma, autoimmune hemolytic and thrombocytopenic states, Goodpasture's syndrome (and associated glomerulonephritis and pulmonary hemorrhage) (Ma, R., et al. (2013) J Clin Immunol 33(1): 172-178), immunovasculitis, and complement mediated thrombotic microangiopathies including atypical haemolytic uremic syndrome (Song, D., et al. (2015) Am J Reprod Immunol 74(4): 345-356; Davin, J. C., N. C. van de Kar (2015) Ther Adv Hematol 6(4): 171-185), mixed cryoglobulinemia, atopic dermatitis (Neuber, K., R. et al. (1991) Immunology 73(1): 83-87; Dang, L., et al. (2015) Mol Med Rep 11(6): 4183-4189), and chronic urticaria (Kaplan, A. P. (2004) J Allergy Clin Immunol 114(3): 465-474; Yan, S., et al. (2014) J Dermatol Sci 76(3): 240-245). Furthermore, the compound eculizumab has been shown to have potential utility for the treatment of myasthenia gravis, and anti-phospholipid syndrome.

C5a is present in psoriatic plaques and C5aR expression has also been reported in psoriasis where T cells, neutrophils mast cells and dendritic cells are involved in pathogenesis of the disease and are chemotactic to C5a (Diani, M., G. Altomare and E. Reali (2015) Autoimmun Rev 14(4): 286-292). Neutrophil accumulation under the stratum corneum is observed in the highly inflamed areas of psoriatic plaques, and psoriatic lesion (scale) extracts contain highly elevated levels of C5a and exhibit potent chemotactic activity towards neutrophils, an effect that can be inhibited by addition of a C5a antibody. Furthermore, T cells and neutrophils are chemo-attracted by C5a under certain conditions (Nataf, S., et al. (1999) J Immunol 162(7): 4018-4023; Tsuji, R. F., et al. (2000) J Immunol 165(3): 1588-1598; Werfel, T., et al. (1997) Arch Dermatol Res 289(2): 83-86; Mrowietz, U., et al. (2001) Exp Dermatol 10(4): 238-245) meaning C5aR antagonists may be of benefit in treating psoriasis.

Furthermore, complement has been implicated in the pathogenesis of glaucoma (Howell et al. (2011), J. Clin. Invest. 121(4): 1429-1444). In addition, there is experimental evidence to suggest a beneficial role of C5aR antagonists in treating cancer with checkpoint blockers. For example, an antibody against the C5aR receptor (IPH5401) has been reported to be efficacious in murine models of cancer (web page Innate Pharma IPH5401, 2018; https://www.innate-pharma.com/en/ pipeline/iph5401-first-class-anti-c5ar-mab; Zah H., et al. (2017) Oncoimmunology 6(10): e1349587; Wang Y., et al., (2016) Cancer Discovery 6(9) 1022-1035).

There is therefore a requirement for new small organic molecule modulators of the C5a receptor (C5aR), especially antagonists of the C5aR, that could be useful for inhibiting pathogenic events associated with elevated levels of C5a and/or with C5aR activation.

The present invention relates to lipid-based pharmaceutical compositions, which are self-emulsifying, self-microemulsifying, or self-nanoemulsifying in aqueous medium, for the oral administration of the COMPOUND; thus, forming a self-emulsifying drug delivery system (SEDDS), self-microemulsifying drug delivery system (SMEDDS), or self-nanoemulsifying drug delivery system (SNEDDS).

WO 2005/063209 A1 discloses self-microemulsifying systems for achieving suitable oral bioavailability, appropriate drug load and stability. The drug delivery system encompasses poorly water-soluble compound (1) ethanolate 14%, Polyoxyl 40 Hydrogenated Castor oil as hydrophilic surfactant 36%, Propylene glycol monocaprylate as lipophilic excipient 24%, Purified diethylene glycol monoethyl ether 25%. Table 4 discloses compositions with Cremophor RH40 as hydrophilic surfactant and Capηol 90 (lipophilic excipient).

A classification of lipid-based delivery systems has been originally proposed by Pouton et al. in 2000 (see for example C.W. Pouton, Eur. J. Pharm. Sci. 11 (2000) S93-S98; Feeney et al.; Advanced Drug Delivery Reviews 101 (2016) 167-194), and can be summarized as follows:

| **Type I** | **Type II** | **Type IIIA / Type IIIB** | **Type IV** |
|---|---|---|---|
| **Oils** | **SEDDS** | **SEDDS, SMEDDS, or SNEDDS** | **Lipid Free** |
| Lipids, no surfactant | No water-soluble components | Includes lipid excipient and water-soluble surfactants and possibly co-solvents | Comprises only water-soluble surfactants and co-solvents |
| No or limited dispersion | Emulsion | Type IIIA: fine emulsion | Micellar solution |
| | | Type IIIB: transparent dispersion | |
| Requires digestion | Will be digested | Digestion may not be necessary | Limited digestion |

Upon dilution in aqueous medium Type IIIA compositions typically show some loss in solvent capacity, and especially for Type IIIB and Type IV compositions significant phase changes and potential loss of solvent capacity may be observed. Solvent capacity upon dilution may, thus, be important for the selection of particular excipients in such compositions for a given active ingredient. Typical compositions for the different lipid-based delivery systems may be summarized as follows:

| Excipients in Formulation | Content of formulation (% w/w) | | | | |
|---|---|---|---|---|---|
| | Type I | Type II | Type IIIa | Type IIIb | Type IV |
| Oils (triglycerides or mixed mono- and di-glycerides) | 100% | 40-80% | 40-80% | < 20% | - |
| Water-insoluble surfactants (HLB < 12) | - | 20-60% | - | - | 0-20% |
| Water-soluble surfactants (HLB > 12) | - | - | 20-40% | 20-50% | 30-80% |
| Hydrophilic co-solvents | - | - | 0-40% | 20-50% | 0-50% |

Such lipid-based pharmaceutical compositions generally form an isotropic mixture, wherein usually the active ingredient is dissolved in liquid or semi-solid excipients. Thus, a good solubility profile without precipitation of the active ingredient from the excipients, as well as a good dispersion and/or digestion profile without precipitation of the active ingredient when administered in the gastrointestinal tract may be considered important. Such properties may be tested using well known in vitro assays. In addition, a composition encompassing COMPOUND may be susceptible to oxidative degradation of the active ingredient. Therefore, a suitable pharmaceutical composition of the COMPOUND requires a good chemical stability of the compound in such formulation.

It has been found that the BCS class II compound 2-(2,2-difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one may be formulated in lipid-based compositions that are self-emulsifying drug delivery systems (SEDDS), self-microemulsifying drug delivery systems (SMEDDS), or self-nanoemulsifying drug delivery systems (SMEDDS), suitable for a drug product intended for prevention / prophylaxis or treatment of diseases and disorders related to pathogenic events associated with elevated levels of C5a and/or with C5aR activation. Such self-emulsifying, self-microemulsifying, or self-nanoemulsifying (SEDDS, SMEDDS, or SNEDDS) pharmaceutical compositions may have beneficial properties such as a good bioavailability and/or chemically stability and/or physical stability. In addition, these compositions may be suitable for a relatively high drug load; and may lead to a rapid *in vivo* absorption of the active ingredient providing for a short onset of the pharmacological effect, and/or a low *in vivo* inter-subject absorption variability when compared to alternative or standard formulations.

Particular compositions of the present invention are SEDDS, SMEDDS, or SNEDDS compositions and are similar to the particular subset of Type III systems as classified by Pouton et al.; i.e. these compositions comprise one or more lipophilic excipient(s), one or more hydrophilic surfactant(s), and optionally one or more hydrophilic co-solvent(s). Certain preferred compositions are characterized in that they comprise one or more lipophilic excipient(s) which is/are hydrophobic surfactant(s), one or more hydrophilic surfactant(s), and preferably one or more hydrophilic co-solvent(s).

### Description of the Figures

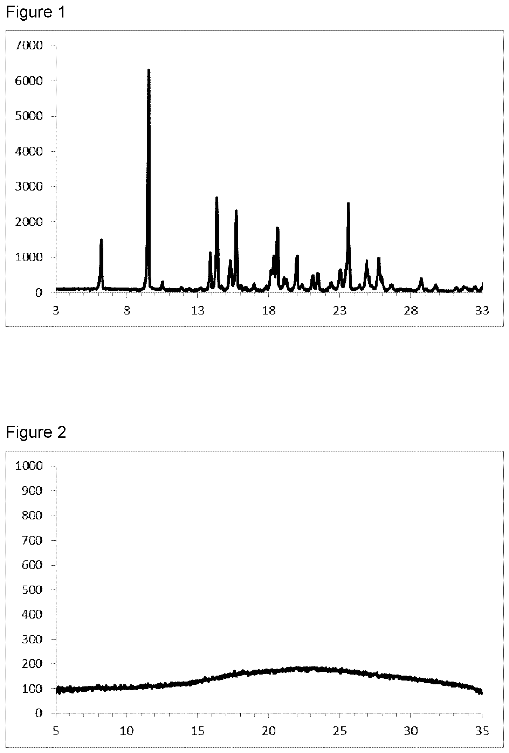

Figure 1 shows the X-ray powder diffraction diagram of COMPOUND in a crystalline form 1 as obtained from Example 2. The X-ray diffraction diagram shows peaks having a relative intensity, as compared to the most intense peak in the diagram, of the following percentages (relative peak intensities given in parenthesis) at the indicated angles of refraction 2theta (selected peaks from the range 3-30° 2theta are reported): 6.2° (22%), 9.5° (100%), 13.9° (16%), 14.4° (42%), 15.3° (13%), 15.7° (35%), 18.4° (15%), 18.6° (28%), 20.0° (15%), 21.5° (8%), 23.6° (37%), 24.9° (13%), and 25.8° (14%).

For avoidance of any doubt, the above-listed peaks describe the experimental results of the X-ray powder diffraction shown in Figure 1. It is understood that, in contrast to the above peak list, only a selection of characteristic peaks is required to fully and unambiguously characterize of the COMPOUND in the respective crystalline form of the present invention.

In the X-ray diffraction diagrams of Fig. 1 the angle of refraction 2theta (2θ) is plotted on the horizontal axis and the counts on the vertical axis.

Figure 2 shows the X-ray powder diffraction diagram of COMPOUND in amorphous form as obtained from Example 1.

### Detailed Description of the Invention

1) A first aspect of the invention relates to pharmaceutical compositions which are self-emulsifying drug delivery systems (SEDDS), self-microemulsifying drug delivery systems (SMEDDS), or self-nanoemulsifying drug delivery systems (SNEDDS), said pharmaceutical compositions comprising the compound 2-(2,2-difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one (COMPOUND): 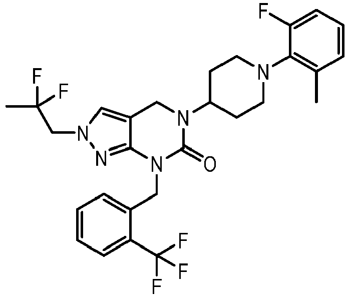
   wherein COMPOUND is in free base form, or in a pharmaceutically acceptable salt form (preferably in free base form);
   wherein said pharmaceutical compositions comprise a mixture of excipients comprising
      - one or more lipophilic excipient(s) (which is/are especially one or more hydrophobic surfactant(s) and/or one or more oil-like excipients);
      - one or more hydrophilic surfactant(s); and
      - optionally one or more hydrophilic co-solvent(s).
2) Another embodiment relates to the pharmaceutical compositions according to embodiment 1), wherein said mixture of excipients comprises
   - a total of about 20 to 50 ww% of one or more lipophilic excipient(s) (which is/are especially one or more hydrophobic surfactant(s) and/or one or more oil-like excipients);
   - a total of about 30 to 80 ww% of one or more hydrophilic surfactant(s); and
   - a total of about 0 to 25 ww% of one or more hydrophilic co-solvent(s).

   wherein the total ww% of said mixture of excipients is 100;
   wherein preferably the total amount of hydrophilic surfactant(s) is at least about 10 ww% higher than the total amount of lipophilic excipient(s).
   Such pharmaceutical compositions of embodiments 1) or 2) preferably comprise COMPOUND in a total amount of about 0.05 ww% to 5 ww%, in particular in a total amount of about 0.075 ww% to about 3 ww%. Said mixture of excipients is preferably present in said pharmaceutical compositions in a total amount of at least about 80 ww%, especially of at least about 90 ww%, wherein the total ww% of said pharmaceutical composition is 100. Said pharmaceutical compositions may additionally comprise conventional ingredients or additives, wherein said conventional ingredients or additives are notably selected from one or more polymers including polymeric crystallization inhibitors, one or more antioxidants, one or more acids, and/or one or more chelating agents; especially one or more antioxidants. Preferably, such pharmaceutical compositions are chemically and physically stable over a certain period of time such as especially 1 year or more.
3) A second aspect of the invention relates to pharmaceutical compositions, especially to pharmaceutical compositions according to embodiments 1) or 2), comprising
   - the compound 2-(2,2-Difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one: 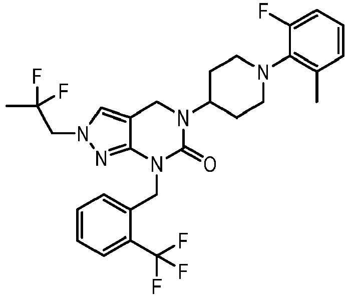 wherein COMPOUND is in free base form, or in a pharmaceutically acceptable salt form (preferably in free base form);
      and
   - a mixture of excipients comprising:
      > a total of about 20 to 50 ww% of one or more lipophilic excipient(s), wherein said lipophilic excipient(s) is/are independently selected from
         ▪ hydrophobic surfactants selected from 1,2-propandiol medium chain mono-fatty acid esters and glycerin medium chain mono-/di-fatty acid esters; and/or
         ▪ oil-like excipients selected from medium chain triglyceride oils and 1,2-propandiol medium chain di-fatty acid esters;
      > a total of about 30 to 80 ww% of one or more hydrophilic surfactant(s), wherein said hydrophilic surfactant(s) is/are independently selected from polyethyleneglycol derivatized long chain lipids and polyethyleneglycol derivatized glycerin medium chain mono-/di-fatty acid esters; and
      > a total of about 0 to 25 ww% of one or more hydrophilic co-solvents;
         wherein the total ww% of said mixture of excipients is 100;
         wherein preferably the total amount of hydrophilic surfactant(s) is higher, especially at least about 10 ww% higher, (with respect to the respective ww% in the mixture of excipients) than the total amount of the respective lipophilic excipient(s).
4) Another embodiment relates to pharmaceutical compositions according to any one of embodiment 1) to 3), said compositions comprising
   - the compound 2-(2,2-Difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one: 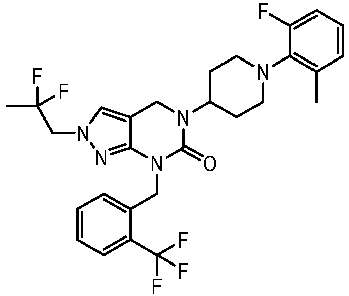 wherein COMPOUND is in free base form, or in a pharmaceutically acceptable salt form (preferably in free base form);
      and
   - a mixture of excipients comprising:
      > about 20 to 50 ww% of a lipophilic excipient, wherein said lipophilic excipient is independently selected from
         ▪ hydrophobic surfactants selected from 1,2-propandiol medium chain mono-fatty acid esters and glycerin medium chain mono-/di-fatty acid esters; or
         ▪ oil-like excipients selected from medium chain triglyceride oils and 1,2-propandiol medium chain di-fatty acid esters;
      > about 30 to 80 ww% of a hydrophilic surfactant, wherein said hydrophilic surfactant is independently selected from polyethyleneglycol derivatized long chain lipids and polyethyleneglycol derivatized glycerin medium chain mono-/di-fatty acid esters; and
      > a total of about 0 to 25 ww% of one or more hydrophilic co-solvents;
         wherein the total ww% of said mixture of excipients is 100;
         wherein preferably the total amount of hydrophilic surfactant is higher, especially at least about 10 ww% higher, (with respect to the respective ww% in the mixture of excipients) than the total amount of the lipophilic excipient.

Where the plural form is used for compounds, salts, pharmaceutical compositions, excipients, diseases and the like, this is intended to mean also a single compound, salt, or the like.

Any reference to COMPOUND is to be understood as referring also to the salts (and especially the pharmaceutically acceptable salts) of such compounds, as appropriate and expedient. Preferably, COMPOUND refers to the free base.

The term "pharmaceutically acceptable salts" refers to salts that retain the desired biological activity of the subject compound and exhibit minimal undesired toxicological effects. Such salts include inorganic or organic acid and/or base addition salts depending on the presence of basic and/or acidic groups in the subject compound. For reference see for example "Handbook of Pharmaceutical Salts. Properties, Selection and Use.", P. Heinrich Stahl, Camille G. Wermuth (Eds.), Wiley-VCH, 2008; and "Pharmaceutical Salts and Co-crystals", Johan Wouters and Luc Quéré (Eds.), RSC Publishing, 2012.

The term "comprising" means in the context of the present invention that the respective composition contains ingredient(s)/ excipient(s) of a certain type/family (e.g. a medium chain triglyceride oil) only the specified ingredient(s)/ excipient(s) (e.g. a medium chain triglyceride oil which is a glyceryl tri-caprylate/caprate) in the specified relative ranges or amounts, and no other ingredient(s)/ excipient(s) of the same type/family. Such compositions, however, may contain additional ingredients/excipients, wherein it is understood that such additional ingredients/excipients shall not be present in an amount that would significantly change the characteristic properties of the respective composition. Thus, for example the addition of a further active ingredient, or of additional conventional ingredients or additives such as one or more polymers including polymeric crystallization inhibitors, one or more antioxidants, one or more acids, one or more chelating agents, etc. would be encompassed. In general, the term "comprising" is to be understood as referring to the respective composition consisting essentially of the specified ingredient(s)/excipient(s) in the amounts as specified; preferably (for the term "comprising") in an amount of at least 90, notably of at least 95 per cent by weight of the total of said composition; i.e. additional ingredients/excipients would not exceed 10, notably 5 per cent by weight of the total of the respective composition.

The pharmaceutical composition according to the present invention will preferably be filled into capsules. Any type of capsule that is usually used to contain pharmaceutical compositions in liquid or semi-solid form may be used in the present invention. Such capsules may be hydroxypropyl methylcellulose capsules, or notably gelatine capsules such as for example hard gelatine capsules or soft gelatine capsules. Preferably, such capsules are soft gelatine capsules. Capsules may be filled under an inert gas atmosphere (such as notably a nitrogen atmosphere, or an argon atmosphere). Such inert gas atmosphere may reduce oxidative degradation of the active ingredient. Conventional packaging means such as aluminium blisters may be used for the above capsules. In one embodiment, inert gas (such as nitrogen) is used to purge oxygen during the blistering step.

The present compositions are isotropic mixtures, which are usually liquid or semi-solid, and which contain COMPOUND, preferably in free base form. Such isotropic mixtures are especially those wherein the active ingredient is dissolved in the mixture of excipients, wherein the excipients are liquid or semi-solid. COMPOUND or a salt thereof may be used for the preparation of the present compositions in amorphous form, or in one or more crystalline forms, or in mixtures of amorphous and crystalline forms. Crystalline forms of COMPOUND free base, of COMPOUND in salt form, or COMPOUND free base cocrystals may be anhydrous, or solvate or hydrate forms. Such salt forms and morphological forms are encompassed in the scope of COMPOUND. Preferably COMPOUND is used in crystalline form of the free base, especially an anhydrous crystalline form of the free base. The present compositions encompass COMPOUND in essentially pure form. The ww% amount of COMPOUND may need to be adjusted to take into account the actual chemical purity, or the presence of a cocrystal former, a salt former such as an acid, a solvate, or a hydrate.

The term "lipophilic excipient" in the context of the present invention refers to hydrophobic surfactant(s) and oil-like excipient(s), including any mixture thereof; suitable for constituting a SEDDS, SMEDDS, or SNEDDS composition. Preferably the term refers to hydrophobic surfactants, especially to 1,2-propandiol medium chain mono-fatty acid esters.

The term " hydrophobic surfactant" in the context of the present invention refers to one or more hydrophobic surfactant(s) suitable for constituting a SEDDS, SMEDDS, or SNEDDS composition; and may in particular be defined as referring to 1,2-propandiol medium chain mono-fatty acid esters (preferred), glycerin medium chain mono-/di-fatty acid esters, or sorbitan fatty acid esters.

The term "oil-like excipient" in the context of the present invention refers to one or more water-insoluble oils / oil-like excipient(s) suitable for constituting a SEDDS, SMEDDS, or SNEDDS composition; and may in particular be defined as referring to medium chain triglyceride oils, or 1,2-propandiol medium chain di-fatty acid esters.

The term "hydrophilic surfactant" in the context of the present invention refers to one or more hydrophilic surfactant(s), suitable for constituting a SEDDS, SMEDDS, or SNEDDS composition; and may in particular be defined as referring to polyethyleneglycol derivatized long chain lipids (preferred), polyethyleneglycol derivatized glycerin medium chain mono-/di-fatty acid esters, or polyethyleneglycol derivatized sorbitan fatty acid esters. Preferred examples of such hydrophilic surfactants are polyethyleneglycol derivatized long chain lipids such as polyethyleneglycol derivatized hydrogenated castor oils (such as especially Kolliphor^{®} RH40). Further examples of polyethyleneglycol derivatized long chain lipids are polyethyleneglycol derivatized castor oils (such as especially Kolliphor^{™} EL), vitamin E TPGS (d-α-tocopheryl polyethylene glycol 1000 succinate), or polyethyleneglycol derivatized hydroxystearic acid (such as especially Solutol^{®} HS15).

Certain excipients as used within the scope of the present invention may be defined as surfactants. Such surfactants may be further defined by their respective hydrophilic-lipophilic balance (HLB) value. The HLB value is an empirical parameter commonly used to characterize the relative hydrophilicity and hydrophobicity of nonionic amphiphilic compounds. Surfactants with lower HLB values (generally equal or below about 8) are more hydrophobic (hydrophobic surfactant), whereas surfactants with higher HLB values (generally equal or greater than about 12) are more hydrophilic and show greater solubility in aqueous medium (hydrophilic surfactant).

In particular, hydrophobic surfactants suitable for the present invention, such as especially 1,2-propandiol medium chain mono-fatty acid esters (e.g. propylene glycol mono-caprylate), glycerin medium chain mono- or di-fatty acid esters (e.g. glyceryl mono-/di-caprylate), or sorbitan fatty acid esters, have an HLB value of about 8 or below, notably of about 6. Such hydrophobic surfactants may, thus, be defined as lipophilic excipients.

Likewise, hydrophilic surfactants suitable for the present invention, such as especially polyethyleneglycol derivatized long chain lipids, polyethyleneglycol derivatized glycerin medium chain mono-/di-fatty acid esters, or polyethyleneglycol derivatized sorbitan fatty acid esters, generally have an HLB value of about 12 or greater.

Preferably, for the compositions of the present invention, the total amount of hydrophilic surfactant(s) is higher, especially at least about 10 ww% higher (with respect to the respective ww% in the mixture of excipients) than the total amount of the respective lipophilic excipient(s).

Surfactants, especially commercially available surfactant products, are usually not pure compounds but may be rather complex mixtures of compounds containing one primary major surfactant component. These commercially available surfactant products may contain variable amounts of said primary major surfactant component, and residual amounts of further components such as for example their respective corresponding fatty acid (poly-)esters, or fatty acids; as well as variable amounts of solvents, such as water, or organic solvents such as ethanol, or 1,2-propandiol, or, in the case of commercially available polyethyleneglycol derivatized long chain lipids, residual polyethyleneglycols (which may stem from the chemical production process). If present, the total weight of residual amounts of further components (with respect to the total weight of the respective surfactant product) preferably is below about 55 ww%, notably it is about 10 to 30 ww%, depending on the respective commercial product. The above-mentioned residual further components are encompassed in the scope of terms hydrophilic surfactant, lipophilic excipient, hydrophobic surfactant, oil, oil-like excipient, as used herein.

If not explicitly stated otherwise, it is assumed that the surfactants as used within the scope of the present invention preferably contain said primary major surfactant component in an amount of greater than about 45 ww%, especially greater than about 65 ww%, notably greater than about 90 ww% per total weight of the respective surfactant. In particular, the surfactants are essentially devoid of free fatty acids (i.e. they contain less than 10 ww% of free fatty acids per total weight of the respective surfactant, notably less than 5 ww%, especially less than 1 ww%) and contain less than 15 ww% (notably less than 10 ww%, especially less than 5 ww%) of ethanol or water per total weight of the respective surfactant.

The term "fatty acid" as used herein refers to saturated or partially unsaturated straight chain carboxylic acids having 6 to 28 carbon atoms, preferably 8 to 20 carbon atoms.

The term "fatty acid ester" refers to an ester composed of an alcohol component as explicitly specified [i.e. glycerin, or 1,2-propandiol (propylene glycol)]; and one or more fatty acid components as defined before and as explicitly specified.

The term "medium chain" in the context of lipids refers to lipids having a carbon chain of a length of 6 to 12 carbon atoms. Within the fatty acids, medium chain fatty acids may thus be defined as having 6 to 12 carbon atoms, preferably 8 to 10 carbon atoms. Examples are caprylic acid, capric acid, or lauric acid especially caprylic acid and capric acid.

Likewise, the term "long chain" in the context of lipids refers to lipids having a branched or straight carbon chain of a backbone length of 14 or more carbon atoms, preferably 14 to 20 carbon atoms. Within the fatty acids, long chain fatty acids may be defined as having 14 or more carbon atoms, preferably 14 to 20 carbon atoms. Examples are myristic acid, palmitic acid, stearic acid, 12-hydroxy-stearic acid, ricinoleic acid and arachidic acid.

Long chain lipids may be defined as including long chain fatty acids and internal esters thereof (such as for example 12-((12-hydroxy-stearoyl)oxy)-stearic acid), mono-, di-, and triglyceride long chain fatty acid esters, 1,2-propandiol long chain mono- and di-fatty acid esters, long chain waxes, and long chain fat-soluble vitamins (such as especially vitamin E including vitamin E succinate).

The term "1,2-propandiol medium chain mono-fatty acid ester" refers to a hydrophobic surfactant, especially as a commercially available product, containing as the primary major component a mono-propyleneglycolate composed of the alcohol component 1,2-propandiol (propyleneglycol) and one medium chain fatty acid moiety (i.e. the second hydroxy group of 1,2-propandiol is unsubstituted). An example is propylene glycol mono-caprylate [such as Capryol^{™} (especially Capryol^{™} 90)]. For avoidance of doubt, the term "1,2-propandiol medium chain mono-fatty acid ester" includes products comprising said primary major component and as a secondary component a small fraction of the corresponding di-ester. 1,2-Propandiol medium chain mono-fatty acid esters contain said primary major component in an amount of greater than about 45%, especially greater than about 75 ww%, notably greater than about 90 ww% per total weight of the excipient. Propylene glycol mono-caprylate, as available from commercial suppliers is used for the present invention, preferably Capryol^{™} (especially Capryol^{™} 90) from Gattefossé. Alternative commercial products are for example Lauroglycol^{™} from Gattefossé.

The term "glycerin medium chain mono-/di-fatty acid ester" refers to a hydrophobic surfactant, especially as a commercially available product, containing as the primary major component a mono- and/or di-glycerate composed of the alcohol component glycerin (propan-1,2,3-triol) and one or two medium chain fatty acid moieties (i.e. one or two hydroxy group(s) of glycerin is/are unsubstituted). Preferred examples of glycerin medium chain mono-/di-fatty acid esters are commercially available glyceryl mono-/di-caprylates (such as Capmul^{®} MCM produced by Abitec). While all grades of the Capmul^{®} MCM product line are suitable for use in the present invention, it may be desirable to use to European Pharmacopeia (EP) grade as it includes 3% glycerol, whereas the US National Formulary (NF) grade includes 7% glycerol. Alternative commercial products are for example Capmul^{®} MCM C8, orCapmul^{®} MCM C10 produced by Abitec; Imwitor^{®} 742 or Imwitor^{®} 988 produced by Sasol Germany GMBH.

The term "sorbitan fatty acid esters" refers to a hydrophobic surfactant, especially as a commercially available product, containing as the primary major component a sorbitan, which is esterified (in general mono-esterified) with a higher medium chain (e.g. C₁₂) or long chain fatty acid. Sorbitan monolaurate, sorbitan monopalmitate, sorbitan monostearate, sorbitan monooleate are typical commercial products, e.g. as produced by Croda International as Span^{®} 20, Span^{®} 40, Span^{®} 60, and, respectively, Span^{®} 80 products.

The term "medium chain triglyceride oil" (MCT oil), refers to an oil-like excipient, especially as a commercially available product containing as the primary major component a glycerin medium chain tri-fatty acid ester, i.e. glycerin that is esterified on each of its three hydroxy groups with a medium chain fatty acid, wherein it is understood that the medium chain fatty acids attached to the same glycerin molecule may be the same or different; and wherein it is understood that the term encompasses mixtures of different glycerin medium chain fatty acid esters. Such mixtures may be present in certain commercial products. A preferred example of such a glycerin medium chain tri-fatty acid ester is glyceryl tri-caprylate/caprate. Glycerin medium chain tri-fatty acid esters as available from commercial suppliers are preferably Captex^{®} MCT oils (such as for example Captex^{®} 300, Captex^{®} 350, Captex^{®} 355 produced by Abitec), Miglyol^{®}810, Miglyol^{®}812, or Miglyol^{®}8108 produced by Sasol Germany GMBH; Neobee^{®} M5 by the Stepan Company; Crodamol^{®} GTC/C produced by Croda Inc; and Labrafac^{®} Lipophile WL 1349 produced by the Gattefossé Group. Preferred is Miglyol^{®}812.

The term "1,2-propandiol medium chain di-fatty acid ester" refers to an oil-like excipient, especially as a commercially available product containing as the primary major component a di-propyleneglycolate composed of the alcohol component 1,2-propandiol (propyleneglycol) and two medium chain fatty acid moieties (i.e. both hydroxy groups of 1,2-propandiol are esterified with a medium chain fatty acid, wherein it is understood that the medium chain fatty acids attached to the same 1,2-propandiol molecule may be the same or different; and wherein it is understood that the term encompasses mixtures of different 1,2-propandiol medium chain fatty acid esters). Such mixtures may be used in certain commercial products. A preferred example of such a 1,2-propandiol medium chain di-fatty acid ester is propyleneglycol di-caprylate/di-caprate. 1,2-propandiol medium chain di-fatty acid ester as available from commercial suppliers are preferably Labrafac^{™} PG produced by the Gattefossé Group and Captex^{®} 200 produced by the Abitec Corporation.

The term "polyethyleneglycol derivatized long chain lipid" refers to a hydrophilic surfactant, especially as a commercially available product, containing as the primary major component a long chain lipid (such as long chain fatty acids including hydroxy-substituted long chain fatty acids such as 12-hydroxy-stearic acid or a mixture of 12-hydroxy-stearic acid and 12-((12-hydroxy-stearoyl)oxy)-stearic acid, vitamin E succinate, castor oil, or especially hydrogenated castor oil), wherein said long chain lipid in turn is derivatized (in general by chemical reaction with ethylene oxide) on one or more free hydroxy and/or carboxylic acid group(s), as the case may be, with polyethyleneglycol. Examples are polyethyleneglycol derivatized 12-hydroxy-stearic acid, polyethyleneglycol derivatized vitamin E succinate, polyethyleneglycol derivatized castor oils, and, especially, polyethyleneglycol derivatized hydrogenated castor oils. Polyethyleneglycol derivatized long chain lipids suitable for the present invention generally have an HLB value of about 12 or greater, notably between about 12 and 15. Reported HLB values may be characteristic to the respective product and usually vary between different commercial products even when containing the same primary major surfactant component. Polyethyleneglycol derivatization preferably consists of 5 to 60 moles of PEG per mole of lipid, notably 10 to 50 moles of PEG per mole of the respective long chain lipid. In general, commercially available polyethyleneglycol derivatized long chain lipids may contain various amounts of free polyethylene glycol.

A preferred example of such polyethyleneglycol derivatized long chain lipids is "polyethyleneglycol derivatized hydrogenated castor oil", which term refers to a nonionic hydrophilic surfactant, especially a commercially available surfactant product, containing as the primary major surfactant component hydrogenated castor oil, a 1,2,3-propantriol tri-fatty acid ester (triglyceride) composed of the alcohol component 1,2,3-propantriol and three ricinoleic acid moieties which in turn are hydrogenated and which triglyceride is derivatized with polyethyleneglycol. Polyethyleneglycol derivatization preferably consists of 5 to 60 moles of PEG per mole of triglyceride, notably 20 to 50, especially 25 to 45 moles of PEG per mole of triglyceride. Examples of such polyethyleneglycol derivatized hydrogenated castor oils are PEG-20-, PEG-25-, PEG-30-, PEG-40-, PEG-45-, PEG-50-, or PEG-60 hydrogenated castor oil; commercially available for example as Kolliphor^{®} RH, formerly named Cremophor^{®} RH variants comprising hydrogenated castor oil such as especially PEG-40 hydrogenated castor oil: Kolliphor ^{®} RH40, or equivalents thereof. Polyethyleneglycol derivatized hydrogenated castor oils preferably contain said primary major surfactant component in an amount of greater than about 45 ww%, notably greater than about 75 ww%, especially greater than about 90 ww% per total weight of the excipient. PEG derivatized hydrogenated castor oil as available from commercial suppliers is used for the present invention; preferably PEG-40 hydrogenated castor oil (such as Kolliphor^{®} RH40, formerly named Cremophor^{®} RH40 from BASF) is used for the present invention.

Another example of polyethyleneglycol derivatized long chain lipids is "polyethyleneglycol derivatized castor oil" (macrogolglycerol ricinoleate or polyoxyl castor oil), commercially available for example as Kolliphor^{®} EL, formerly named Cremophor^{®} EL variants comprising castor oil such as especially PEG-35 castor oil: Kolliphor^{®} EL, or equivalents thereof.

Another example of polyethyleneglycol derivatized long chain lipids is polyethyleneglycol derivatized 12-hydroxy-stearic acid. Commercially available polyethyleneglycol derivatized 12-hydroxy-stearic acid contains as the primary major surfactant component a mixture of polyethyleneglycol esters of 12-hydroxy-stearic acid and polyethyleneglycol esters of 12-((12-hydroxy-stearoyl)oxy)-stearic acid. In addition, such commercial polyethyleneglycol derivatized 12-hydroxy-stearic acid may contain various amounts (e.g. about 30 ww% in Kolliphor^{®} HS15) of free polyethylene glycol. In addition small amounts of 12-hydroxy-stearic acid etherified at the 12-hydroxy group with polyethyleneglycol may be present. Preferred is commercially available Kolliphor^{®} HS15 (formerly Solutol^{®} HS15) which is obtained by reacting about 15 moles of ethylene oxide with 1 mole of 12-hydroxy-stearic acid.

Another example of polyethyleneglycol derivatized long chain lipids is vitamin E TPGS (d-α-tocopheryl polyethylene glycol 1000 succinate).

The term "polyethyleneglycol derivatized glycerin medium chain mono-/di-fatty acid esters" refers to a hydrophilic surfactant, especially as a commercially available product, containing as the primary major component a glycerin medium chain mono-/di-fatty acid ester lipid (such as glyceryl mono-/dicaprylate/caprate), wherein said medium chain mono-/di-fatty acid ester lipid in turn is derivatized (in general by chemical reaction with ethylene oxide) on one or more free hydroxy and/or carboxylic acid group(s), as the case may be, with polyethyleneglycol. Polyethyleneglycol derivatized glycerin medium chain mono-/di-fatty acid esters suitable for the present invention generally have an HLB value of about 12 or greater, notably between about 12 and 15. Reported HLB values may be characteristic to the respective product and usually vary between different commercial products even when containing the same primary major surfactant component. Polyethyleneglycol derivatization preferably consists of 5 to 20 moles of PEG per mole of lipid, notably 5 to 10 moles of PEG per mole of the respective medium chain lipid. In general, commercially available polyethyleneglycol derivatized glycerin medium chain mono-/di-fatty acid esters may contain various amounts of free polyethylene glycol. Polyethyleneglycol derivatized glycerin medium chain mono-/di-fatty acid esters preferably contain said primary major surfactant component in an amount of greater than about 45 ww%, notably greater than about 75 ww%, especially greater than about 90 ww% per total weight of the excipient. Such polyethyleneglycol derivatized glycerin medium chain mono-/di-fatty acid esters are commercially available for example as products consisting of mainly PEG-8 glycerin mono- and diesters of caprylic (C8) and/or capric (C10) acid, and a small fraction of mono-, di- and triglycerides, e.g. Labrasol^{®} ALF from Gattefossé, or equivalents thereof.

The term "polyethyleneglycol derivatized sorbitan fatty acid esters" refers to a hydrophilic surfactant, especially as a commercially available product, containing as the primary major component a polyethylene glycol derivatized sorbitan, which in turn is esterified (in general mono-esterified) with a higher medium chain (e.g. C₁₂) or long chain fatty acid. Polyethyleneglycol derivatization preferably consists of a total of about 20 moles of polyethylene glycol per mole of sorbitan, wherein polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 80 (polyoxyethylene (20) sorbitan monooleate) are typical commercial products, e.g. as produced by Croda International as Tween^{®} 20, Tween^{®} 40, Tween^{®} 60, and, respectively, Tween^{®} 80 products.

Polyethylene glycol (PEG) refers to an oligomer or polymer of ethylene oxide and is generally prepared by polymerization of ethylene oxide. The numbers generally included in the names of PEGs indicate their average molecular weights (e.g. a PEG with an average molecular weight of approximately 400 daltons would be labeled PEG 400). In general, PEGs include molecules with a distribution of molecular weights (i.e. they are polydisperse). Liquid or semi-solid polyethyleneglycols are generally lower molecular weigth PEGs such as PEG 400 / macrogol 400 and may be used as hydrophilic co-solvents.

The excipients as used within the scope of the present invention are preferably liquid at a temperature of about 40 to 50°C. Especially, the composition of embodiment 1) forms a liquid and clear isotropic mixture (in general a solution) at about 40 to 50°C. In another embodiment, in case the pharmaceutical composition is filled into soft gelatine capsules, the composition of embodiment 1) forms a liquid and clear isotropic mixture (in general a solution) at a temperature between room temperature and about 40°C (notably between about 30°C and about 38°C, especially at about 35°C).

For avoidance of any doubt, ww% quantities refer to the total of the respective (commercially available) excipient, (commercially available) hydrophilic co-solvent, etc. as added to the mixture of excipients / the pharmaceutical composition; and are calculated with respect to the total weight of the mixture of excipients / the total weight of the pharmaceutical composition (as the case may be). Thus, it is understood that for calculating ww% amounts of a certain excipient, any residual polyethyleneglycol / solvent(s) / other chemicals (such as Vitamin E) which may be present in such excipient (e.g. in a hydrophilic surfactant) are considered as being part of said excipient and take part in the ww% of such excipient. Likewise, ww% amounts of hydrophilic co-solvents are calculated on basis of the weight amount of hydrophilic co-solvent as added to the mixture of other excipients / ingredients of the composition.

For avoidance of any doubt, it is well understood that the pharmaceutical composition as defined in any one of embodiments 1) to 36) may additionally comprise further conventional excipients, ingredients and/or additives, which may be used alone or in combination (quantum satis, i.e. wherein the maximum amounts of said further conventional ingredients or additives and/or the maximum amounts of the respective mixture of excipients may need to be reduced to make up the total ww% of 100).

Further excipients which may be used in the present pharmaceutical compositions are conventional ingredients or additives, such as especially polymers and/or antioxidants and/or chelating agents and/or acids, which may be used alone or in combination. Reference is made to the extensive literature on the subject for these and other pharmaceutically acceptable excipients and procedures mentioned herein, see for example R.C. Rowe, P.J. Seskey, S.C. Owen, Handbook of Pharmaceutical Excipients, 5th edition, Pharmaceutical Press 2006; Remington, The Science and Practice of Pharmacy, 21st Edition (2005), Part 5, "Pharmaceutical Manufacturing" [published by Lippincott Williams & Wilkins]. In the context of this invention, such conventional ingredients or additives are considered excipients, i.e. in case such conventional ingredients or additives are present, their corresponding ww% is generally to be considered in relation to the total weight of the mixture of excipients.

For the present invention, the mixture of excipients especially contains one or more antioxidants.

The term "antioxidant" encompasses all types of pharmaceutically acceptable antioxidants capable of inhibiting the oxidation of other molecules. Such antioxidants that are suitable to be used in pharmaceutical compositions are well known in the art. More than one of such antioxidants may be used in combination.

In case more than one antioxidant is used, preferably a mixture of one oxygen scavenger and one chain terminator is used. One or more antioxidants may be used in combination with a chelating agent. Oxygen scavengers, chain terminators, and/or chelating agents may be used alone or in any combination; and their combined use may have complementary effect.

A sub-group of antioxidants are oxygen scavengers. Such oxygen scavengers that are suitable to be used in pharmaceutical compositions are well known in the art. Examples are especially ascorbic acid (E300) and/or esters thereof. A preferred example is ascorbyl palmitate (AP). An oxygen scavenger, if present, is comprised in the composition (with respect to the total weight of the pharmaceutical composition) in an amount of below about 2 ww% (notably about 0.1-1 ww%, especially about 0.5 to 1 ww%, in particular about 0.1 ww%, about 0.5 ww%, or about 1 ww%).

Another sub-group of antioxidants are (radical) chain terminators. Such chain terminators capable of terminating radical chain reactions and suitable to be used in pharmaceutical compositions are well known in the art. Preferred are those which form an isotropic mixture with (usually: are soluble in) a mixture of excipients according to the present invention. Examples are propyl gallate (PG, E310), tertiary butylhydroquinone (TBHQ), butylated hydroxytoluene, such as 2,6-di-tert-butyl-4-methylphenol (BHT, E321); and butylated hydroxyanisole, such as the isomeric mixture of 2-tert-butyl-4-hydroxyanisole and 3-tert-butyl-4-hydroxyanisole (BHA, E320). Preferred is propyl gallate (PG, E310). Other preferred examples are compounds of the Vitamin E family / tocopherols such as alpha-tocopherol (E306). In certain instances, an excipient which is based on Vitamin E (e.g. Vitamin E TPGS) may contain sufficient amounts of free Vitamin E that no additional antioxidant / oxygen scavenger is required. A preferred example of such chain terminators of the Vitamin E family is DL-alpha-Tocopheryl acetate (Vitamin E acetate). Preferred oxygen scavengers are those which form an isotropic mixture with (usually: are soluble in) a mixture of excipients according to the present invention. Such chain terminators may also be used in combination. A chain terminator, if present, is comprised in the composition (with respect to the total weight of the pharmaceutical composition) in an amount of below about 0.3 ww% (notably about 0.01 to 0.2 ww%, especially about 0.05 to 0.1 ww%, in particular about 0.1 ww% (e.g. for PG, BHT, or BHA), or about 0.05 ww% (e.g. for DL-alpha-Tocopheryl acetate, BHT, or BHA)).

Chelating agents that are suitable to be used in pharmaceutical compositions are well known in the art. Preferred example are histidine and ethylenediaminetetraacetic acid (EDTA), or salts thereof, such as for example disodium EDTA. Such chelating agents may also be used in combination. A chelating agent, if present, is comprised in the composition (with respect to the total weight of the pharmaceutical composition) in a total amount of below about 0.1 ww%, notably in a total amount of below about 0.01 ww%. Preferably no chelating agent is present.

Further examples of such conventional ingredients or additives are polymers. Polymers may be used in the present pharmaceutical compositions for example as polymeric crystallization inhibitors (PCI) to stabilize the supersaturated state and retard the drug precipitation process. PCI adsorb on the nuclei surface and prohibits formation of crystalline nuclei / lattice [see for example: Simonelli, Mehta, Higuchi; "Inhibition of Sulfathiazole Crystal Growth by Polyvinyl Pyrrolidone.", J.Pharm.Sci., 59, 633-638 (1970). Umesh S. Kestur, Lynne S. Taylor; "Role of polymer chemistry in influencing crystal growth rates from amorphous felodipine"; CrystEngComm, 2010, 12, 2390 - 2397]. If present, the total amount of polymers such as especially PCI (with respect to the total weight of the pharmaceutical composition) is below about 15 ww%, notably it is about 4 to 7 ww%. Examples of commonly used polymeric crystallization inhibitors (PCI) are cellulose derivatives with different molecular weights and different substitution such as hydroxy propyl methyl cellulose (HPMC), methylcellulose (MC), hydroxypropyl methyl cellulose acetate succinate (HPMC-AS), cellulose acetate butyrates (CAB), hydroxypropyl cellulose (HPC), polyvinyl acetate (PVA), or especially polyvinyl pyrrolidone (PVP) derivatives with different molecular weights. Preferably no polymers are used in the present compositions.

Further examples of such conventional ingredients or additives are acids. Acids may be used to (partly) protonate the basic nitrogen of the active ingredient molecule and, thus, also may enhance chemical stability of the active ingredient in the composition. Examples of such acids are citric acid, propionic acid and lactic acid. If present, the total weight of acids (with respect to the total weight of the mixture of excipients) is below about 5 ww%, notably it is about 0 to 2 ww%. Preferably no acid is present.

The absolute amounts of each pharmaceutically acceptable excipient and the amounts relative to other pharmaceutically acceptable excipients are dependent on the desired properties of the capsule and can be chosen by routine experimentation.

The term "hydrophilic co-solvent" refers one or more hydrophilic co-solvents that may be used in the mixture of excipients such as water, or preferably hydrophilic organic solvents such as especially triethyl citrate (e.g. Citrofol^{®} Al), ethanol, or diethylene glycol monoethylether (e.g. Transcutol^{®} HP); or in addition, dimethylacetamide (DMA), dimethylsulfoxide (DMSO), glyceryl triacetate (triacetin), N-methyl-pyrrolidinone (NMP), dimethylisosorbide (DMI), or 1,2-propandiol (propylene glycol). In addition, the present compositions may contain liquid or semi-solid polyethyleneglycols as hydrophilic co-solvents [such as for example PEG 300 or PEG 400], used alone or in combination with one or more other hydrophilic co-solvents as defined before. Preferred hydrophilic co-solvents are triethyl citrate (e.g. Citrofol^{®} Al), ethanol, and diethylene glycol monoethylether (e.g. Transcutol^{®} HP), or any mixture thereof. Most preferred is triethyl citrate.

The total amount of hydrophilic co-solvent as added to the composition is about 0 to 25 ww%. Notably, if a hydrophilic co-solvent is added, about 10 to 25 ww% (especially about 20 ww%) of hydrophilic co-solvent is added; wherein said hydrophilic co-solvent is an organic solvent as defined before, especially triethyl citrate.

If present, the total amount of water and/or ethanol as added to the mixture of excipients, in this particular case accumulated with residual water and/or ethanol which may be contained in the surfactants part of the mixture of excipients, is below about 20 ww%. For avoidance of any doubt, for example in a particular case where the hydrophilic co-solvents water and/or ethanol are present in the mixture of excipients in an amount of about 20 ww%, the surfactants of the mixture of excipients may not contain further residual water and/or ethanol.

The total weight per cent (ww%) of the pharmaceutical composition is 100.

Unless used regarding temperatures, the term "about" placed before a numerical value "X" refers in the current application to an interval extending from X minus 10% of X to X plus 10% of X, and preferably to an interval extending from X minus 5% of X to X plus 5% of X (wherein it is well understood that values below 0%, respectively higher than 100%, are not applicable). In case the term about is placed before a range, the respective interval is to be applied to both values of the range. In the particular case of temperatures, the term "about" placed before a temperature "Y" refers in the current application to an interval extending from the temperature Y minus 10 °C to Y plus 10 °C; and preferably, in case the temperature is at least 30 °C to an interval extending from Y minus 5 °C to Y plus 5 °C; or, in case the temperature is below 30 °C, to an interval extending from Y minus 2 °C to Y plus 2 °C. In case such temperature relates to a melting point, the term "about" refers preferably to an interval extending from Y minus 3 °C to Y plus 3 °C. Room temperature means a temperature of about 25 °C. When in the current application the term n equivalent(s) is used wherein n is a number, it is meant and within the scope of the current application that n is referring to about the number n, preferably n is referring to the exact number n.

Whenever the word "between" or "to" is used to describe a numerical range, it is to be understood that the end points of the indicated range are explicitly included in the range. For example: if a temperature range is described to be between 40°C and 80°C (or 40°C to 80°C), this means that the end points 40°C and 80°C are included in the range; or if a variable is defined as being an integer between 1 and 4 (or 1 to 4), this means that the variable is the integer 1, 2, 3, or 4.

The term "consisting essentially of" is understood in the context of the present invention to mean especially that the respective composition consists in an amount of at least 90, notably of at least 95, especially of at least 99, and preferably in an amount of 100 per cent by weight (i.e. in the meaning of "consisting of") of the respective composition in the amounts as explicitly stated in the respective embodiment. The term "comprising" is preferably to be understood in the meaning of the term "consisting essentially of".

The term "essentially", for example when used in a term such as "essentially pure" is understood in the context of the present invention to mean especially that the respective composition / compound etc. consists in an amount of at least 90, especially of at least 95, and notably of at least 99 per cent by weight of the respective pure composition / compound etc..

When defining the presence of a peak in e.g. an X-ray powder diffraction diagram, a common approach is to do this in terms of the S/N ratio (S = signal, N = noise). According to this definition, when stating that a peak has to be present in an X-ray powder diffraction diagram, it is understood that the peak in the X-ray powder diffraction diagram is defined by having an S/N ratio (S = signal, N = noise) of greater than x (x being a numerical value greater than 1), usually greater than 2, especially greater than 3.

In the context with stating that the crystalline form essentially shows an X-ray powder diffraction pattern as depicted in Fig. 1, respectively, the term "essentially" means that at least the major peaks of the diagram depicted in said figures, i.e. those having a relative intensity of more than 10%, especially more than 20%, as compared to the most intense peak in the diagram, have to be present. However, the person skilled in the art of X-ray powder diffraction will recognize that relative intensities in X-ray powder diffraction diagrams may be subject to strong intensity variations due to preferred orientation effects.

The expression ww% refers to a percentage by weight compared to the total weight of the composition considered. If not explicitly stated otherwise (e.g. reference to the total weight of the mixture of excipients which is the composition without the active ingredient), the considered total weight is the total weight of the pharmaceutical composition which is the composition including the active ingredient. The expression (wt/wt) relating to a ratio refers to a ratio by weight of the respective components. It is understood that the total amount expressed in "ww%" of a certain composition is 100.

The expression (wt/wt) relating to a ratio refers to a ratio by weight of the respective components.

In case a certain value is given as % value, in absence of further specification such value refers to ww%, or if in the context of purity, area% as measured by HPLC.

Likewise, the expression v/v refers to a ratio by volume of the two components considered. The expression "vol" signifies volumes (in L, e.g. of solvent) per weight (in kg, e.g. of a reactant). For example, 7 vol signifies 7 liters (of solvent) per kg (e.g. of a reactant).

The term "solid-liquid separation" refers to routine solid-liquid separation techniques well known to a skilled person (see for example Perry's Chemical Engineers' Handbook, 7th edition, Perry, R.H.; Green, D. W. McGraw-Hill 1997). In particular, the term includes techniques such as filtration, centrifugation, and gravity sedimentation; especially filtration.

Further embodiments of the invention are presented hereinafter:
5) Another embodiment relates to a pharmaceutical composition according to any one of embodiments 1) to 4), wherein
   - said lipophilic excipient(s) is/are independently selected from hydrophobic surfactants selected from 1,2-propandiol medium chain mono-fatty acid esters; and
      said hydrophilic surfactant(s) is/are independently selected from polyethyleneglycol derivatized long chain lipids and polyethyleneglycol derivatized glycerin medium chain mono-/di-fatty acid esters; or
   - said lipophilic excipient(s) is/are independently selected from oil-like excipients selected from medium chain triglyceride oils and 1,2-propandiol medium chain di-fatty acid esters; and
      said hydrophilic surfactant(s) is/are independently selected from polyethyleneglycol derivatized long chain lipids.
6) Another embodiment relates to a pharmaceutical composition according to any one of embodiments 1) to 4), wherein
   - said lipophilic excipient(s) is/are independently selected from hydrophobic surfactants selected from 1,2-propandiol medium chain mono-fatty acid esters; and
      said hydrophilic surfactant(s) is/are independently selected from polyethyleneglycol derivatized long chain lipids; or
   - said lipophilic excipient(s) is/are independently selected from oil-like excipients selected from medium chain triglyceride oils; and
      said hydrophilic surfactant(s) is/are independently selected from polyethyleneglycol derivatized long chain lipids;
   - said lipophilic excipient(s) is/are independently selected from hydrophobic surfactants selected from 1,2-propandiol medium chain mono-fatty acid esters; and
      said hydrophilic surfactant(s) is/are independently selected from polyethyleneglycol derivatized glycerin medium chain mono-/di-fatty acid esters; or
   - said lipophilic excipient(s) is/are independently selected from oil-like excipients selected from 1,2-propandiol medium chain di-fatty acid esters; and
      said hydrophilic surfactant(s) is/are independently selected from polyethyleneglycol derivatized long chain lipids.
7) Another embodiment relates to a pharmaceutical composition according to embodiments 4), wherein
   - said lipophilic excipient is a hydrophobic surfactant which is a 1,2-propandiol medium chain mono-fatty acid ester (especially a propylene glycol mono-caprylate, in particular Capryol^{™} 90); and
      said hydrophilic surfactant is a polyethyleneglycol derivatized hydrogenated castor oil (especially PEG-40 hydrogenated castor oil, in particular Kolliphor^{®} RH40); or
   - said lipophilic excipient is a hydrophobic surfactant which is a 1,2-propandiol medium chain mono-fatty acid ester (especially a propylene glycol mono-caprylate, in particular Capryol^{™} 90); and
      said hydrophilic surfactant is a polyethyleneglycol derivatized castor oil (especially PEG-35 castor oil, in particular Kolliphor^{®} EL); or
   - said lipophilic excipient is a hydrophobic surfactant which is a 1,2-propandiol medium chain mono-fatty acid ester (especially a propylene glycol mono-caprylate, in particular Capryol^{™} 90); and
      said hydrophilic surfactant is a polyethyleneglycol derivatized glycerin medium chain mono-/di-fatty acid ester (especially a PEG-8 glycerin mono- and diester of caprylic and/or capric acid, in particular Labrasol^{®} ALF); or
   - said lipophilic excipient is an oil-like which is a glycerin medium chain tri-fatty acid ester (especially glyceryl tri-caprylate/caprate, in particular Miglyol^{®}812); and
      said hydrophilic surfactant is a polyethyleneglycol derivatized hydrogenated castor oil (especially PEG-40 hydrogenated castor oil, in particular Kolliphor^{®} RH40);
   - said lipophilic excipient is an oil-like which is a glycerin medium chain tri-fatty acid ester (especially glyceryl tri-caprylate/caprate, in particular Miglyol^{®}812); and
      said hydrophilic surfactant is a polyethyleneglycol derivatized castor oil (especially PEG-35 castor oil, in particular Kolliphor^{®} EL); or
   - said lipophilic excipient is an oil-like which is a 1,2-propandiol medium chain di-fatty acid ester (especially propyleneglycol di-caprylate/di-caprate, in particular Labrafac^{™} PG); and
      said hydrophilic surfactant is a polyethyleneglycol derivatized castor oil (especially PEG-35 castor oil, in particular Kolliphor^{®} EL).
8) Another embodiment relates to a pharmaceutical composition according to embodiments 4), wherein said lipophilic excipient is a hydrophobic surfactant which is a 1,2-propandiol medium chain mono-fatty acid ester (especially a propylene glycol mono-caprylate, in particular Capryol^{™} 90); and said hydrophilic surfactant is a polyethyleneglycol derivatized hydrogenated castor oil (especially PEG-40 hydrogenated castor oil, in particular Kolliphor^{®} RH40).
9) A third aspect of the invention relates to pharmaceutical compositions according to any one of embodiments 1) to 8), comprising:
   - a total amount of about 0.05 to 5 ww% (notably a total amount of about 0.075 to 4.5 ww%, especially about 0.075 to 3 ww%, in particular about 0.075 ww%, 0.15 ww%, 0.75 ww%, 1.5 ww%, or 3 ww%) (based on the total weight of the pharmaceutical composition) of COMPOUND; wherein COMPOUND is preferably in free base form; or in a pharmaceutically acceptable salt form; and
   - a total amount of at least about 80 ww% (especially of at least about 90 ww%) [based on the total weight of the pharmaceutical composition] of a mixture of excipients as defined in any one of embodiments 1) to 8);
   wherein the total ww% of the pharmaceutical composition is 100.

For avoidance of doubt, the ww% of active ingredient given for the pharmaceutical composition of embodiment 9) (and *mutatis mutandis* for embodiments 10) to 36) below) correspond notably to a drug load of 0.5 mg to 30 mg, preferably 0.5 mg to 20 mg of COMPOUND in free base form (e.g. COMPOUND can be used in anhydrous crystalline or amorphous form), per capsule (e.g. a gelatin capsule of size 5 to 16, especially size 8.5, 10 or 12) (in particular 0.5 mg, 1 mg, 5 mg, 10 mg, or 20 mg). In case COMPOUND is used in form of a salt or in form of a solvate or cocrystal, the ww% of active ingredient are to be understood as referring to the active ingredient in the respective form that is actually used, i.e. to the salt, to a hydrate crystalline form, to a cocrystal form. The respective ww% needed to reach a certain drug load (0.5 mg, 1 mg, etc.) may therefore vary depending on the form in which the active ingredient used. Preferably COMPOUND in (anhydrous) free base form is used.

It is further understood that the pharmaceutical compositions as defined herein may, if not explicitly stated otherwise, additionally comprise conventional ingredients or additives *(quantum satis,* i.e. wherein the amounts of the mixture of excipients may need to be adjusted to the amount of said conventional ingredients or additives present in the pharmaceutical composition to make up the total ww% of 100 of the pharmaceutical composition). Preferably the total amount of such additional conventional ingredients or additives is 0 ww% to a total maximum of about 5 ww% (especially 0 ww% to a total of about 2 ww%), or, in case polymers such as PCI are present, 0 ww% to a total maximum of about 20 ww%.

Thus, where such pharmaceutical composition of embodiment 9) [and, likewise, 10) to 36) below] comprises a total amount of about 0.05 to 5 ww% (notably a total amount of about 0.075 to 4.5 ww%, especially about 0.075 to 3 ww%, in particular about 0.075 ww%, 0.15 ww%, 0.75 ww%, 1.5 ww%, or 3 ww%) (based on the total weight of the pharmaceutical composition) of COMPOUND, preferably the sum in ww% of the total amount of said mixture of excipients, and, if present, of the total amount of said additional conventional ingredients or additives in ww% is 100 ww% minus the respective ww% of COMPOUND [in particular about 95 to 99.95 ww% (notably about 94.5 to 99.925 ww%, especially about 97 to 99.925 ww%, in particular about 99.925 ww%, 99.85 ww%, 99.25 ww%, 98.5 ww%, or 97 ww%); wherein the total ww% of the pharmaceutical composition is 100].

In an illustrative example, such pharmaceutical composition of the present invention comprises a total amount of about 0.75 ww% of COMPOUND, an oxygen scavenger in a total amount of about 1 ww%, and a chain terminator in a total amount of about 0.05 ww% (and no further additional conventional ingredients or additives are present, thus, the total amount of said additional conventional ingredients or additives is about 1.05 ww%). In consequence, the resulting total amount of said mixture of excipients according to embodiment 9) is about (100 - 0.75) - 1.05)) ww% = 98.2 ww%.

10) Another embodiment relates to a pharmaceutical composition according to embodiment 1), comprising:
- a total amount of about 0.05 to 5 ww% (notably a total amount of about 0.075 to 4.5 ww%, especially about 0.075 to 3 ww%, in particular about 0.075 ww%, 0.15 ww%, 0.75 ww%, 1.5 ww%, or 3 ww%) (based on the total weight of the pharmaceutical composition) of COMPOUND; wherein COMPOUND is preferably in free base form; or in a pharmaceutically acceptable salt form; and
- a total amount of at least about 80 ww% (especially of at least about 90 ww%) (based on the total weight of the pharmaceutical composition) of a mixture of excipients; wherein said mixture of excipients comprises:
   > a total of about 20 to 50 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 30 to 80 ww% of a hydrophilic surfactant, wherein said hydrophilic surfactant is a polyethyleneglycol derivatized hydrogenated castor oil (especially PEG-40 hydrogenated castor oil, in particular Kolliphor^{®} RH40); and
      a total of about 0 to 25 ww% of one or two hydrophilic co-solvents;
   > a total of about 20 to 50 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 30 to 80 ww% of a hydrophilic surfactant, wherein said hydrophilic surfactant is a polyethyleneglycol derivatized castor oil (especially PEG-35 castor oil, in particular Kolliphor^{®} EL); and
      a total of about 0 to 25 ww% of one or two hydrophilic co-solvents;
   > a total of about 20 to 50 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 30 to 80 ww% of a hydrophilic surfactant, wherein said hydrophilic surfactant is a polyethyleneglycol derivatized glycerin medium chain mono-/di-fatty acid ester (especially a PEG-8 glycerin mono- and diester of caprylic and/or capric acid, in particular Labrasol^{®} ALF); and
      a total of about 0 to 25 ww% of one or two hydrophilic co-solvents;
   > a total of about 20 to 50 ww% of a lipophilic excipient, wherein said lipophilic excipient is a glycerin medium chain tri-fatty acid ester (especially glyceryl tri-caprylate/caprate, in particular Miglyol^{®}812);
      a total of about 30 to 80 ww% of a hydrophilic surfactant, wherein said hydrophilic surfactant is a polyethyleneglycol derivatized hydrogenated castor oil (especially PEG-40 hydrogenated castor oil, in particular Kolliphor^{®} RH40); and
      a total of about 0 to 25 ww% of one or two hydrophilic co-solvents;
   > a total of about 20 to 50 ww% of a lipophilic excipient, wherein said lipophilic excipient is a glycerin medium chain tri-fatty acid ester (especially glyceryl tri-caprylate/caprate, in particular Miglyol^{®}812);
      a total of about 30 to 80 ww% of a hydrophilic surfactant, wherein said hydrophilic surfactant is a polyethyleneglycol derivatized castor oil (especially PEG-35 castor oil, in particular Kolliphor^{®} EL); and
      a total of about 0 to 25 ww% of one or two hydrophilic co-solvents; or
   > a total of about 20 to 50 ww% of a lipophilic excipient, wherein said lipophilic excipient is 1,2-propandiol medium chain di-fatty acid ester (especially propyleneglycol di-caprylate/di-caprate, in particular Labrafac^{™} PG);
      a total of about 30 to 80 ww% of a hydrophilic surfactant, wherein said hydrophilic surfactant is a polyethyleneglycol derivatized castor oil (especially PEG-35 castor oil, in particular Kolliphor^{®} EL); and
      a total of about 0 to 25 ww% of one or two hydrophilic co-solvents;
      wherein the total ww% of said mixture of excipients is 100; and
   wherein the total ww% of the pharmaceutical composition is 100.

11) Another embodiment relates to a pharmaceutical composition according to embodiment 1), comprising:
- a total amount of about 0.075 to 4.5 ww% (especially a total amount of about 0.075 to 3 ww%, in particular about 0.075 ww%, 0.15 ww%, 0.75 ww%, 1.5 ww%, or 3 ww%) (based on the total weight of the pharmaceutical composition) of COMPOUND; wherein COMPOUND is preferably in free base form; or in a pharmaceutically acceptable salt form; and
- a total amount of at least about 80 ww% (especially of at least about 90 ww%) (based on the total weight of the pharmaceutical composition) of a mixture of excipients; wherein said mixture of excipients comprises:
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 40 to 70 ww% of a hydrophilic surfactant, wherein said hydrophilic surfactant is a polyethyleneglycol derivatized hydrogenated castor oil (especially PEG-40 hydrogenated castor oil, in particular Kolliphor^{®} RH40); and
      no hydrophilic co-solvent; or preferably a total of about 10 to 20 ww% of one or two hydrophilic co-solvents; especially selected from triethyl citrate, ethanol, and diethylene glycol monoethylether;
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 40 to 70 ww% of a hydrophilic surfactant, wherein said hydrophilic surfactant is a polyethyleneglycol derivatized castor oil (especially PEG-35 castor oil, in particular Kolliphor^{®} EL); and
      no hydrophilic co-solvent; or preferably a total of about 10 to 20 ww% of one or two hydrophilic co-solvents; especially selected from triethyl citrate, ethanol, and diethylene glycol monoethylether;
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 40 to 70 ww% of a hydrophilic surfactant, wherein said hydrophilic surfactant is a polyethyleneglycol derivatized glycerin medium chain mono-/di-fatty acid ester (especially a PEG-8 glycerin mono- and diester of caprylic and/or capric acid, in particular Labrasol^{®} ALF); and
      no hydrophilic co-solvent; or preferably a total of about 10 to 20 ww% of one or two hydrophilic co-solvents; especially selected from triethyl citrate, ethanol, and diethylene glycol monoethylether;
   > a total of about 30 to 50 ww% of a lipophilic excipient, wherein said lipophilic excipient is a glycerin medium chain tri-fatty acid ester (especially glyceryl tri-caprylate/caprate, in particular Miglyol@812);
      a total of about 40 to 70 ww% of a hydrophilic surfactant, wherein said hydrophilic surfactant is a polyethyleneglycol derivatized castor oil (especially PEG-35 castor oil, in particular Kolliphor^{®} EL); and
      no hydrophilic co-solvent; or preferably a total of about 10 to 20 ww% of one or two hydrophilic co-solvents; especially selected from triethyl citrate, ethanol, and diethylene glycol monoethylether; or
   > a total of about 35 to 45 ww% of a lipophilic excipient, wherein said lipophilic excipient is 1,2-propandiol medium chain di-fatty acid ester (especially propyleneglycol di-caprylate/di-caprate, in particular Labrafac^{™} PG);
      a total of about 40 to 60 ww% of a hydrophilic surfactant, wherein said hydrophilic surfactant is a polyethyleneglycol derivatized castor oil (especially PEG-35 castor oil, in particular Kolliphor^{®} EL); and
      no hydrophilic co-solvent; or preferably a total of about 10 to 20 ww% of one or two hydrophilic co-solvents; especially selected from triethyl citrate, ethanol, and diethylene glycol monoethylether;
      wherein the total ww% of said mixture of excipients is 100; and
   wherein the total ww% of the pharmaceutical composition is 100.

12) Another embodiment relates to a pharmaceutical composition according to embodiment 1), comprising:
- a total amount of about 0.075 to 4.5 ww% (especially a total amount of about 0.075 to 3 ww%, in particular about 0.075 ww%, 0.15 ww%, 0.75 ww%, 1.5 ww%, or 3 ww%) (based on the total weight of the pharmaceutical composition) of COMPOUND; wherein COMPOUND is preferably in free base form; or in a pharmaceutically acceptable salt form; and
- a total amount of at least about 80 ww% (especially of at least about 90 ww%) (based on the total weight of the pharmaceutical composition) of a mixture of excipients; wherein said mixture of excipients comprises:
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 40 to 70 ww% of a hydrophilic surfactant, wherein said hydrophilic surfactant is a polyethyleneglycol derivatized hydrogenated castor oil (especially PEG-40 hydrogenated castor oil, in particular Kolliphor^{®} RH40); and
      no hydrophilic co-solvent; or preferably a total of about 10 to 20 ww% of one or two hydrophilic co-solvents selected from triethyl citrate, ethanol, and diethylene glycol monoethylether (especially 10 to 20 ww% of triethyl citrate or ethanol);
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 40 to 70 ww% of a hydrophilic surfactant, wherein said hydrophilic surfactant is a polyethyleneglycol derivatized glycerin medium chain mono-/di-fatty acid ester (especially a PEG-8 glycerin mono- and diester of caprylic and/or capric acid, in particular Labrasol^{®} ALF); and
      no hydrophilic co-solvent; or preferably a total of about 10 to 20 ww% of one or two hydrophilic co-solvents selected from triethyl citrate, ethanol, and diethylene glycol monoethylether (especially 10 to 20 ww% of triethyl citrate or ethanol);
   > a total of about 30 to 50 ww% of a lipophilic excipient, wherein said lipophilic excipient is a glycerin medium chain tri-fatty acid ester (especially glyceryl tri-caprylate/caprate, in particular Miglyol^{®}812);
      a total of about 40 to 70 ww% of a hydrophilic surfactant, wherein said hydrophilic surfactant is a polyethyleneglycol derivatized castor oil (especially PEG-35 castor oil, in particular Kolliphor^{®} EL);
         and
      no hydrophilic co-solvent; or preferably a total of about 10 to 20 ww% of one or two hydrophilic co-solvents selected from triethyl citrate, ethanol, and diethylene glycol monoethylether (especially no hydrophilic co-solvent, or 10 to 20 ww% of diethylene glycol monoethylether); or
   > a total of about 35 to 45 ww% of a lipophilic excipient, wherein said lipophilic excipient is 1,2-propandiol medium chain di-fatty acid ester (especially propyleneglycol di-caprylate/di-caprate, in particular Labrafac^{™} PG);
      a total of about 40 to 60 ww% of a hydrophilic surfactant, wherein said hydrophilic surfactant is a polyethyleneglycol derivatized castor oil (especially PEG-35 castor oil, in particular Kolliphor^{®} EL); and
      no hydrophilic co-solvent; or preferably a total of about 10 to 20 ww% of one or two hydrophilic co-solvents selected from triethyl citrate, ethanol, and diethylene glycol monoethylether (especially no hydrophilic co-solvent, or 10 to 20 ww% of ethanol or diethylene glycol monoethylether);
      wherein the total ww% of said mixture of excipients is 100; and
   wherein the total ww% of the pharmaceutical composition is 100.

13) Another embodiment relates to a pharmaceutical composition according to embodiment 1), comprising:
- a total amount of about 0.075 to 4.5 ww% (especially a total amount of about 0.075 to 3 ww%, in particular about 0.075 ww%, 0.15 ww%, 0.75 ww%, 1.5 ww%, or 3 ww%) (based on the total weight of the pharmaceutical composition) of COMPOUND; wherein COMPOUND is preferably in free base form; or in a pharmaceutically acceptable salt form; and
- a total amount of at least about 80 ww% (especially of at least about 90 ww%) (based on the total weight of the pharmaceutical composition) of a mixture of excipients; wherein said mixture of excipients comprises:
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 40 to 70 ww% of a hydrophilic surfactant, wherein said hydrophilic surfactant is a polyethyleneglycol derivatized hydrogenated castor oil (especially PEG-40 hydrogenated castor oil, in particular Kolliphor^{®} RH40); and
      no hydrophilic co-solvent; or preferably a total of about 10 to 20 ww% of one or two hydrophilic co-solvents selected from triethyl citrate, ethanol, and diethylene glycol monoethylether (especially 10 to 20 ww% of triethyl citrate or ethanol); or
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{®} 90);
      a total of about 40 to 70 ww% of a hydrophilic surfactant, wherein said hydrophilic surfactant is a polyethyleneglycol derivatized glycerin medium chain mono-/di-fatty acid ester (especially a PEG-8 glycerin mono- and diester of caprylic and/or capric acid, in particular Labrasol^{®} ALF); and
      no hydrophilic co-solvent; or preferably a total of about 10 to 20 ww% of one or two hydrophilic co-solvents selected from triethyl citrate, ethanol, and diethylene glycol monoethylether (especially 10 to 20 ww% of triethyl citrate or ethanol);
      wherein the total ww% of said mixture of excipients is 100; and
   wherein the total ww% of the pharmaceutical composition is 100.

14) Another embodiment relates to a pharmaceutical composition according to embodiment 1), comprising:
- a total amount of about 0.075 to 4.5 ww% (especially a total amount of about 0.075 to 3 ww%, in particular about 0.075 ww%, 0.15 ww%, 0.75 ww%, 1.5 ww%, or 3 ww%) (based on the total weight of the pharmaceutical composition) of COMPOUND; wherein COMPOUND is preferably in free base form; or in a pharmaceutically acceptable salt form; and
- a total amount of at least about 80 ww% (especially of at least about 90 ww%) (based on the total weight of the pharmaceutical composition) of a mixture of excipients; wherein said mixture of excipients comprises:
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 40 to 70 ww% of a hydrophilic surfactant, wherein said hydrophilic surfactant is a polyethyleneglycol derivatized hydrogenated castor oil (especially PEG-40 hydrogenated castor oil, in particular Kolliphor^{®} RH40); and
      no hydrophilic co-solvent; or preferably a total of about 10 to 20 ww% of one or two hydrophilic co-solvents selected from triethyl citrate, ethanol, and diethylene glycol monoethylether (especially 10 to 20 ww% of triethyl citrate or ethanol);
      wherein the total ww% of said mixture of excipients is 100; and
   wherein the total ww% of the pharmaceutical composition is 100.

15) Another embodiment relates to a pharmaceutical composition according to any one of embodiments 1) to 11), wherein said mixture of excipients comprises no hydrophilic co-solvent, or said mixture of excipients comprises one, or two hydrophilic co-solvents, wherein said hydrophilic co-solvent(s) is/are independently selected from triethyl citrate, ethanol, diethylene glycol monoethylether, dimethylacetamide (DMA), dimethylsulfoxide (DMSO), glyceryl triacetate (triacetin), N-methyl-pyrrolidinone (NMP), dimethylisosorbide (DMI), 1,2-propandiol (propylene glycol) and liquid or semi-solid polyethyleneglycol.

16) Another embodiment relates to a pharmaceutical composition according to any one of embodiments 1) to 11), wherein said mixture of excipients comprises no hydrophilic co-solvent, or said mixture of excipients comprises one or two hydrophilic co-solvents (especially one hydrophilic co-solvent), wherein said hydrophilic co-solvent(s) is/are independently selected from triethyl citrate, ethanol, and diethylene glycol monoethylether.

17) Another embodiment relates to a pharmaceutical composition according to any one of embodiments 1) to 14), wherein said mixture of excipients comprises no hydrophilic co-solvent.

18) Another embodiment relates to a pharmaceutical composition according to any one of embodiments 1) to 14), wherein said mixture of excipients comprises one hydrophilic co-solvent which is triethyl citrate, ethanol, or diethylene glycol monoethylether; especially triethyl citrate.

19) Another embodiment relates to a pharmaceutical composition according to embodiment 1), consisting essentially of:
- a total amount of about 0.075 to 4.5 ww% (especially a total amount of about 0.075 to 3 ww%, in particular about 0.075 ww%, 0.15 ww%, 0.75 ww%, 1.5 ww%, or 3 ww%) (based on the total weight of the pharmaceutical composition) of COMPOUND; wherein COMPOUND is preferably in free base form; or in a pharmaceutically acceptable salt form; and
- a total amount of at least about 80 ww% (especially of at least about 90 ww%) (based on the total weight of the pharmaceutical composition) of a mixture of excipients; wherein said mixture of excipients comprises:
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 40 to 70 ww% of a hydrophilic surfactant, wherein said hydrophilic surfactant is a polyethyleneglycol derivatized hydrogenated castor oil (especially PEG-40 hydrogenated castor oil, in particular Kolliphor^{®} RH40); and
      no hydrophilic co-solvent; or preferably a total of about 10 to 20 ww% of one or two hydrophilic co-solvents selected from triethyl citrate, ethanol, and diethylene glycol monoethylether (especially 10 to 20 ww% of triethyl citrate or ethanol); or
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 40 to 70 ww% of a hydrophilic surfactant, wherein said hydrophilic surfactant is a polyethyleneglycol derivatized glycerin medium chain mono-/di-fatty acid ester (especially a PEG-8 glycerin mono- and diester of caprylic and/or capric acid, in particular Labrasol^{®} ALF); and
      no hydrophilic co-solvent; or preferably a total of about 10 to 20 ww% of one or two hydrophilic co-solvents selected from triethyl citrate, ethanol, and diethylene glycol monoethylether (especially 10 to 20 ww% of triethyl citrate or ethanol);
      wherein the total ww% of said mixture of excipients is 100; and
- optionally one or more additional conventional ingredients or additives selected from one or more antioxidants, one or more polymeric crystallization inhibitors, one or more acids, and/or one or more chelating agents (especially one or two antioxidants, one polymeric crystallization inhibitor, and/or one chelating agent, in particular one or two antioxidants which are one oxygen scavenger and/or one chain terminator);

wherein the total ww% of the pharmaceutical composition is 100;
[wherein preferably the sum of (the total amount of said mixture of excipients in ww% and, if present, of the total amount of said conventional ingredients or additives in ww%) is equal to (100 ww% minus the respective ww% of COMPOUND); thus, the sum of the total amount of said mixture of excipients and of the total amount of said one or two antioxidants is notably about 95.5 to 99.925 ww%; especially of about 97 to 99.925 ww%, in particular about 99.925 ww%, 99.85 ww%, 99.25 ww%, 98.5 ww%, or 97 ww%].

20) Another embodiment relates to a pharmaceutical composition according to embodiment 1), consisting essentially of:
- a total amount of about 0.075 to 4.5 ww% (especially a total amount of about 0.075 to 3 ww%, in particular about 0.075 ww%, 0.15 ww%, 0.75 ww%, 1.5 ww%, or 3 ww%) (based on the total weight of the pharmaceutical composition) of COMPOUND; wherein COMPOUND is preferably in free base form; or in a pharmaceutically acceptable salt form; and
- a total amount of at least about 80 ww% (especially of at least about 90 ww%) (based on the total weight of the pharmaceutical composition) of a mixture of excipients; wherein said mixture of excipients comprises:
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 40 to 70 ww% of a hydrophilic surfactant, wherein said hydrophilic surfactant is a polyethyleneglycol derivatized hydrogenated castor oil (especially PEG-40 hydrogenated castor oil, in particular Kolliphor^{®} RH40); and
      no hydrophilic co-solvent; or preferably a total of about 10 to 20 ww% of one or two hydrophilic co-solvents selected from triethyl citrate, ethanol, and diethylene glycol monoethylether (especially 10 to 20 ww% of triethyl citrate or ethanol);
      wherein the total ww% of said mixture of excipients is 100; and
- optionally one or more additional conventional ingredients or additives selected from one or more antioxidants, one or more polymeric crystallization inhibitors, one or more acids, and/or one or more chelating agents (especially one or more antioxidants, one polymeric crystallization inhibitor, and/or one chelating agent, in particular one or two antioxidants which are one oxygen scavenger and/or one chain terminator);
   wherein the total ww% of the pharmaceutical composition is 100;
   [wherein preferably the sum of (the total amount of said mixture of excipients in ww% and, if present, of the total amount of said conventional ingredients or additives in ww%) is equal to (100 ww% minus the respective ww% of COMPOUND); thus, the sum of the total amount of said mixture of excipients and of the total amount of said one or two antioxidants is notably about 95.5 to 99.925 ww%; especially of about 97 to 99.925 ww%, in particular about 99.925 ww%, 99.85 ww%, 99.25 ww%, 98.5 ww%, or 97 ww%].

21) Another embodiment relates to a pharmaceutical composition according to embodiment 1), consisting essentially of:
- a total amount of about 0.075 to 4.5 ww% (especially a total amount of about 0.075 to 3 ww%, in particular about 0.075 ww%, 0.15 ww%, 0.75 ww%, 1.5 ww%, or 3 ww%) (based on the total weight of the pharmaceutical composition) of COMPOUND; wherein COMPOUND is preferably in free base form; or in a pharmaceutically acceptable salt form; and
- a total amount of at least about 90 ww% (based on the total weight of the pharmaceutical composition) of a mixture of excipients; wherein said mixture of excipients comprises:
   > a total of about 20 to 40 ww% of a lipophilic excipient, wherein said lipophilic excipient is a 1,2-propandiol medium chain mono-fatty acid ester (especially a propylene glycol mono-caprylate, in particular Capryol^{™} 90);
      a total of about 40 to 70 ww% of a hydrophilic surfactant, wherein said hydrophilic surfactant is a polyethyleneglycol derivatized hydrogenated castor oil (especially PEG-40 hydrogenated castor oil, in particular Kolliphor^{®} RH40); and
      no hydrophilic co-solvent; or preferably a total of about 10 to 20 ww% of one or two hydrophilic co-solvents selected from triethyl citrate, ethanol, and diethylene glycol monoethylether (especially 10 to 20 ww% of triethyl citrate or ethanol);
      wherein the total ww% of said mixture of excipients is 100; and
- optionally one or two conventional ingredients or additives selected from one or two antioxidants selected from
   > one oxygen scavenger in an amount of below about 2 ww% (based on the total weight of the pharmaceutical composition) (notably about 0.1-1 ww%, especially about 0.5 to 1 ww%, in particular about 1 ww%), and/or
   > one chain terminator in an amount of below about 0.3 ww% (based on the total weight of the pharmaceutical composition) (notably about 0.05-0.2 ww%, especially about 0.05 to 0.1 ww%, in particular about 0.05 ww% or 0.1 ww%);

[wherein preferably the sum of (the total amount of said mixture of excipients in ww% and, if present, of the total amount of said conventional ingredients or additives in ww%) is equal to (100 ww% minus the respective ww% of COMPOUND); thus, the sum of the total amount of said mixture of excipients and of the total amount of said one or two antioxidants is notably about 95.5 to 99.925 ww%; especially of about 97 to 99.925 ww%, in particular about 99.925 ww%, 99.85 ww%, 99.25 ww%, 98.5 ww%, or 97 ww%];
wherein the total ww% of the pharmaceutical composition is 100.

22) In another embodiment, the invention relates to a pharmaceutical composition according to any one of embodiment 1) to 21), wherein said pharmaceutical composition consists of
- the COMPOUND,
- said mixture of excipients (according to any one of embodiment 1) to 21)), and
- optionally one or more conventional ingredients or additives (according to any one of embodiments
   1) to 21) or according to any one of embodiments 23) to 28) below);
      wherein
      > the total amount of COMPOUND is about 0.07 - 0.08 ww%; or
      > the total amount of COMPOUND is about 0.14 - 0.16 ww%; or
      > the total amount of COMPOUND is about 0.7 - 0.8 ww%; or
      > the total amount of COMPOUND is about 1.4 -1.6 ww%; or
      > the total amount of COMPOUND is about 2.8 - 3.2 ww%;
      [wherein it is understood that, in consequence, the sum in ww% of:
      i. the total amount of said mixture of excipients in ww%, and
      ii. if present, the total amount of said one or more conventional ingredients or additives is 100 ww% minus the respective ww% of COMPOUND as defined in this embodiment 22); thus, said sum is, respectively, about 99.92 - 99.93 ww%; or about 99.84 - 99.86 ww%; or about 99.2 - 99.3 ww%; or about 98.4 - 98.6 ww%; or about 96.8 - 97.2 ww%];
   wherein the total ww% of the pharmaceutical composition is 100.

The amounts given in embodiment 22) are intended to achieve a drug load of 0.5 mg, 1 mg, 5 mg, 10 mg, or, respectively, 20 mg of COMPOUND in free base form, wherein especially COMPOUND in crystalline free base form according to any one of embodiments 37) to 42) below is used for the preparation of such composition.

In the following, further embodiments relating to the compositions of embodiments 1) to 22) are given. For avoidance of any doubt, it is well understood that the pharmaceutical compositions as defined in embodiments 1) to 22) may additionally comprise conventional ingredients or additives, wherein said conventional ingredients or additives are selected from one or more polymers including polymeric crystallization inhibitors, one or more antioxidants, one or more acids, and/or one or more chelating agents except explicitly stated otherwise *(quantum satis,* i.e. wherein the maximum amounts of the mixture of excipients may need to be adjusted to the amount of said polymers, acids, and/or antioxidants to make up the total ww% of 100 of the pharmaceutical composition, in analogy to the example given in embodiment 9)).

23) Another embodiment relates to a pharmaceutical composition according to any one of embodiments 1) to 22), wherein said pharmaceutical composition further comprises additional conventional ingredients or additives, wherein said conventional ingredients or additives are especially selected from one or more polymers which is /are polymeric crystallization inhibitors, one or more antioxidants, one or more acids, and/or one or more chelating agents.

In a sub-embodiment said pharmaceutical composition consists essentially of the COMPOUND, the mixture of excipients as explicitly defined (i.e. it does not comprise any conventional ingredient(s) or additive(s)). In another sub-embodiment said pharmaceutical composition preferably consists essentially of the COMPOUND, the mixture of excipients as explicitly defined, and, in addition, conventional ingredients or additives selected from one or more polymers which is /are polymeric crystallization inhibitors, one or more antioxidants, one or more acids, and/or one or more chelating agents (especially one or two antioxidants and optionally a chelating agent) (i.e. other than said conventional ingredients or additives, especially one or two antioxidants and optional chelating agent, said pharmaceutical composition does not comprise any further conventional ingredient(s) or additive(s)).

24) In another embodiment, the invention relates to a pharmaceutical composition according to any one of embodiments 1) to 22), wherein said pharmaceutical composition comprises
- no chelating agent; no acid; no polymer; and no polymeric crystallization inhibitor (PCI); and
- no antioxidants, or one or two antioxidants.

25) Another embodiment relates to a pharmaceutical composition according to any one of embodiments 1) to 24), wherein said pharmaceutical composition comprises a maximum of one or two antioxidants (especially one oxygen scavenger and/or one chain terminator).

In a sub-embodiment said pharmaceutical composition consists of the COMPOUND, the mixture of excipients as explicitly defined, and, in addition, one or two antioxidants (especially one oxygen scavenger and/or one chain terminator); i.e. other than said antioxidants, it does not contain any further conventional ingredient(s) or additive(s).

26) In another embodiment, the invention relates to a pharmaceutical composition according to any one of embodiments 1) to 25), wherein said pharmaceutical composition comprises
- one oxygen scavenger; or
- a mixture of one oxygen scavenger and one chain terminator.

27) In another embodiment, the invention relates to a pharmaceutical composition according to any one of embodiments 1) to 26), wherein, if present, said oxygen scavenger (which preferably is ascorbyl palmitate) is present with respect to the total weight of the pharmaceutical composition in an amount of below about 2 ww% (notably about 0.1-1 ww%, especially about 0.5 to 1 ww%, in particular about 1 ww%).

28) In another embodiment, the invention relates to a pharmaceutical composition according to any one of embodiments 1) to 27), wherein, if present, a chain terminator (which preferably is DL-alpha-Tocopheryl acetate, propyl gallate, BHT, or BHA, especially propyl gallate or DL-alpha-Tocopheryl acetate) is present with respect to the total weight of the pharmaceutical composition in an amount of below about 0.3 ww% (notably about 0.05-0.2 ww%, especially about 0.05 to 0.1 ww%, in particular about 0.05 ww% or 0.1 ww%).

29) In another embodiment, the invention relates to a pharmaceutical composition according to any one of embodiments 1) to 28), which is filled into capsules (especially soft gelatine capsules, in particular soft gelatine capsules of size 5 to 16, in particular size 8.5, 10, or 12).

30) In another embodiment, the invention relates to a pharmaceutical composition according to embodiment 29), wherein said capsules are filled under an inert gas atmosphere (such as especially a nitrogen atmosphere, or an argon atmosphere).

31) In another embodiment, the invention relates to a pharmaceutical composition according to embodiments 29) or 30), wherein the pharmaceutical composition is filled into capsules, especially into soft gelatine capsules, at a temperature between room temperature and about 50 °C (notably between about 30 to 40 °C, especially at about 35 °C).

32) In another embodiment, the invention relates to a pharmaceutical composition according to any one of embodiments 1) to 28), wherein the pharmaceutical composition is a liquid at a temperature between room temperature and about 60 °C (notably between about 30 to 50 °C, especially at about 35 °C).

33) In another embodiment, the invention relates to a pharmaceutical composition according to any one of embodiments 1) to 32), wherein, in case the pharmaceutical composition is filled into soft gelatine capsules, said pharmaceutical composition is filled into said soft gelatine capsules at a temperature between room temperature and about 40°C (especially between about 30°C and 38°C, notably at about 35°C); and the pharmaceutical composition is a liquid at a temperature between room temperature and about 40°C (notably between about 30°C and 38°C, especially at about 35°C).

34) In a fourth aspect, the invention relates to a pharmaceutical composition according to any one of embodiments 1) to 33), wherein said COMPOUND is used in crystalline form, especially in essentially pure crystalline form, for the preparation of said composition.

It is understood, that the crystalline form used according to embodiment 34) comprises COMPOUND in a crystalline form which can be a crystalline form of the COMPOUND in free base form; a crystalline form of the COMPOUND in free base form wherein said crystalline form is a cocrystal, or a crystalline form of the COMPOUND in form of a pharmaceutically acceptable salt, or a solvate of any of such forms. Furthermore, said crystalline forms may comprise non-coordinated and / or coordinated solvent. Coordinated solvent is used herein as term for a crystalline solvate. Likewise, non-coordinated solvent is used herein as term for physiosorbed or physically entrapped solvent (definitions according to Polymorphism in the Pharmaceutical Industry (Ed. R. Hilfiker, VCH, 2006), Chapter 8: U.J. Griesser: The Importance of Solvates). Such crystalline form may be especially an anhydrate, i.e. it comprises no significant amounts of coordinated water; or a hydrate (such as a hemihydrate, a mono-hydrate, or a dihydrate), i.e. it comprises for example about 0.5 to 2 equivalents of coordinated water (such as notably 0.5, 1, or 2 eq. of water), and may comprise additional non-coordinated solvent such as isopropanol, ethanol and / or water, especially water.

35) In another embodiment, the invention relates to a pharmaceutical composition according to any one of embodiments 1) to 34), wherein the COMPOUND in free base form is used for the preparation of said composition.

36) In another embodiment, the invention relates to a pharmaceutical composition according to any one of embodiments 1) to 34), wherein said COMPOUND in free base form is used in crystalline form, especially in essentially pure crystalline form, for the preparation of said composition.

In a preferred sub-embodiment, such crystalline form of the COMPOUND in free base form according to embodiment 36) is an anhydrate.

In another sub-embodiment, such crystalline form of the COMPOUND in free base form according to embodiment 36) is a hydrate containing about 0.5 to 2 eq. (especially about 1 eq.) of coordinated water.

37) In fourth aspect, the invention relates to a crystalline form of COMPOUND, characterized by the presence of peaks in the X-ray powder diffraction diagram at the following angles of refraction 2θ: 6.2°, 9.5°, and 14.4°.

38) Another embodiment relates to a crystalline form of COMPOUND according to embodiment 37), characterized by the presence of peaks in the X-ray powder diffraction diagram at the following angles of refraction 2θ: 6.2°, 9.5°, 14.4°, 15.7°, and 18.6°.

39) Another embodiment relates to a crystalline form of COMPOUND according to embodiment 37), characterized by the presence of peaks in the X-ray powder diffraction diagram at the following angles of refraction 2θ: 6.2°, 9.5°, 13.9°, 14.4°, 15.3°, 15.7°, 18.6°, 20.0°, 21.5°, and 23.6°.

For avoidance of any doubt, whenever one of the above embodiments refers to "peaks in the X-ray powder diffraction diagram at the following angles of refraction 2θ", said X-ray powder diffraction diagram is obtained by using combined Cu Kα1 and Kα2 radiation, without Kα2 stripping; and it should be understood that the accuracy of the 2θ values as provided herein is in the range of +/- 0.1-0.2°. Notably, when specifying an angle of refraction 2theta (2θ) for a peak in the invention embodiments and the claims, the 2θ value given is to be understood as an interval from said value minus 0.2° to said value plus 0.2° (2θ +/- 0.2°); and preferably from said value minus 0.1° to said value plus 0.1° (2θ +/- 0.1°).

40) Another embodiment relates to a crystalline form of COMPOUND according to any one of embodiments 37) to 39), which essentially shows the X-ray powder diffraction pattern as depicted in Figure 1.

41) Another embodiment relates to a crystalline form of COMPOUND according to any one of embodiments 37) to 40), which has a melting point of about 163°C as determined by differential scanning calorimetry e.g. using the method as described herein (wherein it is understood that the term "melting point" refers to the peak temperature as observed in the DSC).

42) Another embodiment relates to a crystalline form of COMPOUND according to any one of embodiments 37) to 41), wherein said form is obtainable by:
a) mixing COMPOUND as amorphous material with about 7 vol of ethanol;
b) heating to IT of about 78 °C (to form a clear solution);
c) cooling to an IT of about 0°C and stirring for at least 1 h (especially at least about 10 h) at 0 °C;
d) filtering and washing the cake with 2 vol of cold ethanol; and
e) drying the product (especially at about 40 °C) at reduced pressure (especially of about 10 mbar).

43) A further aspect of the invention relates to a crystalline form of COMPOUND according to any one of embodiments 37) to 42), for use in the manufacture of a pharmaceutical composition, wherein said pharmaceutical composition comprises as active ingredient the COMPOUND, and at least one pharmaceutically acceptable carrier material. Especially, this embodiment 43) relates to a crystalline form of COMPOUND according to any one of embodiments 37) to 42), for use in the manufacture of a pharmaceutical composition according to any one of embodiments 1) to 36).

For avoidance of any doubt, aspects of embodiment 43) refer to the crystalline form according to any one of embodiments 37) to 42) which is suitable for the manufacture of a pharmaceutical composition / which is used as final isolation step of COMPOUND (e.g. in order to meet the purity requirements of pharmaceutical production), whereas the final pharmaceutical composition according to embodiment 43) does not contain said crystalline form (because the originally crystalline form of COMPOUND is dissolved in the pharmaceutically acceptable carrier material(s); thus, in the final pharmaceutical composition, COMPOUND is present in dissolved form). Any reference to a crystalline form of COMPOUND for use in the manufacture of a certain pharmaceutical composition is to be understood as also referring to the use of said crystalline form in the manufacture of said pharmaceutical composition, and to a method of manufacturing said pharmaceutical composition comprising the use of said crystalline form of COMPOUND.

44) A further embodiment of the invention thus relates to a pharmaceutical composition comprising as active ingredient the COMPOUND [especially a pharmaceutical composition according to any one of embodiments 1) to 36)], wherein said pharmaceutical composition is manufactured using a crystalline form of COMPOUND according to any one of embodiments 37) to 42), and at least one pharmaceutically acceptable carrier material. The total ww% of the pharmaceutical composition as defined in any one of embodiments 1) to 36) and 44) is 100.

The term "pharmaceutical composition" is interchangeable with the terms "formulation", or "composition".

A pharmaceutical composition of the present invention is considered physically "stable", if during a certain period of time at least 70%, preferably at least 80% and most preferably at least 95% of the initial content of the COMPOUND is maintained over said period of time in a solubilized state. Additionally, the appearance may be considered as criterion to determine physical stability of a composition. The physical stability of the pharmaceutical compositions may be tested in conventional manner, e.g. by measurement of appearance of the composition and/or its water content; e.g. after storage at a certain temperature and relative humidity for defined periods of time.

The chemical stability of the pharmaceutical compositions may be tested in conventional manner, e.g. by measurement of the COMPOUND and its degradation products. The content of COMPOUND and its degradation products may be evaluated via conventional HPLC.

A pharmaceutical composition is considered chemically "stable", if during a certain period of time at least 80%, notably at least 95%, especially at least 98%, and preferably at least 99% of the initial content of the COMPOUND is maintained over said period of time without degradation.

Preferably, the pharmaceutical compositions of this invention will be chemically and physically "stable" for at least 6, preferably for at least 12 months when kept at a temperature of 5 °C to 50 °C and a rH of about 75 % or below. More preferably, they will be stable for at least 6 or preferably for 12 months when kept at a temperature of 15 °C to 45 °C and a rH of about 75 % or below. Most preferred, they will be stable for at least 6 or preferably for 12 months when kept at a temperature of 25 °C to 40 °C and a rH of about 75 % or below, especially at 40 °C and 75 % rH.

In a more preferred embodiment, the pharmaceutical compositions are chemically and physically stable over a certain period of time such as 1 year, and preferably 2 years.

The chemical and physical stability of the pharmaceutical compositions may be tested in conventional manner, e.g. by measurement of the COMPOUND and its degradation products; dissolution; disintegration time; appearance and/or microscopy, e.g. after storage at 25 °C and 60% relative humidity (RH), and/or storage at 40 °C and 75% relative humidity (RH) for defined periods of time; and by measurement of the ability of the formulation to maintain drug solubilization and to prevent precipitation on dispersion and digestion. The content of the COMPOUND and its degradation products may be evaluated via conventional HPLC.

Pharmaceutical compositions according to the present invention may be prepared using conventional methods. Methods for softgel or hard shell encapsulation are well known in the art. Procedures which may be used are conventional and well known in the art or based on such well known procedures e.g. those described in USP 23, General Information, Pharmaceutical Dosage Forms 1151: 1942-1943 (1995); E.T. Cole, "Liquid Filled Hard Gelatin Capsules", Pharm. Technol. Int., Sept./Oct. 1989; H. Seager, Soft Gelatin Capsules: a Solution to Many Tableting Problems, Pharm. Tech. 9 (1985).

The capsules may vary in size and may have, dependent on the targeted drug load and resulting amount of the required composition, any size from size 2 to 16. Preferred are size 7.5, 8.5, 10 and size 12 capsules, e.g. in form of oval capsules. The capsules of the invention may be coloured and/or marked so as to impart an individual appearance and to make them instantly recognizable.

The pharmaceutical compositions according to the invention may be used as a medicament.

45) A sixth aspect of the invention, thus, relates to pharmaceutical compositions according to any one of embodiments 1) to 36) or 44), for the prevention / prophylaxis or treatment of diseases and disorders related to pathogenic events associated with elevated levels of C5a and/or with C5aR activation.

Such diseases and disorders related to pathogenic events associated with elevated levels of C5a and/or with C5aR activation are especially:
- vasculitic diseases or disorders,
- inflammatory diseases or disorders involving intravascular microvesicle release,
- immune complex (IC) diseases or disorders,
- neurodegenerative diseases or disorders,
- complement related inflammatory diseases or disorders,
- bullous diseases or disorders,
- diseases or disorders related to ischemia and/or ischemic reperfusion injury,
- inflammatory bowel diseases or disorders,
- autoimmune diseases or disorders, or, in addition to the above listed,
- cancer.

In addition to the above-listed diseases and disorders, further diseases and disorders related to pathogenic events associated with elevated levels of C5a and/or with C5aR activation are:
- further inflammatory diseases or disorders associated with elevated levels of C5a and/or with C5aR activation such as especially neutropenia, sepsis, septic shock, stroke, inflammation associated with severe burns, osteoarthritis, acute (adult) respiratory distress syndrome (ARDS), chronic obstructive pulmonary disorder (COPD), asthma (especially bronchial asthma), systemic inflammatory response syndrome (SIRS), tissue graft rejection, hyperacute rejection of transplanted organs, multiple organ dysfunction syndrome (MODS), diabetic retinopathy, neuromyelitis optica, and glomerulonephritis including Heyman nephritis / membranous glomerulonephritis, Berger's disease (IgA nephropathy), and other forms of glomerulonephritis such as C3 glomerulopathy including dense deposit disease; as well as
- hemotological diseases which are associated with activation of coagulation and fibrinolytic systems, disseminated intravascular coagulation (DIC), pernicious anemia, warm and cold autoimmune hemolytic anemia (AIHA), anti-phospholipid syndrome and its associated complications, arterial or venous thrombosis, pregnancy complications such as recurrent miscarriage and fetal death, preeclampsia, placental insufficiency, fetal growth restriction, cervical remodeling and preterm birth, idiopathic thrombocytopenic purpura (ITP), atypical hemolytic uremic syndrome (aHUS), paroxysmal nocturnal hemoglobinuria (PNH), allergic transfusion reactions, acute antibody-mediated kidney allograft rejection, cold agglutinin disease and glaucoma.

The present compounds may in addition be useful for
- the prevention or treatment of deleterious consequences of contact sensitivity and inflammation caused by contact with artificial surfaces;
- the prevention or treatment of increased leukocyte and platelet activation (and infiltration to tissues thereof);
- the prevention or treatment of pathologic sequelae (such as especially prevention or treatment of the development of tissue injury, especially of pulmonary tissue injury) associated to an intoxication or an injury such as a trauma, an hemorrhage, a shock, or surgery including transplantation, including multiple organ failure (MOF), septic shock, shock due to intoxication (such as shock due to snake venom), or acute lung inflammatory injury;
- the prevention or treatment of pathologic sequelae associated with insulin-dependent diabetes mellitus;
- the prevention of / the reduction of the risk of myocardial infarction or thrombosis; prevention or treatment of edema or increased capillary permeability;
- the prevention of / the reduction of coronary endothelial dysfunction induced by cardiopulmonary bypass and/or cardioplegia.

Vasculitic diseases or disorders include especially vasculitis, ANCA associated vasculitis and glomerulonephritis (GN, especially rapidly progressive GN) associated with ANCA associated vasculitis, leukoclastic vasculitis, granulomatosis with polyangiitis (GPA, also referred to as Wegener's granulomatosis), microscopic polyangiitis, Churg-Strauss syndrome, Henoch-Schönlein purpura, polyateritis nodosa, cryoglobulinaemia, giant cell arteritis (GCA), Behcet's disease, and Takayasu's arteritis (TAK).

Inflammatory diseases or disorders involving intravascular microvesicle release include especially thrombotic microangiopathy, and sickle cell disease.

Immune complex (IC) diseases or disorders include especially cryoglobulinemia, Sjögren's syndrome (and associated immunological profiles), Goodpasture syndrome (antiglomerular basement antibody disease) and glomerulonephritis (GN, especially rapidly progressive GN) or pulmonary hemorrhage associated with Goodpasture syndrome, and hypersensitivity.

Neurodegenerative diseases and disorders include especially amyotrophic lateral sclerosis (ALS), Alzheimer's disease, Parkinson's disease, Huntington's disease, Guillain-Barre syndrome, neuropathy, , and cognitive function decline associated with cardiopulmonary bypass surgery and related procedures.

Complement related inflammatory diseases or disorders include especially coronary thrombosis, vascular occlusion, post-surgical vascular reocclusion, atherosclerosis, traumatic central nervous system injury, arrhythmogenic cardiomyopathy, bronchoconstriction, acute respiratory distress syndrome (ARDS), Chronic Obstructive Pulmonary Disorder (COPD), complement mediated thrombotic microangiopathies including atypical haemolytic uremic syndrome, and Gaucher disease.

Bullous diseases or disorders include especially bullous pemphigoid, bullous acquisita, pemphigus foliaceus, pemphigus vulgaris, sub-epidermal blisters, and hidradenitis suppurativa.

Diseases or disorders related to ischemia and/or ischemic reperfusion injury include especially ischemic reperfusion injury (including myocardial ischemia-reperfusion injury, and ischemic / reperfusion injury resulting from transplantation, including solid organ transplant), ischemic colitis, and cardiac ischemia.

Inflammatory bowel diseases or disorders include especially irritable bowel syndrome, ulcerative colitis, Crohn's disease, and inflammatory bowel disease (IBD).

Autoimmune diseases or disorders include especially rheumatoid arthritis, osteoarthritis, systemic lupus erythematosus (SLE) and glomerulonephritis (GN, especially rapidly progressive GN) associated with lupus erythematosus (lupus nephritis), central nervous system (CNS) lupus, dermatomyositis, pemphigus, systemic sclerosis (scleroderma), autoimmune hemolytic and thrombocytopenic states, immunovasculitis, mixed cryoglobulinemia, atopic dermatitis, chronic urticaria, psoriasis, myasthenia gravis, and anti-phospholipid syndrome.

Further inflammatory diseases or disorders associated with elevated levels of C5a and/or with C5aR activation include especially neutropenia, sepsis, septic shock, stroke, inflammation associated with severe burns, osteoarthritis, acute (adult) respiratory distress syndrome (ARDS), chronic obstructive pulmonary disorder (COPD), asthma, especially bronchial asthma, systemic inflammatory response syndrome (SIRS), tissue graft rejection, hyperacute rejection of transplanted organs, multiple organ dysfunction syndrome (MODS), diabetic retinopathy, neuromyelitis optica, and glomerulonephritis including Heyman nephritis / membranous glomerulonephritis, Berger's disease (IgA nephropathy), and other forms of glomerulonephritis such as C3 glomerulopathy including dense deposit disease.

The term "cancer" notably refers to skin cancer including melanoma including metastatic melanoma; lung cancer including non-small cell lung cancer; bladder cancer including urinary bladder cancer, urothelial cell carcinoma; renal carcinomas including renal cell carcinoma, metastatic renal cell carcinoma, metastatic renal clear cell carcinoma; gastro-intestinal cancers including colorectal cancer, metastatic colorectal cancer, familial adenomatous polyposis (FAP), oesophageal cancer, gastric cancer, gallbladder cancer, cholangiocarcinoma, hepatocellular carcinoma, and pancreatic cancer such as pancreatic adenocarcinoma or pancreatic ductal carcinoma; endometrial cancer; ovarian cancer; cervical cancer; neuroblastoma; prostate cancer including castrate-resistant prostate cancer; brain tumors including brain metastases, malignant gliomas, glioblastoma multiforme, medulloblastoma, meningiomas; breast cancer including triple negative breast carcinoma; oral tumors; nasopharyngeal tumors; thoracic cancer; head and neck cancer; leukemias including acute myeloid leukemia, adult T-cell leukemia; carcinomas; adenocarcinomas; thyroid carcinoma including papillary thyroid carcinoma; choriocarcinoma; Ewing's sarcoma; osteosarcoma; rhabdomyosarcoma; Kaposi's sarcoma; lymphoma including Burkitt's lymphoma, Hodgkin's lymphoma, MALT lymphoma; multiple myelomas; or virally induced tumors.

When used for the prevention / prophylaxis or treatment of a cancer, such use includes use of the present compounds as single therapeutic agents and their use in combination with one or more chemotherapy agents and / or radiotherapy and / or targeted therapy (especially in combination with targeted therapy).

The terms "radiotherapy" or "radiation therapy" or "radiation oncology", refer to the medical use of ionizing radiation in the prevention / prophylaxis (adjuvant therapy) and / or treatment of cancer; including external and internal radiotherapy.

The term "targeted therapy" refers to the prevention / prophylaxis (adjuvant therapy) and / or treatment of cancer with one or more anti-neoplastic agents such as small molecules or antibodies which act on specific types of cancer cells or stromal cells. Some targeted therapies block the action of certain enzymes, proteins, or other molecules involved in the growth and spread of cancer cells. Other types of targeted therapies help the immune system kill cancer cells (immunotherapies); or inhibit angiogenesis, the growth and formation of new blood vessels in the tumor; or deliver toxic substances directly to cancer cells and kill them. An example of a targeted therapy which is in particular suitable to be combined with the compounds of the present invention is immunotherapy, especially immunotherapy targeting the progammed cell death receptor 1 (PD-1 receptor) or its ligand PD-L1.

When used in combination with the present compounds, the term "targeted therapy" especially refers to agents such as:
a) Epidermal growth factor receptor (EGFR) inhibitors or blocking antibodies (for example Gefitinib, Erlotinib, Afatinib, Icotinib, Lapatinib, Panitumumab, Zalutumumab, Nimotuzumab, Matuzumab and Cetuximab);
b) RAS/RAF/MEK pathway inhibitors (for example Vemurafenib, Sorafenib, Dabrafenib,GDC-0879, PLX-4720, LGX818, RG7304, Trametinib (GSK1120212), Cobimetinib (GDC-0973/XL518), Binimetinib (MEK162, ARRY-162), Selumetinib (AZD6244));
c) Aromatase inhibitors (for example Exemestane, Letrozole, Anastrozole, Vorozole, Formestane, Fadrozole);
d) Angiogenesis inhibitors, especially VEGF signalling inhibitors such as Bevacuzimab (Avastin), Ramucirumab, Sorafenib or Axitinib;
e) Immune Checkpoint inhibitors (for example: anti-PD1 antibodies such as Pembrolizumab (Lambrolizumab, MK-3475), Nivolumab, Pidilizumab (CT-011), AMP-514/MED10680, PDR001, SHR-1210; REGN2810, BGBA317; fusion proteins targeting PD-1 such as AMP-224; small molecule anti-PD1 agents such as for example compounds disclosed in WO2015/033299, WO2015/044900 and WO2015/034820; anti-PD1L antibodies, such as BMS-936559, atezolizumab (MPDL3280A, RG7446), MEDI4736, avelumab (MSB0010718C), durvalumab (MEDI4736); anti-PDL2 antibodies, such as AMP224; anti-CTLA-4 antibodies, such as ipilimumab, tremilmumab; anti-Lymphocyte-activation gene 3 (LAG-3) antibodies, such as BMS-986016, IMP701, MK-4280, ImmuFact IMP321; anti T cell immunoglobulin mucin-3 (TIM-3) antibodies, such as MBG453; anti-CD137/4-1BB antibodies, such as BMS-663513 / urelumab, PF-05082566; anti T cell immunoreceptor with Ig and ITIM domains (TIGIT) antibodies, such as RG6058 (anti-TIGIT, MTIG7192A);
f) Vaccination approaches (for example dendritic cell vaccination, peptide or protein vaccination (for example with gp100 peptide or MAGE-A3 peptide);
g) Re-introduction of patient derived or allogenic (non-self) cancer cells genetically modified to secrete immunomodulatory factors such as granulocyte monocyte colony stimulating factor (GMCSF) gene-transfected tumor cell vaccine (GVAX) or Fms-related tyrosine kinase 3 (Flt-3) ligand gene-transfected tumor cell vaccine (FVAX),or Toll like receptor enhanced GM-CSF tumor based vaccine (TEGVAX);
h) T-cell based adoptive immunotherapies, including chimeric antigen receptor (CAR) engineered T-cells (for example CTL019);
i) Cytokine or immunocytokine based therapy (for example Interferon alpha, interferon beta, interferon gamma, interleukin 2, interleukin 15);
j) Toll-like receptor (TLR) agonists (for example resiquimod, imiquimod, glucopyranosyl lipid A, CpG oligodesoxynucleotides);
k) Thalidomide analogues (for example Lenalidomide, Pomalidomide);
l) Indoleamin-2,3-Dioxgenase (IDO) and/or Tryptophane-2,3-Dioxygenase (TDO) inhibitors (for example RG6078 / NLG919 / GDC-0919; Indoximod / 1MT (1-methyltryptophan), INCB024360 / Epacadostat, PF-06840003 (EOS200271), F001287);
m) Activators of T-cell co-stimulatory receptors (for example anti-OX40/CD134 (Tumor necrosis factor receptor superfamily, member 4, such as RG7888 (MOXR0916), 9B12; MEDI6469, GSK3174998, MEDI0562), anti OX40-Ligand/CD252; anti-glucocorticoid-induced TNFR family related gene (GITR) (such asTRX518, MEDI1873, MK-4166, BMS-986156), anti-CD40 (TNF receptor superfamily member 5) antibodies (such as Dacetuzumab (SGN-40), HCD122, CP-870,893, RG7876, ADC-1013, APX005M, SEA-CD40); anti-CD40-Ligand antibodies (such as BG9588); anti-CD27 antibodies such as Varlilumab);
n) Molecules binding a tumor specific antigen as well as a T-cell surface marker such as bispecific antibodies (for example RG7802 targeting CEA and CD3) or antibody fragments, antibody mimetic proteins such as designed ankyrin repeat proteins (DARPINS), bispecific T-cell engager (BITE, for example AMG103, AMG330);
o) Antibodies or small molecular weight inhibitors targeting colony-stimulating factor-1 receptor (CSF-1R) (for example Emactuzumab (RG7155), Cabiralizumab (FPA-008), PLX3397);
p) Agents targeting immune cell check points on natural killer cells such as antibodies against Killer-cell immunoglobulin-like receptors (KIR) for example Lirilumab (IPH2102/BMS-986015);
q) Agents targeting the Adenosine receptors or the ectonucleases CD39 and CD73 that convert ATP to Adenosine, such as MEDI9447 (anti-CD73 antibody), PBF-509; CPI-444 (Adenosine A2a receptor antagonist).

When used in combination with the present compounds, immune checkpoint inhibitors, and especially those targeting the PD-1 receptor or its ligand PD-L1, are preferred.

The invention further relates to a method of modulating (especially downregulating) the consequences of the complement activation (especially by activating innate cells) in a subject in need thereof [especially in a subject having a disease or disorder related to pathogenic events associated with elevated levels of C5a and/or with C5aR activation; in particular in a subject having a vasculitic disease or disorder, an inflammatory disease or disorder involving intravascular microvesicle release, an immune complex (IC) disease or disorder, a neurodegenerative disease or disorder, a complement related inflammatory disease or disorder, a bullous disease or disorder, a disease or disorder related to ischemia and/or ischemic reperfusion injury, an inflammatory bowel disease or disorder, or an autoimmune disease or disorder; or in a subject having a contact sensitivity or an inflammation caused by contact with artificial surfaces; an increased leukocyte and platelet activation (and infiltration to tissues thereof); a pathologic sequelae associated to an intoxication or an injury such as a trauma, an hemorrhage, a shock, or surgery including transplantation, including multiple organ failure (MOF), septic shock, shock due to intoxication (such as shock due to snake venom), or acute lung inflammatory injury; a pathologic sequelae associated with insulin-dependent diabetes mellitus; a myocardial infarction or thrombosis; an edema or an increased capillary permeability; or a reduction of coronary endothelial dysfunction induced by cardiopulmonary bypass and/or cardioplegia], comprising administering to said subject a pharmaceutically active amount of a compound of formula (I) as defined in any one of embodiments 1) to 50). For avoidance of doubt, the term "modulating the complement activation" is to be understood as downregulating / reducing the amplification of the immune response and downregulating / reducing the activation of the cell-killing membrane attack complex, especially by activating innate cells.

For avoidance of any doubt, if compounds / compositions are described as useful for the prevention or treatment of certain diseases or disorders, such compounds / compositions are likewise suitable for use in the preparation of a medicament for the prevention or treatment of said diseases; and suitable for use in a method of preventing or treating said diseases comprising administering to a subject (notably a mammal, especially a human) in need thereof a pharmaceutically active amount of said compound / composition.

The term "prevent" or "prevention" or "preventing" used with reference to a disease means either that said disease does not occur in the patient or animal, or that, although the animal or patient is affected by the disease, part or all the symptoms of the disease are either reduced or absent. The terms "prevention" may also be understood to mean "prophylaxis".

The term "treat" or "treatment" or "treating" used with reference to a disease means either that said disease is cured in the patient or animal, or that, although the animal or patient remains affected by the disease, part or all the symptoms of the disease are either reduced or eliminated.

The pharmaceutical composition of the present invention may be formulated as capsule. Capsules with a strength of 0.5 mg to 20 mg (for example (soft gelatine) capsules (oval, size 12) of 667 mg per capsule) may be prepared as follows:

| **Material** | **Unit Dose (mg/capsule)** | **Percentage (%w/w)** | **Function** |
|---|---|---|---|
| COMPOUND (amounts for free base) | 0.5 - 20 | 0.075 - 3 | Active Ingredient |

| Excipients | | | |
|---|---|---|---|
| Capryol^{™} 90 | 180 - 210 | 27 - 31.5 | Lipophilic excipient / Hydrophobic surfactant |
| Kolliphor^{®} RH40 | 310 - 340 | 46.5 - 51 | Hydrophilic surfactant |
| Triethyl citrate | 120 - 140 | 18 - 21 | Hydrophilic co-solvent |

| Optional conventional ingredients or additives | | | |
|---|---|---|---|
| Ascorbyl palmitate | 0.5 - 10 | 0.075 -1.5 | Antioxidant / Oxygen scavenger |
| DL-alpha-Tocopheryl acetate | 0.1 - 0.5 | 0.015 - 0.075 | Antioxidant / Chain terminator |
| **Total** | **quantum satis to a total of 667** | **quantum satis: the total %wlw is 100** | |

A particular example (soft gelatine) capsule (oval, size 12) of 667 mg per capsule having a drug load of about 5 mg may be prepared as follows:

| **Material** | **Unit Dose (mg/capsule)** | **Percentage (%w/w)** | **Function** |
|---|---|---|---|
| COMPOUND (amounts for free base) | 5 | 0.75 | Active Ingredient |
| Capryol^{™} 90 | 196.5 | 29.46 | Lipophilic excipient / Hydrophobic surfactant |
| Kolliphor^{®} RH40 | 327.5 | 49.10 | Hydrophilic surfactant |
| Triethyl citrate | 131 | 19.64 | Hydrophilic co-solvent |
| Ascorbyl palmitate | 6.7 | 1 | Antioxidant / Oxygen scavenger |
| DL-alpha-Tocopheryl acetate | 0.3 | 0.05 | Antioxidant / Chain terminator |
| **Total** | **667** | **100** | |

A particular example (soft gelatine) capsule (oval, size 12) of 667 mg per capsule having a drug load of about 10 mg may be prepared as follows:

| **Material** | **Unit Dose (mg/capsule)** | **Percentage (%w/w)** | **Function** |
|---|---|---|---|
| COMPOUND (amounts for free base) | 10 | 1.5 | Active Ingredient |
| Capryol^{™} 90 | 195 | 29.235 | Lipophilic excipient / Hydrophobic surfactant |
| Kolliphor^{®} RH40 | 325 | 48.725 | Hydrophilic surfactant |
| Triethyl citrate | 130 | 19.49 | Hydrophilic co-solvent |
| Ascorbyl palmitate | 6.7 | 1 | Antioxidant / Oxygen scavenger |
| DL-alpha-Tocopheryl acetate | 0.3 | 0.05 | Antioxidant / Chain terminator |
| **Total** | **667** | **100** | |

The amounts of excipients may be adjusted for the purity of the active ingredient, which may give rise to increased amounts of COMPOUND. The process for the preparation of a pharmaceutical composition in the form of capsules according to the present invention can in particular be performed in analogy to the Examples. It may comprise the following steps: step 0 (preparation), step 1 (preparation of bulk fill solutions), step 2 (capsule filling process), step 3 (capsule sealing process), step 4 (capsule weight sorting), and step 5 (packaging); and can be carried out for example according to the following process flow chart:

| **Step** | **Material** | **Operation** | **Equipment** |
|---|---|---|---|
| 0 | | Pre-heating of excipients | Oven |
| | | | |
| | | Weighting of all ingredients | |
| 1 | | Mixing with heating and stirring | Stainless steel vessel equipped with a heating system |
| | | Solubilization with heating and stirring | |
| | COMPOUND | Solubilization with heating and stirring | |
| | | Bulk capsule fill solution | |
| 2 | Soft gelatine capsules | Bulk mixing and heating during the liquid filling in capsules | |
| 3 | | Sealing of capsules and drying | Automatic capsule filling machine |
| | | Vacuum for visual examination | Liquid encapsulation microspray sealing equipment |
| | | Bulk capsules | Vaccum chamber |
| 4 | | Weight sorting of capsules | |
| 5 | Polyethylene bags or aluminium blisters | Packaging | Capsule sorter |

The following examples are provided to further illustrate the invention. These examples are illustrative only and should not be construed as limiting the invention in any way.

### EXAMPLES

Raw materials can be purchased from commercial suppliers: In particular: PEG 40 hydrogenated castor oil (Kolliphor^{®} RH 40) can be purchased from BASF. Propylene glycol monocaprylate (Capryol^{™} 90), Gelucire^{®} 48/16, Labrafac^{™} PG, Labrasol^{®} ALF, Diethylene glycol monoethylether (Transcutol^{®}): Gattefossé. Middle chain triglycerides (Miglyol^{®} 812): Hanseler AG. BHA (Butylated Hydroxy Anisol): Merck. Triethylcitrate: Jungbunzlauer.: Gattefossé. Kolliphor^{®} EL, Vitamin E TPGS, Ethanol, Propyl gallate, EDTA (Ethylenediaminetetraacetic acid), Ascorbyl palmitate and Tocopheryl acetate: Sigma.

All temperatures are stated in °C. Commercially available starting materials can be used as received without further purification.

### Characterization of compounds

Compounds described in the invention can be characterized by LC-MS data (retention time *t*_{R} is given in min) and/or NMR using the conditions described below.

### Analytical LC-MS:

LC-MS (Method I): Waters Acquity UPLC i-Class system with Waters i-Class BSM binary pump, Thermo MSQ Plus MS detector and Waters Acquity PDA detector.
Eluents (acidic conditions): A: H₂O + 0.04% TFA; B: MeCN; gradient: 5% B → 95% B; runtime: 1.2 min; flow: 0.8 mL/min; detection: UV/Vis + MS
Column Agilent Zorbax RRHD SB-aq, 2.1 x 50 mm, 1.8 µm
LC-MS (Method II): Dionex Ultimate 3000 system with Dionex HPG-3200RS binary pump, Thermo MSQ Plus MS detector and Dionex DAD-3000RS PDA detector.
Eluents (acidic conditions): A: H₂O + 0.04% TFA; B: MeCN; gradient: 5% B → 95% B; runtime: 1.5 min; flow: 4.5 mL/min; detection: UV/Vis + MS
Column Agilent Zorbax SB-aq, 4.6 x 50 mm, 3.5 µm

### HPLC 1

Samples were directly analyzed by HPLC without dilution (expected concentration 1000 µg/mL). The HPLC area of each treated sample was compared to the HPLC area of the reference samples.

HPLC system: High pressure mixing Shimadzu Prominence (HPLC_08_DAD); Flow: 1.5 mL/min; Column temperature: 50°C; Autosampler temperature: 25°C; Injection volume: 3.0 µL; Column: Waters XBridge BEH C18 2.5 µm 2.1*50 mm Column XP; Wavelength: DAD 260 nm

| | |
|---|---|
| Solvent A: Water + 0.05% formic acid (v/v) | Solvent B: Acetonitrile + 0.05% formic acid (v/v) |

### Gradient:

| Time (min) % | solvent A % | solvent B | Time (min) % | solvent A % | solvent B |
|---|---|---|---|---|---|
| 0.0 | 80 | 20 | 2.5 | 36 | 64 |
| 3.5 | 33 | 67 | 4.5 | 2 | 98 |
| 4.9 | 2 | 98 | 5.0 | 80 | 20 |
| 5.2 | 80 | 20 | | | |

### NMR spectroscopy:

Bruker Avance HD spectrometer equipped with a 500 MHz Ultrashield^{™} Magnet and a 5 mm DCH cryoprobe or Bruker Avance II spectrometer equipped with a 400 MHz Ultrashield^{™} Magnet and a BBO 5mm probehead. Chemical shifts (δ) are reported in parts per million (ppm) relative to proton resonances resulting from incomplete deuteration of the NMR solvent, *e*.*g*. for dimethylsulfoxide δ(H) 2.49 ppm, for chloroform δ(H) 7.24 ppm. The abbreviations *s*, *d, t, q* and m refer to singlet, doublet, triplet, quartet, multiplet, respectively and brto broad. Coupling constants *J* are reported in Hz.

### X-ray powder diffraction analysis (XRPD)

X-ray powder diffraction patterns were collected on a Bruker D8 Advance X-ray diffractometer equipped with a Lynxeye detector operated with CuKα-radiation in reflection mode (coupled two Theta/Theta). Typically, the X-ray tube was run at of 40kV/40mA. A step size of 0.02° (2θ) and a step time of 76.8 sec over a scanning range of 3 - 50° in 2θ were applied. The divergence slits were set to fixed 0.3°. Powders were slightly pressed into a silicon single crystal sample holder with depth of 0.5 mm and samples were rotated in their own plane during the measurement. Diffraction data are reported without application of K α2 stripping. The accuracy of the 2θ values as provided herein is in the range of +/- 0.1-0.2° as it is generally the case for conventionally recorded X-ray powder diffraction patterns.

### Gravimetric vapour sorption (GVS) analysis

Measurements are performed on a multi sample instrument SPS-100n (ProUmid, Ulm, Germany) operated in stepping mode at 25°C. The sample is allowed to equilibrate at 40% RH before starting a pre-defined humidity program (40-0-95-0-95-40% RH, steps of 5% ΔRH and with a maximal equilibration time of 24 hours per step are applied). About 20 to 30 mg of each sample is used. The hygroscopic classification is done on the basis of the European Pharmacopeia Technical Guide (1999, page 86), e.g., Non-hygroscopic: increase in mass is less than 0.2% mass/mass; slightly hygroscopic: increase in mass is less than 2% and equal to or greater than 0.2% mass/mass; hygroscopic: increase in mass is less than 15% and equal to or greater than 2% mass/mass. The mass change between 40% relative humidity and 80% relative humidity in the first adsorption scan is considered.

### Differential scanning calorimetry (DSC)

DSC data were collected on a Mettler Toledo STARe System (DSC822e module, measuring cell with ceramic sensor and STAR software version 13.00) equipped with a 34 position auto-sampler. The instrument was calibrated for energy and temperature using certified indium. Typically 2 mg of each sample, in an automatically pierced 40µL Mettler aluminium pan, was heated at 10°C min⁻¹, from -20°C to 320°C. A nitrogen purge at 20 ml min⁻¹ was maintained over the sample.

### Purification of compounds

The compounds can be purified by either column chromatography on silica-gel and/or prep. LC-MS using the conditions described below.

### Column chromatography

Column chromatography (CC) can be performed using prepacked cartridges (SNAP Ultra^{™}, SNAP KP-SIL^{™}, SNAP KP-NH^{™}, Isolute^{™} Silica II or Isolute^{™} NH₂) from Biotage.

### Preparative LC-MS:

Gilson 333/334 Prep-Scale HPLC pump equipped with Gilson LH215 autosampler, Dionex SRD-3200 degasser, Dionex ISO-3100A make-up pump, Dionex DAD-3000 DAD detector and Thermo MSQ Plus Single Quadrupole MS detector. Flow: 75 mL/min. Detection: UV/Vis and/or MS.
Additional information for the purification is summarized in the table below with following definitions: XBridge: column Waters XBridge C18, 10 µm, 30 x 75 mm
Zorbax: column Agilent Zorbax SB-aq, 5 µm, 30 x 75 mm
Atlantis: column Waters Atlantis T3, 10 µm, 30 x 75 mm
Acidic: eluant: A = H₂O with 0.5% HCOOH, B = MeCN
Basic: eluant: A = H₂O with 0.125% NH₄OH, B = MeCN
Very lipophilic gradient: 50% B → 95% B over 4 min then 95%B over 2 min
Lipophilic gradient: 30% B → 95% B over 4 min then 95%B over 2 min
Normal gradient: 20% B → 95% B over 4 min then 95%B over 2 min
Polar gradient: 10% B → 95% B over 4 min then 95%B over 2 min
Very polar gradient: 5% B → 50% B over 3 min then 50% B → 95% B over 1 min and finally 95%B over 2 min

| | **XBridge** | | **Zorbax** | **Atlantis** |
|---|---|---|---|---|
| | **acidic** | **basic** | **acidic** | **basic** |
| **Very lipophilic gradient** | Method 10 | Method 8 | Method 9 | Method 6 |
| **Lipophilic gradient** | Method 4 | Method 5 | Method 2 | |
| **Normal gradient** | Method 3 | Method 1 | Method 11 | |
| **Polar gradient** | | Method 7 | | |
| **Very polar gradient** | | Method 12 | | |

### Digestion media composition:

| FaSSIF (pH 6.5) | FeSSIF (pH 5.0) | FaSSGF (pH 1.6) |
|---|---|---|
| Sodium taurocholate : 3 mM | Sodium taurocholate : 10.00 mM | Sodium taurocholate : 0.08 mM |
| Lecithin 0.75 mM | Lecithin 2.00 mM | Lecithin 0.02 mM |
| Sodium Chloride 105.90 mM | Glycerol monooleate 5.00 mM | Sodium Chloride 34.20 mM |
| Monobasic sodium phosphate 28.40 mM | Sodium oleate 0.80 mM | Hydrochloric acid 25.10 mM |
| Sodium hydroxide 8.70 mM | Sodium Chloride 125.50 mM | |
| | Sodium hydroxide 81.65 mM | |
| | Maleic acid 55.02 mM | |

### Abbreviations (as used hereinbefore or hereinafter):

- Ac: acetyl
- AcOH: acetic acid
- aq.: aqueous
- Boc: tert.-butyloxycarbonyl
- CC: column chromatography
- CDI: carbonyl diimidazole
- CDT: 1,1'-carbonyl-di-(1,2,4-triazole)
- DCM: dichloromethane
- dioxane: 1,4-dioxane
- DIPEA: diisopropylethylamine
- DMA: dimethylacetamide
- DMF: dimethylformamide
- DMSO: dimethylsulfoxide
- DSC: Differential Scanning Calorimetry
- eq: equivalent(s)
- Et: ethyl
- EtOAc: ethyl acetate
- EtOH: ethanol
- Et₂O: diethylether
- 9: gram(s)
- IT: internal temperature
- h: hour(s)
- Hept: heptane
- HPLC: high performance liquid chromatography
- io: ionisation
- LC-MS: liquid chromatography - mass spectrometry
- MeCN: acetonitrile
- MeOH: methanol
- mg: milligram(s)
- min: minute(s)
- mL: milliliter(s)
- mmol: millimole(s)
- MS: mass spectroscopy
- NaBH(OAc)₃: sodium triacetoxyborohydride
- NMR: nuclear magnetic resonance spectroscopy
- OAc: acetate
- org.: organic
- ON: overnight
- prep.: preparative
- QuadraPure^{®} MPA: mercaptophenyl amino functionalized polystyrene beads
- rac: racemic
- RT: room temperature
- rxn: reaction
- sat.: saturated
- SEM: 2-(trimethylsilyl)ethoxymethyl
- soln.: solution
- TEA: triethylamine
- TFA: trifluoroacetic acid
- TFE: trifluoroethanol
- THF: tetrahydrofuran
- *t*_{R}: retention time
- XRPD: X-ray powder diffraction

### I. Chemistry

The synthesis of the COMPOUND and its biological activity as a C5a receptor modulator is described in PCT/EP2019/050372.

When not commercially available (as for example 2-(Trifluoromethyl) benzyl bromide), building blocks are prepared according to the procedures described below.

### Synthesis of 5-Nitro-1H-pyrazole-4-carboxylic acid methyl ester or 3-Nitro-1H-pyrazole-4-carboxylic acid methyl ester

To a soln. of 3-Nitro-1H-pyrazole-4-carboxylic acid (1 eq) in anh. MeOH (4 mL/mmol) is added AcCl (3 eq) and the rxn mixture is stirred for 2.5 h at 80°C. MeOH is evaporated off and the residue is partitioned between a sat. aq. soln. of NaHCO₃ and EtOAc. The org. phase is washed with a 10% aq. soln. of Na₂CO₃ and with brine, dried over MgSO₄ and concentrated in vacuo.

t_{R} [min] (LC/MS method): 0.55 (I); m/z [M+H]⁺ no ionisation.

1H NMR (500 MHz,DMSO-d6) δ: 14.34 (s, 1 H), 8.60 (s, 1 H), 3.79 (s, 3 H).

### Synthesis of 3-Nitro-1-(2-trimethylsilanyl-ethoxymethyl)-1H-pyrazole-4-carboxylic acid methyl ester

To a suspension of 5-Nitro-1H-pyrazole-4-carboxylic acid methyl ester or 3-Nitro-1H-pyrazole-4-carboxylic acid methyl ester (1 eq) and SEM-Cl (1.3 eq) in DCM (3.5 mL/mmol) is added dropwise DIPEA (1.5 eq) at 0°C. The rxn mixture is stirred at 0°C for 0.5 h and quenched with a sat. aq. soln. of NaHCO₃. It is extracted with DCM, the org. phase is washed with a sat. aq. soln. of NaHCO₃, dried over MgSO₄ and concentrated in vacuo. The crude is purified by CC using Hept/EtOAc.

t_{R} [min] (LC/MS method): 1.00 (I); m/z [M+H]⁺ 302.15.

1H NMR (500 MHz,DMSO-d6) δ: 8.80 (s, 1 H), 5.54 (s, 2 H), 3.81 (s, 3 H), 3.61 (m, 2 H), 0.87 (m, 2 H), -0.03 (s, 9 H).

### Synthesis of [3-Nitro-1-(2-trimethylsilanyl-ethoxymethyl)-1H-pyrazol-4-yl]-methanol

To a soln. of 3-Nitro-1-(2-trimethylsilanyl-ethoxymethyl)-1H-pyrazole-4-carboxylic acid methyl ester (1 eq) in a mixture of THF (6.3 mL/mmol) and MeOH (0.8 mL/mmol) is added portionwise NaBH₄ (4 to 8 eq) at 0°C. The rxn mixture is stirred at 0°C for 3.5 h, poured into an aq. sat. soln. of NH₄Cl and extracted with EtOAc. The org. phase is washed with brine, dried over MgSO₄ and concentrated in vacuo. The crude is purified by CC using Hept/EtOAc.

t_{R} [min] (LC/MS method): 0.89 (I); m/z [M+H]⁺ no ionisation.

1H NMR (500 MHz,DMSO-d6) δ: 8.06 (s, 1 H), 5.51 (s, 2 H), 5.39 (t, J = 5.4 Hz, 1 H), 4.66 (dd, J = 5.4 Hz, 2 H), 3.59 (m, 2 H), 0.87 (m, 2 H), -0.03--0.01 (m, 9 H).

### Synthesis of 3-Nitro-1-(2-trimethylsilanyl-ethoxymethyl)-1H-pyrazole-4-carbaldehyde

To a soln. of [3-Nitro-1-(2-trimethylsilanyl-ethoxymethyl)-1H-pyrazol-4-yl]-methanol (1 eq) in anh. DCM (10 mL/mmol) is added portionwise MnO₂ (9 to 10 eq) at RT and the rxn mixture is stirred at RT for 18 h. It is filtered over a pad of celite and the filtrate is concentrated in vacuo.

t_{R} [min] (LC/MS method): 1.00 (I); m/z [M+H]⁺ no ionisation.

1H NMR (500 MHz,DMSO-d6) δ: 10.14 (s, 1 H), 8.82 (s, 1 H), 5.58 (s, 2 H), 3.62 (m, 2 H), 0.88 (m, 2 H), -0.02 (m, 9 H).

### Synthesis of [1-(2-Fluoro-6-formyl-phenyl)-piperidin-4-yl]-carbamic acid tert-butyl ester

To a soln. of 4-(Boc-amino) piperidine (1 eq) and 2,3-Difluoro-benzaldehyde (1.1 eq) in DMSO (0.9 to 1.5 mL/mmol) is added K₂CO₃ (2 eq) and the mixture is heated to 100 °C and stirred for 18h. It is quenched with water and extracted with DCM. The org. phase is washed with water and brine, dried over \MgSO₄ and concentrated in vacuo. The crude is purified by CC using DCM/MeOH.

t_{R} [min] (LC/MS method): 0.93 (II); m/z [M+H]⁺ 323.20.

### Synthesis of [1-(2-Fluoro-6-hydroxymethyl-phenyl)-piperidin-4-yl]-carbamic acid tert-butyl ester

A suspension of [1-(2-Fluoro-6-formyl-phenyl)-piperidin-4-yl]-carbamic acid tert-butyl ester (1 eq) in anh. MeOH (2 mL/mmol) is cooled to 0°C and NaBH₄ (1.2 to 1.3 eq) is added portionwise at 0°C. The rxn mixture is stirred for 1h at 0°C to reach completion. It is carefully quenched by dropwise addition of water at 0°C and extracted with EtOAc. The org. phase is washed with water and brine, dried over MgSO₄ and concentrated in vacuo.

t_{R} [min] (LC/MS method): 0.82 (II); m/z [M+H]+ 325.24.

### Synthesis of Acetic acid 2-(4-tert-butoxycarbonylamino-piperidin-1-yl)-3-fluoro-benzyl ester

A soln. of [1-(2-Fluoro-6-hydroxymethyl-phenyl)-piperidin-4-yl]-carbamic acid tert-butyl ester (1 eq) and TEA (1.5 eq) in DCM (0.5 to 5 mL/mmol) is cooled to 0°C and AcCl (1.5 eq) is added dropwise at 0°C. The rxn mixture is stirred for 1h at 0°C to reach completion. It is diluted with DCM and washed with a 10% aq. soln. of citric acid, with a sat. aq. soln. of NaHCO₃ and with brine. The org. phase is dried over MgSO₄ and concentrated in vacuo. The crude is purified by CC using Hept/EtOAc.

t_{R} [min] (LC/MS method): 0.97 (II); m/z [M+H]⁺ 367.25.

### Synthesis of [1 -(2-Fluoro-6-methyl-phenyl)-piperidin-4-yl]-carbamic acid tert-butyl ester

Acetic acid 2-(4-*tert*-butoxycarbonylamino-piperidin-1-yl)-3-fluoro-benzyl ester (1 eq) is dissolved in a mixture of MeOH (6 mL/mmol) and EtOAc (2 mL/mmol) and the flask is evacuated three times and refilled with nitrogen. Wet Pd/C (0.08 eq) is added and the flask is evacuated three times and refilled with hydrogen. The suspension is hydrogenated under atmospheric pressure for 3h and filtered over a pad of Celite. The cake is washed with EtOAc and MeOH and the filtrate is concentrated in vacuo. The crude is purified by CC using Hept/EtOAc.

t_{R} [min] (LC/MS method): 1.00 (II); m/z [M+H]⁺ 309.16.

### Synthesis of 1-(2-Fluoro-6-methyl-phenyl)-piperidin-4-ylamine

To a soln. of [1-(2-Fluoro-6-methyl-phenyl)-piperidin-4-yl]-carbamic acid *tert*-butyl ester (1 eq) in DCM (4 mL/mmol) is added dropwise TFA (1 mL/mmol) and the rxn mixture is stirred for 1h to 18h at RT. It is basified with a 1M aq. soln. of NaOH until pH 12-13 and extracted with DCM. The combined org. phases are dried over MgSO₄ and concentrated in vacuo.

t_{R} [min] (LC/MS method): 0.62 (I); m/z [M+H]⁺ 209.21.

### Synthesis of [1-(2-Fluoro-6-methyl-phenyl)-piperidin-4-yl]-[3-nitro-1-(2-trimethylsilanyl-ethoxymethyl)-1H-pyrazol-4-ylmethyl]-amine

To a soln. of 3-Nitro-1-(2-trimethylsilanyl-ethoxymethyl)-1H-pyrazole-4-carbaldehyde (1 eq) and 1-(2-Fluoro-6-methyl-phenyl)-piperidin-4-ylamine (1 to 1.15 eq) in THF (4 to 8 mL/mmol) are added AcOH (1.5 eq) and the rxn mixture is stirred for 20 min at RT. NaBH(OAc)₃ (1.5 eq) is added portionwise and the rxn mixture is stirred at RT for 2 h. When necessary to reach completion of the rxn, an extra portion of NaBH(OAc)₃ (1 eq) is added at RT. It is partitioned between EtOAc and a sat. aq. soln. of NaHCO₃. The org. phase is washed with brine, dried over MgSO₄ and concentrated in vacuo. The crude is purified by CC using Hept/EtOAc/MeOH.

t_{R} [min] (LC/MS method): 0.94 (I); m/z [M+H]⁺ 464.25.

### Synthesis of [3-Amino-1-(2-trimethylsilanyl-ethoxym ethyl)-1H-pyrazol-4-ylmehyl1-[1-(2-fluoro-6-methyl-phenyl)-piperidin-4-yl]-amine

To a soln. of [1-(2-Fluoro-6-methyl-phenyl)-piperidin-4-yl]-[3-nitro-1-(2-trimethylsilanyl-ethoxymethyl)-1H-pyrazol-4-ylmethyl]-amine (1 eq) in EtOH (3.5 to 7.4 mL/mmol) is added 10% Pd/C moistened with ~50% water (0.02 eq) and the rxn mixture is hydrogenated at RT under atmospheric pressure for 18 h. It is filtered over a pad of celite and the filtrate is concentrated in vacuo. When necessary, the crude is purified by CC using

DCM/MeOH.

t_{R} [min] (LC/MS method): 0.85 (I); m/z [M+H]⁺ 434.10.

### Synthesis of 5-[1-(2-Fluoro-6-methyl-phenyl)-piperidin-4-yl]-2-(2-trimethylsilanyl-ethoxymethyl)-2,4,5,7-tetrahydro-pyrazolo[3,4-d]pyrimidin-6-one

To a soln. of [3-Amino-1-(2-trimethylsilanyl-ethoxymethyl)-1H-pyrazol-4-ylmethyl]-[1-(2-fluoro-6-methylphenyl)-piperidin-4-yl]-amine (1 eq) in MeCN (3.7 to 10 mL/mmol) is added CDI (1.2 to 2 eq) and the rxn mixture is stirred at RT for 1.5 h. When necessary to reach completion of the rxn an extra amount of CDI (0.5 to 1 eq) is added. The solvent is evaporated off and the residue is partitioned between EtOAc or DCM and water. The org. phase is washed with brine, dried over MgSO₄ and concentrated in vacuo. The crude is purified by CC using Hept/EtOAc.

t_{R} [min] (LC/MS method): 1.16 (I); m/z [M+H]⁺ 460.26.

### Synthesis of 5-[1-(2-Fluoro-6-methyl-phenyl)-piperidin-4-yl]-7-(2-trifluoromethyl-benzyl)-2-(2-trimethylsilanyl-ethoxymethyl)-2,4,5,7-tetrahydro-pyrazolo[3,4-d]pyrimidin-6-one

To a soln. of 5-[1-(2-Fluoro-6-methyl-phenyl)-piperidin-4-yl]-2-(2-trimethylsilanyl-ethoxymethyl)-2,4,5,7-tetrahydro-pyrazolo[3,4-d]pyrimidin-6-one (1 eq) in a mixture of anh. THF (3 to 7.3 mL/mmol) and anh. DMF (0 to 0.7 mL/mmol) is added NaH (1.5 to 10 eq, as a 60% dispersion in mineral oil) at 0°C. The suspension is stirred for 10 min and 2-(Trifluoromethyl) benzyl bromide (1.1 to 1.5 eq) is added at 0°C. The rxn mixture is stirred at RT for a 24 h. When necessary to reach completion of the rxn an extra amount of NaH (0.5 eq, as a 60% dispersion in mineral oil) and/or the bromide (0.5 eq) is added. The mixture is quenched with water or a sat. aq. soln. of NaHCO₃ and extracted with EtOAc. The combined org. phases are washed with brine, dried over MgSO₄ and concentrated in vacuo. The crude is purified by CC using Hept/EtOAc.

t_{R} [min] (LC/MS method): 1.31 (I); m/z [M+H]⁺ 618.38.

### Synthesis of 5-[1-(2-Fluoro-6-methyl-phenyl)-piperidin-4-yl]-7-(2-trifluoromethyl-benzyl)-2,4,5,7-tetrahydro-pyrazolo[3,4-d]pyrimidin-6-one

### Step A (TFA treatment):

To a soln. of SEM-protected intermediate 5-[1-(2-Fluoro-6-methyl-phenyl)-piperidin-4-yl]-7-(2-trifluoromethylbenzyl)-2-(2-trimethylsilanyl-ethoxymethyl)-2,4,5,7-tetrahydro-pyrazolo[3,4-d]pyrimidin-6-one (1 eq) in DCM (2 to 4 mL/mmol) is added dropwise TFA (4 to 6 mL/mmol). The soln. is stirred at RT for 2.5 h, quenched at 0°C with a 32% or 1M aq. soln. of NaOH until pH 7-8 and extracted with DCM. The combined org. phases are dried over MgSO₄ and concentrated in vacuo.

### Step B (additional treatment):

The crude is dissolved in THF (5 to 10 mL/mmol) and treated with ethylenediamine (3 eq) for 30 min to 1h at 60°C. The rxn mixture is partitioned between DCM and water and the org. phase is washed with brine, dried over MgSO₄ and concentrated in vacuo. When necessary, the crude is purified by CC using Hept/EtOAc.

t_{R} [min] (LC/MS method): 1.14 (I); m/z [M+H]⁺ 488.24.

### Example 1: Synthesis of 2-(2,2-Difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one (COMPOUND in amorphous form)

To a mixture of 5-[1-(2-Fluoro-6-methyl-phenyl)-piperidin-4-yl]-7-(2-trifluoromethyl-benzyl)-2,4,5,7-tetrahydro-pyrazolo[3,4-d]pyrimidin-6-one (1 eq) and 2,2-Difluoro propanol (1.5 to 2 eq) in toluene (6 to 12 mL/mmol) is added a 1M soln. of (tributylphosphoranylidene)acetonitrile in toluene (2 eq) under argon. The rxn mixture is heated to 110 °C and stirred for 5 h. It is quenched with water and extracted with EtOAc or DCM. The combined org. phases are washed with brine, dried over MgSO₄ and concentrated in vacuo. The crude is purified by CC using Hept/EtOAc.

t_{R} [min] (LC/MS method): 1.24 (I); m/z [M+H]⁺ 566.14.

XRPD pattern: see Fig. 2.

### Example 2: Synthesis of 2-(2,2-Difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one in crystalline form 1

### COMPOUND in amorphous form as obtained from Example 1 (20 g) is suspended EtOH (7 vol) and the suspension heated to IT=78°C to form a clear solution. The solution is cooled to IT=0°C and stirred at IT=0°C for 11h. The product is filtered, washed with 2 vol cold EtOH and dried under vacuum at 40°C. 16.5 g 2-(2,2-Difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one in crystalline form 1 are obtained in 82 % yield. Melting point: 163°C.

XRPD pattern: see Fig. 1.

Hygroscopicity of COMPOUND in crystalline form 1 as measured by GVS: non-hygroscopic.

Stability testing of COMPOUND in crystalline form 1:

| Storage condition About 10mg of powder in a 4mL white glass vial | HPLC 1 area compared to a reference that was stored at -20°C for the same amount of time (at 260nm on diode array detector) | |
|---|---|---|
| | Form 1 | Amorphous |
| Closed vial for 30 days at room temperature and day light exposure | 100% | 57% |
| Closed vial for 30 days at 40°C and 75% relative humidity in a storage cabinet | 100% | 79% |

### II. Biological Assay

### Example 3: In vitro assay

Adherent cells (CHO-K1 C5AR1 beta-arrestin cell line, DiscoverX, CA USA) are washed with PBS, detached by incubation with Dissociation Buffer (Gibco Cat# 13151-014, 2 ml per 165 cm2 dish) for 3 minutes, then washed with 10 ml PBS (without Mg++ and Ca++) and counted. 7'500 cells/384-well are seeded in 384-well plates (Cell culture plate MTP384 white Polystyrene, Corning, Cat# 3570) in 20 µl/well Cell plating medium (F12 HAMs/10% FCS/1% P/S) and incubated at 37°C / 5% CO2 / 24h.

5 µl Antagonist at 6-fold end concentration or DMSO control is added to assay medium and subsequently 5 µl 1 - 10 nM C5a agonist at 6 fold end concentration. Cells are centrifuged for 1 min at 1000 rpm and incubated for 1.5 hour in at 37°C. Plates are equilibrated at room temperature for several minutes before adding 12 µl/well Detection Reagent (PathHunter Detection Kit, DiscoverX, Cat# 93-0001). Plates are centrifuged for 1 min at 1000 rpm and incubated for 45 minutes at RT before being measured on a Fluostar Optima, BMG Labtech. IC₅₀ values are calculated from a serial dilution range of antagonist using inhouse software and given in nmol/l.

The calculated IC₅₀ values may fluctuate depending on the daily cellular assay performance. Fluctuations of this kind are known to those skilled in the art. Average IC₅₀ values from several measurements are given as geometric mean values.

The antagonistic activity of COMPOUND was tested in this assay at 11 nM.

### III. Preparation of Pharmaceutical compositions

COMPOUND is used in the following Examples in crystalline form 1 of Example 2.

The wet gelatine shell for the above capsules may for example be composed as follows, wherein it is understood that small changes in the respective amounts may occur from batch to batch:

| **Material** | **(mg/capsule)** | **(%w/w)** | **Function** |
|---|---|---|---|
| Gelatin | 170.7 | 42.3 | Shell |
| Polysorb 85/70/00 | 98.0 | 24.3 | Plasticizer |
| Purified Water | 126.4 | 31.4 | Solvent |
| Titanium dioxide | 7.9 | 2.0 | Opacifier |
| **approximate total wet shell weight** | **403** | **100** | |

Processing aids are medium chain triglycerides and lecithin, which are used as lubricants.

The corresponding dry gelatin shell is for example composed as follows:

| **Material** | **(mg/capsule)** |
|---|---|
| Gelatin | 171 |
| Polysorb 85/70/00 | 98 |
| Purified Water | N/A |
| Titanium dioxide | 8 |
| **approximate total dry shell weight** | **277** |

Alternatively, the wet gelatine shell for the above capsules may for example be composed as follows:

| **Material** | **(mg/capsule)** | **(%w/w)** | **Function** |
|---|---|---|---|
| Gelatin | 169.9 | 42.15 | Shell |
| Polysorb 85/70/00 | 42.3 | 10.5 | Plasticizer |
| Glycerin | 44.5 | 11.0 | |
| Purified Water | 138.5 | 34.35 | Solvent |
| Titanium dioxide | 7.9 | 2.0 | Opacifier |
| **approximate total wet shell weight** | **403** | **100** | |

Processing aids are medium chain triglycerides and lecithin, which are used as lubricants.

The corresponding dry gelatin shell is for example composed as follows:

| **Material** | **(mg/capsule)** |
|---|---|
| Gelatin | 170 |
| Polysorb 85/70/00 | 42 |
| Glycerin | 45 |
| Purified Water | N/A |
| Titanium dioxide | 8 |
| **approximate total dry shell weight** | **265** |

### Example 4:

Soft gelatine capsules (oval, size 12) of 667 mg per capsule are prepared as follows:

| **Material** | **Unit Dose (mg/capsule)** | **Percentage (%w/w)** | **Function** |
|---|---|---|---|
| COMPOUND | 0.5 | 0.075 | Active Ingredient |
| Capryol^{™} 90 | 197.87 | 29.67 | Lipophilic excipient / Hydrophobic surfactant |

| | | | |
|---|---|---|---|
| Kolliphor^{®} RH40 | 329.77 | 49.44 | Hydrophilic surfactant |
| Triethyl citrate | 131.87 | 19.77 | Hydrophilic co-solvent |
| Ascorbyl palmitate | 6.67 | 1 | Antioxidant |
| DL-alpha-Tocopheryl acetate | 0.334 | 0.05 | Antioxidant |
| **Total** | **667** | **100** | |

### Example 4a:

Alternatively, soft gelatine capsules (oval, size 12) of 667 mg per capsule are prepared as follows:

| **Material** | **Unit Dose (mg/capsule)** | **Percentage (%w/w)** | **Function** |
|---|---|---|---|
| COMPOUND | 0.5 | 0.075 | Active Ingredient |
| Capryol^{™} 90 | 199.65 | 29.933 | Lipophilic excipient / Hydrophobic surfactant |
| Kolliphor^{®} RH40 | 332.75 | 49.888 | Hydrophilic surfactant |
| Triethyl citrate | 133.10 | 19.955 | Hydrophilic co-solvent |
| Ascorbyl palmitate | 0.667 | 0.100 | Antioxidant |
| DL-alpha-Tocopheryl acetate | 0.334 | 0.050 | Antioxidant |
| **Total** | **667** | **100** | |

The capsules of Example 4 or Example 4a are prepared according to the following process:
The raw materials needed for the preparation are weighed. Each excipient is weighed in one or several separate and identified containers. The Kolliphor^{®} RH40, solid at room temperature is liquefied in a heated tunnel at 55°C until its total liquefaction before weighing.

Then, the fill preparation is performed on a closed homogenizer mixer system (Becomix RW15) according to the following steps:
- Kolliphor^{®} RH40, Triethyl citrate and Capryol^{™} 90 transfer by aspiration into preheated closed homogenizer mixer system.
- Excipients mixing under vacuum at 45°C until visually homogeneous. A visual check is performed for excipients mix homogeneity.
- Cooling of the fill at 35°C.
- Premix antioxidants / triethyl citrate preparation.
- Vacuum transfer of antioxidants / triethyl citrate premix into the closed homogenizer mixer system.
- Formulation mixing under vacuum at 35°C until visually homogeneous. A blocking microscopic check is performed after 1h of stirring to confirm solubilization of antioxidants.
- COMPOUND weighing is handled in protective equipment (isolator or equivalent), premixed with a part of the aliquot of Capryol^{™} 90. This mixture is stirred with a disposable spatula to obtain a homogeneous mixture of wet powder.
- COMPOUND/Capryol^{™} 90 premix transfer by aspiration into the closed homogenizer mixer system and premix recipient rinsing with the remaining part of Capryol^{™} 90. A visual check is performed for complete transfer of COMPOUND to the closed homogenizer mixer system.
- Transfer of the rinsing Capryol^{™} 90 in the closed homogenizer mixer system.
- Capryol^{™} 90 transfer into the closed homogenizer mixer system by aspiration to rinse the flexible pipes.
- Fill formulation mixing until COMPOUND is fully solubilized. A visual check is performed for absence of crystals in the fill material and a blocking IPC is performed to confirm by assay COMPOUND solubilization.
- Deaeration of the fill solution under vacuum. A visual check is performed for absence of air bubbles in the fill material.
- Fill mix discharge into a receiver throughout a 100 µm filter.
- Storage vessel blanket with nitrogen waiting for encapsulation.

### Encapsulation Step

- The encapsulation machine is of the rotary die type. It provides a continuous form, fill, and seal operation.
- The machine is fed by two receivers. One contains the melted gel mass used to form the shell, the other contains the fill. The temperature of the gelatin receiver is maintained between 50 and 65°C.
- The melted gel mass flows by gravity through heated tubes to two heated spreader boxes (the melted gel mass is filtered through a 200 µm nylon filter). The spreader boxes simultaneously cast the gelatin mass in two ribbons, which are lubricated with Medium Chain Triglyceride (0.3% of Soya Lecithin is added to the MCT for the lubrication of the external face of the ribbon) and delivered to the rotary dies.
- The two (2) gel ribbons are fed between the two (2) rotating dies. The dies contain paired pockets (12 Oval), which form the shape of the capsule and provide the sealing mechanism.
- At the precise moment that two die half pockets line up, the fill material is injected through an encapsulation wedge between the gelatin ribbons (the fill material flows by gravity into the hopper to the input of the encapsulation pump. The fill material is delivered to the filling point by way of the positive displacement of the piston pump). The fill is maintained under Nitrogen into the vessel and the hopper during the whole encapsulation.
- The seal forms as a result of pressure between the dies and heat applied by the encapsulation wedge.
- A first drying occurs immediately after encapsulation in a rotary dryer system physically attached to the encapsulation machine at 32°C ± 5°C (dry air). The capsules are tumbled dried for a predetermined length of time.

In analogy to Example 4, soft gelatine capsules (oval, size 12) of 667 mg per capsule are prepared as follows:

### Example 5:

| **Material** | **Unit Dose (mg/capsule)** | **Percentage (%w/w)** | **Function** |
|---|---|---|---|
| COMPOUND | 1 | 0.15 | Active Ingredient |
| Capryol^{™} 90 | 197.73 | 29.65 | Lipophilic excipient / Hydrophobic surfactant |
| Kolliphor^{®} RH40 | 329.47 | 49.40 | Hydrophilic surfactant |
| Triethyl citrate | 131.8 | 19.76 | Hydrophilic co-solvent |
| Ascorbyl palmitate | 6.67 | 1 | Antioxidant |
| DL-alpha-Tocopheryl acetate | 0.334 | 0.05 | Antioxidant |
| **Total** | **667** | **100** | |

### Example 6:

| **Material** | **Unit Dose (mg/capsule)** | **Percentage (%w/w)** | **Function** |
|---|---|---|---|
| COMPOUND | 5 | 0.75 | Active Ingredient |
| Capryol^{™} 90 | 196.50 | 29.46 | Lipophilic excipient / Hydrophobic surfactant |
| Kolliphor^{®} RH40 | 327.50 | 49.10 | Hydrophilic surfactant |
| Triethyl citrate | 131 | 19.64 | Hydrophilic co-solvent |
| Ascorbyl palmitate | 6.67 | 1 | Antioxidant |
| DL-alpha-Tocopheryl acetate | 0.334 | 0.05 | Antioxidant |
| **Total** | **667** | **100** | |

### Example 7:

| **Material** | **Unit Dose (mg/capsule)** | **Percentage (%w/w)** | **Function** |
|---|---|---|---|
| COMPOUND | 20 | **3** | Active Ingredient |
| Capryol^{™} 90 | 192.0 | 28.79 | Lipophilic excipient / Hydrophobic surfactant |
| Kolliphor^{®} RH40 | 320 | 47.98 | Hydrophilic surfactant |
| Triethyl citrate | 128 | 19.19 | Hydrophilic co-solvent |
| Ascorbyl palmitate | 6.67 | 1 | Antioxidant |
| DL-alpha-Tocopheryl acetate | 0.334 | 0.05 | Antioxidant |
| **Total** | **667** | **100** | |

### Example 7a:

Alternatively, soft gelatine capsules (oval, size 12) of 667 mg per capsule are prepared as follows:

| **Material** | **Unit Dose (mg/capsule)** | **Percentage (%w/w)** | **Function** |
|---|---|---|---|
| COMPOUND | 20 | 3 | Active Ingredient |
| Capryol^{™} 90 | 193.80 | 29.06 | Lipophilic excipient / Hydrophobic surfactant |
| Kolliphor^{®} RH40 | 323.00 | 48.43 | Hydrophilic surfactant |
| Triethyl citrate | 129.20 | 19.37 | Hydrophilic co-solvent |
| Ascorbyl palmitate | 0.667 | 0.1 | Antioxidant |
| DL-alpha-Tocopheryl acetate | 0.334 | 0.05 | Antioxidant |
| **Total** | **667** | **100** | |

### Example 8:

| **Material** | **Unit Dose (mg/capsule)** | **Percentage (%w/w)** | **Function** |
|---|---|---|---|
| COMPOUND | 1 | 0.15 | Active Ingredient |
| Capryol^{™} 90 | 199.5 | 29.91 | Lipophilic excipient / Hydrophobic surfactant |
| Kolliphor^{®} RH40 | 332.5 | 49.85 | Hydrophilic surfactant |
| Triethyl citrate | 133 | 19.94 | Hydrophilic co-solvent |
| Ascorbyl palmitate | 0.667 | 0.1 | Antioxidant |
| DL-alpha-Tocopheryl acetate | 0.334 | 0.05 | Antioxidant |
| **Total** | **667** | **100** | |

### Example 9:

| **Material** | **Unit Dose (mg/capsule)** | **Percentage (%w/w)** | **Function** |
|---|---|---|---|
| COMPOUND | 20 | 3 | Active Ingredient |
| Labrafac^{™} PG | 258.529 | 38.76 | Lipophilic excipient / oil-like excipient |
| Kolliphor^{®} EL | 323.162 | 48.45 | Hydrophilic surfactant |
| ethanol | 64.632 | 9.69 | Hydrophilic co-solvent |
| propyl gallate | 0.667 | 0.1 | Antioxidant |
| **Total** | **667** | **100** | |

### Example 10:

| **Material** | **Unit Dose (mg/capsule)** | **Percentage (%w/w)** | **Function** |
|---|---|---|---|
| COMPOUND | 20 | 3 | Active Ingredient |
| Labrafac^{™} PG | 258.529 | 38.76 | Lipophilic excipient / oil-like excipient |
| Kolliphor^{®} EL | 323.162 | 48.45 | Hydrophilic surfactant |
| Transcutol^{®} HP | 64.632 | 9.69 | Hydrophilic co-solvent |
| propyl gallate | 0.667 | 0.1 | Antioxidant |
| **Total** | **667** | **100** | |

### Example 11:

| **Material** | **Unit Dose (mg/capsule)** | **Percentage (%w/w)** | **Function** |
|---|---|---|---|
| COMPOUND | 15 | 2.25 | Active Ingredient |
| Miglyol^{®} 812 | 260.53 | 39.06 | Lipophilic excipient / oil-like excipient |
| Kolliphor^{®} EL | 325.696 | 48.83 | Hydrophilic surfactant |
| Transcutol^{®} HP | 65.099 | 9.76 | Hydrophilic co-solvent |
| propyl gallate | 0.667 | 0.1 | Antioxidant |
| **Total** | **667** | **100** | |

### Example 12:

| **Material** | **Unit Dose (mg/capsule)** | **Percentage (%w/w)** | **Function** |
|---|---|---|---|
| COMPOUND | 15 | 2.25 | Active Ingredient |
| Labrafac^{™} PG | 260.530 | 39.06 | Lipophilic excipient / oil-like excipient |
| Vit E TPGS | 390.795 | 58.59 | Hydrophilic surfactant |
| propyl gallate | 0.667 | 0.1 | Antioxidant |
| **Total** | **667** | **100** | |

### Example 13:

| **Material** | **Unit Dose (mg/capsule)** | **Percentage (%w/w)** | **Function** |
|---|---|---|---|
| COMPOUND | 15 | 2.25 | Active Ingredient |
| Labrafac^{™} PG | 260.530 | 39.06 | Lipophilic excipient / oil-like excipient |
| Gelucire^{®} 48/16 | 390.795 | 58.59 | Hydrophilic surfactant |
| propyl gallate | 0.667 | 0.1 | Antioxidant |
| **Total** | **667** | **100** | |

### Example 14:

| **Material** | **Unit Dose (mg/capsule)** | **Percentage (%w/w)** | **Function** |
|---|---|---|---|
| COMPOUND | 10 | 1.5 | Active Ingredient |
| Miglyol^{®} 812 | 262.531 | 39.36 | Lipophilic excipient / oil-like excipient |
| Kolliphor^{®} EL | 393.797 | 59.04 | Hydrophilic surfactant |
| propyl gallate | 0.667 | 0.1 | Antioxidant |
| **Total** | **667** | **100** | |

### Example 15:

| **Material** | **Unit Dose (mg/capsule)** | **Percentage (%w/w)** | **Function** |
|---|---|---|---|
| COMPOUND | 15 | 2.25 | Active Ingredient |
| Labrafac^{™} PG | 260.530 | 39.06 | Lipophilic excipient / oil-like excipient |
| Kolliphor^{®} EL | 390.795 | 58.59 | Hydrophilic surfactant |
| propyl gallate | 0.667 | 0.1 | Antioxidant |
| **Total** | **667** | **100** | |

### IV. Physical and chemical characterization of Example compositions:

### Challenge tests

For all challenge tests, the initial time point is reported after the complete COMPOUND solubilisation.

### Dispersion Studies

Dispersion studies are performed to check behavior of formulations under dilution (absence of COMPOUND precipitation). Dispersion study with the selected COMPOUND formulations is conducted in FaSSGF (pH1.6), FaSSIF (pH6.5) and FeSSIF (pH5.0). Approximately 5 mL of medium is placed in a vial and about 200 mg of the fill formulation are added to the medium at room temperature. The resulting mixture is vortex at the initial time point and then periodically inverted. Drug precipitation is checked over six (6) hours by microscopic observations.

### Temperature Cycling Study

A temperature cycling study is performed to assess, in accelerate condition, COMPOUND precipitation and formulation phase separation under temperature variation. Approximately 5g of selected fill COMPOUND formulations are alternatively stored 24h at 2-8 °C and 37 °C, over at least six (6) days. Samples are visually observed for clarity, particulate matter and phase separation.

### Hold Time study

The hold time study is performed to check any physical change of formulation aspect over time at room temperature. The selected fill COMPOUND formulations are stored at room temperature for, at least, seven (7) days. The samples are observed for a minimum of once a day e.g. for clarity, drug precipitation, drug crystallization, and phase separation. This test is indicative of the short-term physical stability of the formulation. Additionally, the physical aspects of the capsule fill contents may be visually checked.

### Water Challenge

The water challenge test is performed to check the absence of COMPOUND precipitation at the maximum water content that could be present in the fill formulation during product manufacture and storage (no water is added to the formulation as an ingredient, although the capsule shell can contain up to 8% water, some of which could potentially migrate into the fill formulation). The selected fill COMPOUND formulations are challenged by adding approximately from 6.5% to 10% of water to the formulation depending of the behavior of the COMPOUND into the formulation. Approximately 2.7 g of fill is weighed into a vial and approximately 0.3 g of water is added to reach 10% of water in the fill. The vial is vortexed for ten (10) seconds and left at ambient conditions to settle over a period of up to 10 days. Visual and microscopic observations are made (drug precipitation, phase separation, physical aspect).

### Plasticizer challenge

The plasticizers challenge test is performed to check the absence of COMPOUND precipitation at the worst-case plasticizer content that could be present in fill formulation due to their ingress from the capsule shell. The selected fill COMPOUND formulations are challenged by adding approximately 3% of plasticizer to the formulation. Approximately 2.9 g of fill is weighed into a vial and approximately 0.1 g of plasticizer is added. Glycerol and polysorb are evaluated. The vial is vortexed for ten (10) seconds and left at ambient conditions to settle over a period of 10 days. Visual and microscopic observations are made (drug precipitation, phase separation, physical aspect).

### Digestion test

*In vitro* digestion experiments can be performed in analogy to the described literature procedure (Cuiné J. et al. 2008. Evaluation of the impact of surfactant digestion on the bioavailability of danazol after oral administration of lipidic self-emulsifying formulations to dogs. J. Pharm. Sci. 97 (2), 995-1012). The digestion test is performed to check the absence of COMPOUND precipitation after "in-vitro" digestion of the formulation. Approximately 0.5 g of selected fill COMPOUND formulation is dissolved in 50 mL of SIF solution. The resulting mixture is magnetically stirred at 37°C during 3h. Microscopic observations are performed to check COMPOUND precipitation. The digestion test is also performed on placebo formulation (without COMPOUND) in parallel as a reference. The percentage of COMPOUND solubilized in the aqueous phase of the digestion aliquots taken during the digestion experiments can be determined by HPLC.

### Stability assessment of excipient solutions

Formulation samples for physical and chemical stability study follow-up can be prepared and tested for chemical and physical stability upon storage, e.g. at 40°C / 75% relative humidity in amber glass vials for 1 month, 3 months, or 12 months; using controlled by microscopic examinations and HPLC assays.

### Stability assessment of soft gelatine capsules

The stability at 25°C/60%RH and 40°C/75%RH of the capsules containing Example batches is assessed by HPLC for content and related substances using the following analytical method:
HPLC system: Waters UPLC Acquity H-Class or equivalent; Flow: 1.5 mL/min; Column temperature: 50°C; Autosampler temperature: 25°C; Injection volume: 3.0 µL; Column: Waters Cortecs Shield RP181.6 µm 2.1*150 mm; Wavelength: 250 nm; Sample concentration 20 µg/mL; Solvent A: Water + 0.05% TFA (v/v); Solvent B:
Acetonitrile + 0.05% TFA (v/v); Gradient:

| Time (min) % | solvent A % | solvent B |
|---|---|---|
| 0.0 | 98 | 2 |
| 20.0 | 5 | 95 |
| 20.5 | 0 | 100 |
| 27.0 | 0 | 100 |
| 27.5 | 98 | 2 |
| 32.0 | 98 | 2 |

The compositions of Examples 7, 7a, and 8 show less than 0.1 % w/w increase of impurities at 25 °C / 60 %RH at time points of 1, 3, 6, 9 and 12 months.

The compositions of Examples 7, 7a, and 8 show less than 0.1 % w/w increase of impurities at 40 °C / 75 %RH at time points of 1, 3, and 6 months.

The compositions of Examples 7, 7a, and 8 show stable results for content at 25 °C / 60 %RH at time points of 1, 3, 6, 9 and 12 months.

The compositions of Examples 7, 7a, and 8 show stable results for content at 40 °C / 75 %RH at time points of 1, 3, and 6 months.

Therefore, the capsules of all investigated batches may be considered stable for at least 12 months at 25 °C / 60 %RH and for at least 6 months at 40 °C / 75 %RH.

## Claims

1. A pharmaceutical composition which is a self-emulsifying drug delivery system (SEDDS), a self-microemulsifying drug delivery system (SMEDDS), or a self-nanoemulsifying drug delivery system (SNEDDS); said pharmaceutical composition comprising the compound 2-(2,2-difluoro-propyl)-5-[1-(2-fluoro-6-methylphenyl)-piperidin-4-yl]-7-(2-trifluoromethyl-benzyl)-2,4,5,7-tetrahydro-pyrazolo[3,4-d]pyrimidin-6-one: 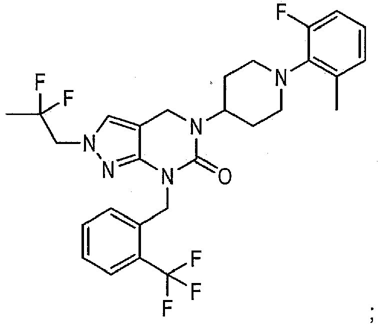
wherein said compound is in free base form, or in a pharmaceutically acceptable salt form;
wherein said pharmaceutical composition comprises a mixture of excipients comprising
• one or more lipophilic excipient(s);
• one or more hydrophilic surfactant(s); and
• optionally one or more hydrophilic co-solvent(s).

2. A pharmaceutical composition according to claim 1; comprising
• the compound 2-(2,2-Difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one: 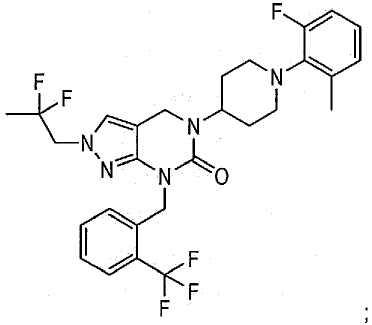 wherein said compound is in free base form, or in a pharmaceutically acceptable salt form;
and
• a mixture of excipients comprising:
> a total of 20±2 to 50±5 ww% of one or more lipophilic excipient(s), wherein said lipophilic excipient(s) is/are independently selected from
▪ hydrophobic surfactants selected from 1,2-propandiol medium chain mono-fatty acid esters and glycerin medium chain mono-ldi-fatty acid esters; and/or
▪ oil-like excipients selected from medium chain triglyceride oils and 1,2-propandiol medium chain di-fatty acid esters;
> a total of 30±3 to 80±8 ww% of one or more hydrophilic surfactant(s), wherein said hydrophilic surfactant(s) is/are independently selected from polyethyleneglycol derivatized long chain lipids and polyethyleneglycol derivatized glycerin medium chain mono-/di-fatty acid esters; and
> a total of 0 to 25±2.5 ww% of one or more hydrophilic co-solvents;
wherein the total ww% of said mixture of excipients is 100.

3. A pharmaceutical composition according to claim 1, comprising
• a total amount of 0.05±0.005 to 5±0.5 ww% of 2-(2,2-Difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one in free base form; and
• a total amount of at least 80±8 ww% of a mixture of excipients; wherein said mixture of excipients comprises:
> a total of 20±2 to 50±5 ww% of a lipophilic excipient, wherein said lipophilic excipient is a 1,2-propandiol medium chain mono-fatty acid ester;
a total of 30±3 to 80±8 ww% of a hydrophilic surfactant, wherein said hydrophilic surfactant is a polyethyleneglycol derivatized hydrogenated castor oil; and
a total of 0 to 25±2.5 ww% of one or two hydrophilic co-solvents;
> a total of 20±2 to 50±5 ww% of a lipophilic excipient, wherein said lipophilic excipient is a 1,2-propandiol medium chain mono-fatty acid ester;
a total of 30±3 to 80±8 ww% of a hydrophilic surfactant, wherein said hydrophilic surfactant is a polyethyleneglycol derivatized castor oil; and
a total of 0 to 25±2.5 ww% of one or two hydrophilic co-solvents;
> a total of 20±2 to 50±5 ww% of a lipophilic excipient, wherein said lipophilic excipient is a 1,2-propandiol medium chain mono-fatty acid ester;
a total of 30±3 to 80±8 ww% of a hydrophilic surfactant, wherein said hydrophilic surfactant is a polyethyleneglycol derivatized glycerin medium chain mono-/di-fatty acid ester; and
a total of 0 to 25±2.5 ww% of one or two hydrophilic co-solvents;
> a total of 20±2 to 50±5 ww% of a lipophilic excipient, wherein said lipophilic excipient is a glycerin medium chain tri-fatty acid ester;
a total of 30±3 to 80±8 ww% of a hydrophilic surfactant, wherein said hydrophilic surfactant is a polyethyleneglycol derivatized hydrogenated castor oil; and
a total of 0 to 25±2.5 ww% of one or two hydrophilic co-solvents;
> a total of 20±2 to 50±5 ww% of a lipophilic excipient, wherein said lipophilic excipient is a glycerin medium chain tri-fatty acid ester;
a total of 30±3 to 80±8 ww% of a hydrophilic surfactant, wherein said hydrophilic surfactant is a polyethyleneglycol derivatized castor oil; and
a total of 0 to 25±2.5 ww% of one or two hydrophilic co-solvents; or
> a total of 20±2 to 50±5 ww% of a lipophilic excipient, wherein said lipophilic excipient is 1,2-propandiol medium chain di-fatty acid ester;
a total of 30±3 to 80±8 ww% of a hydrophilic surfactant, wherein said hydrophilic surfactant is a polyethyleneglycol derivatized castor oil; and
a total of 0 to 25±2.5 ww% of one or two hydrophilic co-solvents;
wherein the total ww% of said mixture of excipients is 100; and
wherein the total ww% of the pharmaceutical composition is 100.

4. A pharmaceutical composition according to claim 1, comprising
• a total amount of 0.075±0.0075 to 4.5±0.45 ww% of 2-(2,2-Difluoro-propyl)-5-[1-(2-fluoro-6-methylphenyl)-piperidin-4-yl]-7-(2-trifluoromethyl-benzyl)-2,4,5,7-tetrahydro-pyrazolo[3,4-d]pyrimidin-6-one in free base form; and
• a total amount of at least 80±8 ww% of a mixture of excipients; wherein said mixture of excipients comprises:
> a total of 20t2 to 40±4 ww% of a lipophilic excipient, wherein said lipophilic excipient is a 1,2-propandiol medium chain mono-fatty acid ester;
> a total of 40±4 to 70±7 ww% of a hydrophilic surfactant, wherein said hydrophilic surfactant is a polyethyleneglycol derivatized hydrogenated castor oil; and
> no hydrophilic co-solvent; or a total of 10±1 to 20±2 ww% of one or two hydrophilic co-solvents selected from triethyl citrate, ethanol, and diethylene glycol monoethylether;
wherein the total ww% of said mixture of excipients is 100; and
wherein the total ww% of the pharmaceutical composition is 100.

5. A pharmaceutical composition according to claim 1, consisting essentially of:
• a total amount of 0.075±0.0075 to 4.5±0.45 ww% of 2-(2,2-Difluoro-propyl)-5-[1-(2-fluoro-6-methylphenyl)-piperidin-4-yl]-7-(2-trifluoromethyl-benzyl)-2,4,5,7-tetrahydro-pyrazolo[3,4-d]pyrimidin-6-one in free base form; and
• a total amount of at least 90±9 ww% based on the total weight of the pharmaceutical composition of a mixture of excipients; wherein said mixture of excipients comprises:
> a total of 20±2 to 40±4 ww% of a lipophilic excipient, wherein said lipophilic excipient is a 1,2-propandiol medium chain mono-fatty acid ester;
> a total of 40±4 to 70±7 ww% of a hydrophilic surfactant, wherein said hydrophilic surfactant is a polyethyleneglycol derivatized hydrogenated castor oil; and
> no hydrophilic co-solvent; or a total of 10±1 to 20±2 ww% of one or two hydrophilic co-solvents selected from triethyl citrate, ethanol, and diethylene glycol monoethylether;
wherein the total ww% of said mixture of excipients is 100; and
• optionally one or two conventional ingredients or additives selected from one or two antioxidants selected from
> one oxygen scavenger in an amount of below 2±0.2 ww% based on the total weight of the pharmaceutical composition, and/or
> one chain terminator in an amount of below 0.3±0.03 ww% based on the total weight of the pharmaceutical composition;
wherein the total ww% of the pharmaceutical composition is 100.

6. A pharmaceutical composition according to claim 5, wherein said oxygen scavenger is present with respect to the total weight of the pharmaceutical composition in an amount of about 0.1 to 1 ww%.

7. A pharmaceutical composition according to claims 5 or 6, wherein said chain terminator is present with respect to the total weight of the pharmaceutical composition in an amount of about 0.05 to 0.2 ww%.

8. A pharmaceutical composition according any one of claims 1 to 7, wherein said pharmaceutical composition is filled into soft gelatine capsules.

9. A pharmaceutical composition according to any one of claims 1 to 8, wherein 2-(2,2-Difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one in crystalline form is used for the preparation of said composition.

10. A crystalline form of 2-(2,2-Difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one, **characterized by** the presence of peaks in the X-ray powder diffraction diagram at the following angles of refraction 2θ: 6.2°, 9.5°, and 14.4°; wherein said X-ray powder diffraction diagram is obtained by using combined Cu Kα1 and Kα2 radiation, without Kα2 stripping; and the accuracy of the 2θ values is in the range of 2θ +/- 0.2°.

11. A crystalline form of 2-(2,2-Difluoro-propyl)-5-[1-(2-fluoro-6-methyl-phenyl)-piperidin-4-yl]-7 -(2-trifluoromethyl-benzyl)-2,4,5,7-tetrahydro-pyrazolo[3,4-d]pyrimidin-6-one according to claim 10, **characterized by** the presence of peaks in the X-ray powder diffraction diagram at the following angles of refraction 2θ: 6.2°, 9.5°, 14.4°, 15.7°, and 18.6°; wherein said X-ray powder diffraction diagram is obtained by using combined Cu Kα1 and Kα2 radiation, without Kα2 stripping; and the accuracy of the 2θ values is in the range of 2θ +/- 0.2°.

12. A crystalline form of 2-(2,2-Difluoro-propyl)-5-[1-(2-fluoro-6-methyl-phenyl)-piperidin-4-yl]-7-(2-trifluoromethyl-benzyl)-2,4,5,7-tetrahydro-pyrazolo[3,4-d]pyrimidin-6-one according to claim 11, which has a melting point of about 163°C as determined by differential scanning calorimetry.

13. A pharmaceutical composition according to any one of claims 1 to 9 for use as a medicament.

14. A pharmaceutical composition according to any one of claims 1 to 9 for use in the prevention / prophylaxis or treatment of diseases and disorders related to pathogenic events associated with elevated levels of C5a and/or with C5aR activation.

15. A pharmaceutical composition according to any one of claims 1 to 9 for use in the prevention / prophylaxis or treatment of diseases and disorders selected from vasculitic diseases or disorders, inflammatory diseases or disorders involving intravascular microvesicle release, immune complex (IC) diseases or disorders, neurodegenerative diseases or disorders, complement related inflammatory diseases or disorders, bullous diseases or disorders, diseases or disorders related to ischemia and/or ischemic reperfusion injury, inflammatory bowel diseases or disorders, autoimmune diseases or disorders, and cancer.

## Patentansprüche

1. Pharmazeutische Zusammensetzung, die ein selbstemulgierendes Arzneimittelabgabesystem (SEDDS), ein selbstmikroemulgierendes Arzneimittelabgabesystem (SMEDDS) oder ein selbstnanoemulgierendes Arzneimittelabgabesystem (SNEDDS) ist; wobei die genannte pharmazeutische Zusammensetzung die Verbindung 2-(2,2-Difluor-propyl)-5-[1-(2-fluor-6-methyl-phenyl)-piperidin-4-yl]-7-(2-trifluormethyl-benzyl)-2,4,5,7-tetrahydro-pyrazolo[3,4-d]pyrimidin-6-on umfasst: 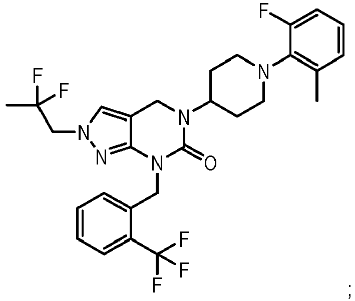
wobei die genannte Verbindung in Form der freien Base oder in einer pharmazeutisch akzeptablen Salzform vorliegt;
wobei die genannte pharmazeutische Zusammensetzung ein Gemisch von Hilfsstoffen umfasst, das Folgendes umfasst:
• einen oder mehrere lipophile Hilfsstoffe;
• ein oder mehrere hydrophile Tenside; und
• optional ein oder mehrere hydrophile Co-Lösungsmittel.

2. Pharmazeutische Zusammensetzung nach Anspruch 1; die Folgendes umfasst:
• die Verbindung 2-(2,2-Difluor-propyl)-5-[1-(2-fluor-6-methyl-phenyl)-piperidin-4-yl]-7-(2-trifluormethyl-benzyl)-2,4,5,7-tetrahydro-pyrazolo[3,4-d]pyrimidin-6-on: 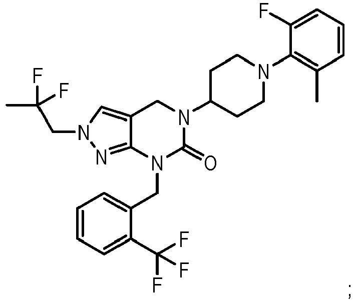 wobei die genannte Verbindung in Form der freien Base oder in einer pharmazeutisch akzeptablen Salzform vorliegt;
und
• ein Gemisch von Hilfsstoffen, das Folgendes umfasst:
> insgesamt 20±2 bis 50±5 Gew.-% eines oder mehrerer lipophiler Hilfsstoffe, wobei der/die genannte(n) lipophile(n) Hilfsstoff(e) unabhängig ausgewählt ist/sind aus
▪ hydrophoben Tensiden, ausgewählt aus mittelkettigen 1,2-Propandiol-Monofettsäureestern und mittelkettigen Glycerin-Mono-/Di-Fettsäureestern; und/oder
▪ ölartigen Hilfsstoffen, ausgewählt aus mittelkettigen Triglyceridölen und mittelkettigen 1,2-Propandiol-Difettsäureestern;
> insgesamt 30±3 bis 80±8 Gew.-% eines oder mehrerer hydrophiler Tenside, wobei das/die genannte(n) hydrophile(n) Tensid(e) unabhängig ausgewählt ist/sind aus Polyethylenglykol-derivatisierten langkettigen Lipiden und Polyethylenglykol-derivatisierten mittelkettigen Glycerin-Mono-/Di-Fettsäureestern; und
> insgesamt 0 bis 25±2,5 Gew.-% eines oder mehrerer hydrophiler Co-Lösungsmittel; wobei der Gesamtgewichtsanteil des genannten Gemischs von Hilfsstoffen 100 beträgt.

3. Pharmazeutische Zusammensetzung nach Anspruch 1, die Folgendes umfasst:
• eine Gesamtmenge von 0,05±0,005 bis 5±0,5 Gew.-% 2-(2,2-Difluor-propyl)-5-[1-(2-fluor-6-methylphenyl)-piperidin-4-yl]-7-(2-trifluormethyl-benzyl)-2,4,5,7-tetrahydro-pyrazolo[3,4-d]pyrimidin-6-on in Form der freien Base; und
• eine Gesamtmenge von mindestens 80±8 Gew.-% eines Gemischs von Hilfsstoffen; wobei das genannte Gemisch von Hilfsstoffen Folgendes umfasst:
> insgesamt 20±2 bis 50±5 Gew.-% eines lipophilen Hilfsstoffs, wobei der genannte lipophile Hilfsstoff ein mittelkettiger 1,2-Propandiol-Monofettsäureester ist;
insgesamt 30±3 bis 80±8 Gew.-% eines hydrophilen Tensids, wobei das genannte hydrophile Tensid ein Polyethylenglykol-derivatisiertes hydriertes Rizinusöl ist; und
insgesamt 0 bis 25±2,5 Gew.-% von einem oder zwei hydrophilen Co-Lösungsmitteln;
> insgesamt 20±2 bis 50±5 Gew.-% eines lipophilen Hilfsstoffs, wobei der genannte lipophile Hilfsstoff ein mittelkettiger 1,2-Propandiol-Monofettsäureester ist;
insgesamt 30±3 bis 80±8 Gew.-% eines hydrophilen Tensids, wobei das genannte hydrophile Tensid ein Polyethylenglykol-derivatisiertes Rizinusöl ist; und
insgesamt 0 bis 25±2,5 Gew.-% von einem oder zwei hydrophilen Co-Lösungsmitteln;
> insgesamt 20±2 bis 50±5 Gew.-% eines lipophilen Hilfsstoffs, wobei der genannte lipophile Hilfsstoff ein mittelkettiger 1,2-Propandiol-Monofettsäureester ist;
insgesamt 30±3 bis 80±8 Gew.-% eines hydrophilen Tensids, wobei das genannte hydrophile Tensid ein Polyethylenglykol-derivatisierter mittelkettiger Glycerin-Mono-/Di-Fettsäureester ist; und
insgesamt 0 bis 25±2,5 Gew.-% von einem oder zwei hydrophilen Co-Lösungsmitteln;
> insgesamt 20±2 bis 50±5 Gew.-% eines lipophilen Hilfsstoffs, wobei der genannte lipophile Hilfsstoff ein mittelkettiger Glycerin-Trifettsäureester ist;
insgesamt 30±3 bis 80±8 Gew.-% eines hydrophilen Tensids, wobei das genannte hydrophile Tensid ein Polyethylenglykol-derivatisiertes hydriertes Rizinusöl ist; und
insgesamt 0 bis 25±2,5 Gew.-% von einem oder zwei hydrophilen Co-Lösungsmitteln;
> insgesamt 20±2 bis 50±5 Gew.-% eines lipophilen Hilfsstoffs, wobei der genannte lipophile Hilfsstoff ein mittelkettiger Glycerin-Trifettsäureester ist;
insgesamt 30±3 bis 80±8 Gew.-% eines hydrophilen Tensids, wobei das genannte hydrophile Tensid ein Polyethylenglykol-derivatisiertes Rizinusöl ist; und
insgesamt 0 bis 25±2,5 Gew.-% von einem oder zwei hydrophilen Co-Lösungsmitteln; oder
> insgesamt 20±2 bis 50±5 Gew.-% eines lipophilen Hilfsstoffs, wobei der genannte lipophile Hilfsstoff ein mittelkettiger 1,2-Propandiol-Difettsäureester ist;
insgesamt 30±3 bis 80±8 Gew.-% eines hydrophilen Tensids, wobei das genannte hydrophile Tensid ein Polyethylenglykol-derivatisiertes Rizinusöl ist; und
insgesamt 0 bis 25±2,5 Gew.-% von einem oder zwei hydrophilen Co-Lösungsmitteln;
wobei der Gesamtgewichtsanteil des genannten Gemischs von Hilfsstoffen 100 beträgt; und
wobei der Gesamtgewichtsanteil der pharmazeutischen Zusammensetzung 100 beträgt.

4. Pharmazeutische Zusammensetzung nach Anspruch 1, die Folgendes umfasst:
• eine Gesamtmenge von 0,075±0,0075 bis 4,5±0,45 Gew.-% 2-(2,2-Difluor-propyl)-5-[1-(2-fluor-6-methyl-phenyl)-piperidin-4-yl]-7-(2-trifluormethyl-benzyl)-2,4,5,7-tetrahydro-pyrazolo[3,4-d]pyrimidin-6-on in Form der freien Base; und
• eine Gesamtmenge von mindestens 80±8 Gew.-% eines Gemischs von Hilfsstoffen; wobei das genannte Gemisch von Hilfsstoffen Folgendes umfasst:
> insgesamt 20±2 bis 40±4 Gew.-% eines lipophilen Hilfsstoffs, wobei der genannte lipophile Hilfsstoff ein mittelkettiger 1,2-Propandiol-Monofettsäureester ist;
> insgesamt 40±4 bis 70±7 Gew.-% eines hydrophilen Tensids, wobei das genannte hydrophile Tensid ein Polyethylenglykol-derivatisiertes hydriertes Rizinusöl ist; und
> kein hydrophiles Co-Lösungsmittel; oder insgesamt 10±1 bis 20±2 Gew.-% von einem oder zwei hydrophilen Co-Lösungsmitteln, ausgewählt aus Triethylcitrat, Ethanol und Diethylenglykolmonoethylether;
wobei der Gesamtgewichtsanteil des genannten Gemischs von Hilfsstoffen 100 beträgt; und
wobei der Gesamtgewichtsanteil der pharmazeutischen Zusammensetzung 100 beträgt.

5. Pharmazeutische Zusammensetzung nach Anspruch 1, die im Wesentlichen besteht aus:
• einer Gesamtmenge von 0,075±0,0075 bis 4,5±0,45 Gew.-% 2-(2,2-Difluor-propyl)-5-[1-(2-fluor-6-methyl-phenyl)-piperidin-4-yl]-7-(2-trifluormethyl-benzyl)-2,4,5,7-tetrahydro-pyrazolo[3,4-d]pyrimidin-6-on in Form der freien Base; und
• einer Gesamtmenge von mindestens 90±9 Gew.-% auf der Basis des Gesamtgewichts der pharmazeutischen Zusammensetzung eines Gemischs von Hilfsstoffen; wobei das genannte Gemisch von Hilfsstoffen Folgendes umfasst:
> insgesamt 20±2 bis 40±4 Gew.-% eines lipophilen Hilfsstoffs, wobei der genannte lipophile Hilfsstoff ein mittelkettiger 1,2-Propandiol-Monofettsäureester ist;
> insgesamt 40±4 bis 70±7 Gew.-% eines hydrophilen Tensids, wobei das genannte hydrophile Tensid ein Polyethylenglykol-derivatisiertes hydriertes Rizinusöl ist; und
> kein hydrophiles Co-Lösungsmittel; oder insgesamt 10±1 bis 20±2 Gew.-% von einem oder zwei hydrophilen Co-Lösungsmitteln, ausgewählt aus Triethylcitrat, Ethanol und Diethylenglykolmonoethylether;
wobei der Gesamtgewichtsanteil des genannten Gemischs von Hilfsstoffen 100 beträgt; und
• optional einem oder zwei herkömmlichen Bestandteilen oder Zusatzstoffen, ausgewählt aus einem oder zwei Antioxidantien, ausgewählt aus
> einem Sauerstofffänger in einer Menge von weniger als 2±0,2 Gew.-% auf der Basis des Gesamtgewichts der pharmazeutischen Zusammensetzung, und/oder
> einem Kettenabbrecher in einer Menge von weniger als 0,3±0,03 Gew.-% auf der Basis des Gesamtgewichts der pharmazeutischen Zusammensetzung;
wobei der Gesamtgewichtsanteil der pharmazeutischen Zusammensetzung 100 beträgt.

6. Pharmazeutische Zusammensetzung nach Anspruch 5, wobei der genannte Sauerstofffänger, bezogen auf das Gesamtgewicht der pharmazeutischen Zusammensetzung, in einer Menge von etwa 0,1 bis 1 Gew.-% vorhanden ist.

7. Pharmazeutische Zusammensetzung nach Anspruch 5 oder 6, wobei der genannte Kettenabbrecher, bezogen auf das Gesamtgewicht der pharmazeutischen Zusammensetzung, in einer Menge von etwa 0,05 bis 0,2 Gew.-% vorhanden ist.

8. Pharmazeutische Zusammensetzung nach einem der Ansprüche 1 bis 7, wobei die genannte pharmazeutische Zusammensetzung in Weichgelatinekapseln abgefüllt ist.

9. Pharmazeutische Zusammensetzung nach einem der Ansprüche 1 bis 8, wobei 2-(2,2-Difluor-propyl)-5-[1-(2-fluor-6-methyl-phenyl)-piperidin-4-yl]-7-(2-trifluormethyl-benzyl)-2,4,5,7-tetrahydro-pyrazolo[3,4-d]pyrimidin-6-on in kristalliner Form für die Herstellung der genannten Zusammensetzung verwendet wird.

10. Kristalline Form von 2-(2,2-Difluor-propyl)-5-[1-(2-fluor-6-methyl-phenyl)-piperidin-4-yl]-7-(2-trifluormethyl-benzyl)-2,4,5,7-tetrahydro-pyrazolo[3,4-d]pyrimidin-6-on, **gekennzeichnet durch** das Vorhandensein von Peaks im Röntgenpulverbeugungsdiagramm bei den folgenden Brechungswinkeln 2θ: 6,2°, 9,5° und 14,4°; wobei das genannte Röntgenpulverbeugungsdiagramm unter Verwendung von kombinierter Cu Kα1 und Kα2 Strahlung ohne Kα2 Stripping erhalten wird; und die Genauigkeit der 2θ Werte im Bereich von 2θ +/- 0,2° liegt.

11. Kristalline Form von 2-(2,2-Difluor-propyl)-5-[1-(2-fluor-6-methyl-phenyl)-piperidin-4-yl]-7-(2-trifluormethyl-benzyl)-2,4,5,7-tetrahydro-pyrazolo[3,4-d]pyrimidin-6-on nach Anspruch 10, **gekennzeichnet durch** das Vorhandensein von Peaks im Röntgenpulverbeugungsdiagramm bei den folgenden Brechungswinkeln 2θ: 6,2°, 9,5°, 14,4°, 15,7° und 18,6°; wobei das genannte Röntgenpulverbeugungsdiagramm unter Verwendung von kombinierter Cu Kα1 und Kα2 Strahlung ohne Kα2 Stripping erhalten wird; und die Genauigkeit der 2θ Werte im Bereich von 2θ +/- 0,2° liegt.

12. Kristalline Form von 2-(2,2-Difluor-propyl)-5-[1-(2-fluor-6-methyl-phenyl)-piperidin-4-yl]-7-(2-trifluormethyl-benzyl)-2,4,5,7-tetrahydro-pyrazolo[3,4-d]pyrimidin-6-on nach Anspruch 11, die einen Schmelzpunkt von etwa 163°C hat, bestimmt durch Differential-Scanning-Kalorimetrie.

13. Pharmazeutische Zusammensetzung nach einem der Ansprüche 1 bis 9 zur Verwendung als Medikament.

14. Pharmazeutische Zusammensetzung nach einem der Ansprüche 1 bis 9 zur Verwendung bei der Verhütung/Prophylaxe oder Behandlung von Krankheiten und Störungen im Zusammenhang mit pathogenen Ereignissen, die mit erhöhten C5a-Spiegeln und/oder mit C5aR-Aktivierung assoziiert sind.

15. Pharmazeutische Zusammensetzung nach einem der Ansprüche 1 bis 9 zur Verwendung bei der Verhütung/Prophylaxe oder Behandlung von Krankheiten und Störungen, ausgewählt aus vaskulitischen Krankheiten oder Störungen, entzündlichen Krankheiten oder Störungen, die eine intravaskuläre Mikrovesikelfreisetzung beinhalten, Immunkomplex-(IC)-Krankheiten oder -Störungen, neurodegenerativen Krankheiten oder Störungen, komplementbezogenen entzündlichen Krankheiten oder Störungen, bullösen Krankheiten oder Störungen, Krankheiten oder Störungen im Zusammenhang mit Ischämie und/oder ischämischer Reperfusionsverletzung, entzündlichen Darmerkrankungen oder -störungen, Autoimmunerkrankungen oder -störungen und Krebs.

## Revendications

1. Composition pharmaceutique qui est un système d'administration de médicaments auto-émulsionnant (SEDDS), un système d'administration de médicaments auto-microémulsionnant (SMEDDS) ou un système d'administration de médicaments auto-nanoémulsionnant (SNEDDS) ; ladite composition pharmaceutique comprenant le composé qu'est la 2-(2,2-difluoro-propyl)-5-[1-(2-fluoro-6-méthyl-phényl)-pipéridin-4-yl]-7-(2-trifluorométhyl-benzyl)-2,4,5,7-tétrahydro-pyrazolo[3,4-d]pyrimidin-6-one : 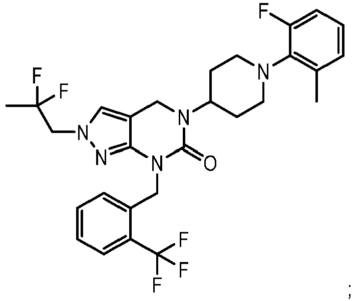
où ledit composé est sous la forme de la base libre ou sous la forme d'un sel pharmaceutiquement acceptable ;
où ladite composition pharmaceutique comprend un mélange d'excipients comprenant
• un ou plusieurs excipient(s) lipophile(s) ;
• un ou plusieurs surfactant(s) hydrophile(s) ; et
• éventuellement un ou plusieurs co-solvant(s) hydrophile(s).

2. Composition pharmaceutique selon la revendication 1 ; comprenant
• le composé qu'est la 2-(2,2-difluoro-propyl)-5-[1-(2-fluoro-6-méthyl-phényl)-pipéridin-4-yl]-7-(2-trifluorométhyl-benzyl)-2,4,5,7-tétrahydro-pyrazolo[3,4-d]pyrimidin-6-one : 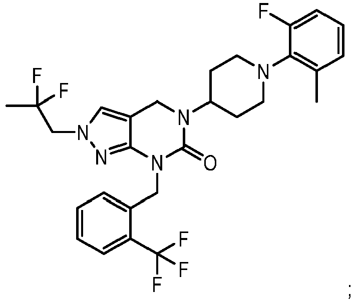 où ledit composé est sous la forme de la base libre ou sous la forme d'un sel pharmaceutiquement acceptable ;
et
• un mélange d'excipients comprenant :
> un total de 20±2 à 50±5 % p/p d'un ou de plusieurs excipient(s) lipophile(s), où le(s)dit(s) excipient(s) lipophile(s) est/sont indépendamment sélectionné(s) parmi
▪ des surfactants hydrophobes sélectionnés parmi des monoesters d'acides gras à chaîne moyenne et de 1,2-propanediol et des mono-/diesters d'acides gras à chaîne moyenne et de glycérine ; et/ou
▪ des excipients de type huile sélectionnés parmi des huiles à base de triglycérides à chaîne moyenne et des diesters d'acides gras à chaîne moyenne et de 1,2-propanediol ;
> un total de 30±3 à 80±8 % p/p d'un ou de plusieurs surfactant(s) hydrophile(s), où le(s)dit(s) surfactant(s) hydrophile(s) est/sont indépendamment sélectionné(s) parmi des lipides à chaîne longue dérivatisés par le polyéthylène glycol et des mono-/diesters d'acides gras à chaîne moyenne et de glycérine dérivatisés par le polyéthylène glycol ; et
> un total de 0 à 25±2,5 % p/p d'un ou de plusieurs co-solvant(s) hydrophile(s) ;
où le % p/p total dudit mélange d'excipients est égal à 100.

3. Composition pharmaceutique selon la revendication 1, comprenant
• une quantité totale de 2-(2,2-difluoro-propyl)-5-[1-(2-fluoro-6-méthyl-phényl)-pipéridin-4-yl]-7-(2-trifluorométhyl-benzyl)-2,4,5,7-tétrahydro-pyrazolo[3,4-d]pyrimidin-6-one sous la forme de la base libre qui va de 0,05±0,005 à 5±0,5 % p/p ; et
• une quantité totale d'un mélange d'excipients qui est d'au moins 80±8 % p/p ; où ledit mélange d'excipients comprend :
> un total de 20±2 à 50±5 % p/p d'un excipient lipophile, où ledit excipient lipophile est un monoester d'un acide gras à chaîne moyenne et de 1,2-propanediol ;
un total de 30±3 à 80±8 % p/p d'un surfactant hydrophile, où ledit surfactant hydrophile est une huile de ricin hydrogénée dérivatisée par le polyéthylène glycol ; et
un total de 0 à 25±2,5 % p/p d'un ou de deux co-solvant(s) hydrophile(s) ;
> un total de 20±2 à 50±5 % p/p d'un excipient lipophile, où ledit excipient lipophile est un monoester d'un acide gras à chaîne moyenne et de 1,2-propanediol ;
un total de 30±3 à 80±8 % p/p d'un surfactant hydrophile, où ledit surfactant hydrophile est une huile de ricin dérivatisée par le polyéthylène glycol ; et
un total de 0 à 25±2,5 % p/p d'un ou de deux co-solvant(s) hydrophile(s) ;
> un total de 20±2 à 50±5 % p/p d'un excipient lipophile, où ledit excipient lipophile est un monoester d'un acide gras à chaîne moyenne et de 1,2-propanediol ;
un total de 30±3 à 80±8 % p/p d'un surfactant hydrophile, où ledit surfactant hydrophile est un mono-/diester d'un acide gras à chaîne moyenne et de glycérine dérivatisé par le polyéthylène glycol ; et
un total de 0 à 25±2,5 % p/p d'un ou de deux co-solvant(s) hydrophile(s) ;
> un total de 20±2 à 50±5 % p/p d'un excipient lipophile, où ledit excipient lipophile est un triester d'un acide gras à chaîne moyenne et de glycérine ;
un total de 30±3 à 80±8 % p/p d'un surfactant hydrophile, où ledit surfactant hydrophile est une huile de ricin hydrogénée dérivatisée par le polyéthylène glycol ; et
un total de 0 à 25±2,5 % p/p d'un ou de deux co-solvant(s) hydrophile(s) ;
> un total de 20±2 à 50±5 % p/p d'un excipient lipophile, où ledit excipient lipophile est un triester d'un acide gras à chaîne moyenne et de glycérine ;
un total de 30±3 à 80±8 % p/p d'un surfactant hydrophile, où ledit surfactant hydrophile est une huile de ricin dérivatisée par le polyéthylène glycol ; et
un total de 0 à 25±2,5 % p/p d'un ou de deux co-solvant(s) hydrophile(s) ; ou
> un total de 20±2 à 50±5 % p/p d'un excipient lipophile, où ledit excipient lipophile est un diester d'un acide gras à chaîne moyenne et de 1,2-propanediol ;
un total de 30±3 à 80±8 % p/p d'un surfactant hydrophile, où ledit surfactant hydrophile est une huile de ricin dérivatisée par le polyéthylène glycol ; et
un total de 0 à 25±2,5 % p/p d'un ou de deux co-solvant(s) hydrophile(s) ;
où le % p/p total dudit mélange d'excipients est égal à 100 ; et
où le % p/p total de la composition pharmaceutique est égal à 100.

4. Composition pharmaceutique selon la revendication 1, comprenant
• une quantité totale de 2-(2,2-difluoro-propyl)-5-[1-(2-fluoro-6-méthyl-phényl)-pipéridin-4-yl]-7-(2-trifluorométhyl-benzyl)-2,4,5,7-tétrahydro-pyrazolo[3,4-d]pyrimidin-6-one sous la forme de la base libre qui va de 0,075±0,0075 à 4,5±0,45 % p/p ; et
• une quantité totale d'un mélange d'excipients qui est d'au moins 80±8 % p/p ; où le mélange d'excipients comprend :
> un total de 20±2 à 40±4 % p/p d'un excipient lipophile, où ledit excipient lipophile est un monoester d'un acide gras à chaîne moyenne et de 1,2-propanediol ;
> un total de 40±4 à 70±7 % p/p d'un surfactant hydrophile, où ledit surfactant hydrophile est une huile de ricin hydrogénée dérivatisée par le polyéthylène glycol ; et
> pas de co-solvant hydrophile ; ou un total de 10±1 à 20±2 % p/p d'un ou de deux co-solvant(s) hydrophile(s) sélectionné(s) parmi les suivants : citrate triéthylique, éthanol et monoéthyléther de diéthylène glycol ;
où le % p/p total dudit mélange d'excipients est égal à 100 ; et
où le % p/p total de la composition pharmaceutique est égal à 100.

5. Composition pharmaceutique selon la revendication 1 consistant essentiellement en :
• une quantité totale de 2-(2,2-difluoro-propyl)-5-[1-(2-fluoro-6-méthyl-phényl)-pipéridin-4-yl]-7-(2-trifluorométhyl-benzyl)-2,4,5,7-tétrahydro-pyrazolo[3,4-d]pyrimidin-6-one sous la forme de la base libre qui va de 0,075±0,0075 à 4,5±0,45 % p/p ; et
• une quantité totale d'un mélange d'excipients qui, rapportée au poids total de la composition pharmaceutique, est d'au moins 90±9 % p/p ; où le mélange d'excipients comprend :
> un total de 20±2 à 40±4 % p/p d'un excipient lipophile, où ledit excipient lipophile est un monoester d'un acide gras à chaîne moyenne et de 1,2-propanediol ;
> un total de 40±4 à 70±7 % p/p d'un surfactant hydrophile, où ledit surfactant hydrophile est une huile de ricin hydrogénée dérivatisée par le polyéthylène glycol ; et
> pas de co-solvant hydrophile ; ou un total de 10±1 à 20±2 % p/p d'un ou de deux co-solvant(s) hydrophile(s) sélectionné(s) parmi les suivants : citrate triéthylique, éthanol et monoéthyléther de diéthylène glycol ;
où le % p/p total dudit mélange d'excipients est égal à 100 ; et
• éventuellement un ou deux ingrédients ou additifs conventionnels sélectionnés parmi un ou deux antioxydants sélectionnés parmi les suivants :
> un piégeur d'oxygène à une quantité, rapportée au poids total de la composition pharmaceutique, inférieure à 2±0,2 % p/p, et/ou
> un terminateur de chaîne à une quantité, rapportée au poids total de la composition pharmaceutique, inférieure à 0,3±0,03 % p/p ;
où le % p/p total de la composition pharmaceutique est égal à 100.

6. Composition pharmaceutique selon la revendication 5, où ledit piégeur d'oxygène est présent à une quantité, rapportée au poids total de la composition pharmaceutique, qui va d'environ 0,1 à 1 % p/p.

7. Composition pharmaceutique selon la revendication 5 ou 6, où ledit terminateur de chaîne est présent à une quantité, rapportée au poids total de la composition pharmaceutique, qui va d'environ 0,05 à 0,2 % p/p.

8. Composition pharmaceutique selon l'une quelconque des revendications 1 à 7, où ladite composition pharmaceutique est mise en capsules de gélatine molles.

9. Composition pharmaceutique selon l'une quelconque des revendications 1 à 8, où la 2-(2,2-difluoro-propyl)-5-[1-(2-fluoro-6-méthyl-phényl)-pipéridin-4-yl]-7-(2-trifluorométhyl-benzyl)-2,4,5,7-tétrahydro-pyrazolo[3,4-d]pyrimidin-6-one sous une forme cristalline est utilisée pour préparer ladite composition.

10. Forme cristalline de la 2-(2,2-difluoro-propyl)-5-[1-(2-fluoro-6-méthyl-phényl)-pipéridin-4-yl]-7-(2-trifluorométhyl-benzyl)-2,4,5,7-tétrahydro-pyrazolo[3,4-d]pyrimidin-6-one, **caractérisée par** la présence de raies sur le diagramme de diffraction des rayons X sur poudre aux angles de réfraction 2θ suivants : 6,2°, 9,5° et 14,4° ; où ledit diagramme de diffraction des rayons X sur poudre est obtenu en utilisant des rayonnements Cu Kα1 et Kα2 combinés sans soustraction des raies de diffraction dues au rayonnement Kα2 ; et l'exactitude des valeurs de 2θ est dans la plage de 2θ +/- 0,2°.

11. Forme cristalline de la 2-(2,2-difluoro-propyl)-5-[1-(2-fluoro-6-méthyl-phényl)-pipéridin-4-yl]-7-(2-trifluorométhyl-benzyl)-2,4,5,7-tétrahydro-pyrazolo[3,4-d]pyrimidin-6-one selon la revendication 10, **caractérisée par** la présence de raies sur le diagramme de diffraction des rayons X sur poudre aux angles de réfraction 2θ suivants : 6,2°, 9,5°, 14,4°, 15,7° et 18,6° ; où ledit diagramme de diffraction des rayons X sur poudre est obtenu en utilisant des rayonnements Cu Kα1 et Kα2 combinés sans soustraction des raies de diffraction dues au rayonnement Kα2 ; et où l'exactitude des valeurs de 2θ est dans la plage de 2θ +/- 0,2°.

12. Forme cristalline de la 2-(2,2-difluoro-propyl)-5-[1-(2-fluoro-6-méthyl-phényl)-pipéridin-4-yl]-7-(2-trifluorométhyl-benzyl)-2,4,5,7-tétrahydro-pyrazolo[3,4-d]pyrimidin-6-one selon la revendication 11, qui a un point de fusion, tel que déterminé par calorimétrie différentielle à balayage, d'environ 163 °C.

13. Composition pharmaceutique selon l'une quelconque des revendications 1 à 9 pour une utilisation en tant que médicament.

14. Composition pharmaceutique selon l'une quelconque des revendications 1 à 9 pour une utilisation dans la prévention/prophylaxie ou le traitement de maladies ou d'affections liées à des événements pathogènes associés à des taux de C5a élevés et/ou une activation du C5aR.

15. Composition pharmaceutique selon l'une quelconque des revendications 1 à 9 pour une utilisation dans la prévention/prophylaxie ou le traitement de maladies ou d'affections sélectionnées parmi les suivantes : maladies ou affections vasculitiques, maladies ou affections inflammatoires qui font intervenir la libération intravasculaire de microvésicules, maladies ou affections à complexe immun (Cl), maladies ou affections neurodégénératives, maladies ou affections inflammatoires liées au complément, maladies ou affections bulleuses, maladies ou affections liées à une ischémie et/ou à une lésion d'ischémie-reperfusion, maladies ou affections intestinales inflammatoires, maladies ou affections auto-immunes et cancer.
